(19) Europäisches Patentamt
European Patent Office
Office européen des brevets

(11) EP 3 452 471 B1

(12) # EUROPÄISCHE PATENTSCHRIFT

(45) Veröffentlichungstag und Bekanntmachung des Hinweises auf die Patenterteilung:
**08.12.2021 Patentblatt 2021/49**

(21) Anmeldenummer: **17718025.4**

(22) Anmeldetag: **24.03.2017**

(51) Int Cl.:
***C07B 59/00*** (2006.01) ***C09B 15/00*** (2006.01)
***C09B 17/00*** (2006.01) ***C09B 19/00*** (2006.01)
***C09B 21/00*** (2006.01) ***C09B 57/00*** (2006.01)
***H01L 51/50*** (2006.01) ***H01L 51/00*** (2006.01)
***C07D 403/10*** (2006.01) ***C07D 209/82*** (2006.01)

(86) Internationale Anmeldenummer:
**PCT/EP2017/057047**

(87) Internationale Veröffentlichungsnummer:
**WO 2017/190885 (09.11.2017 Gazette 2017/45)**

(54) **ORGANISCHE MOLEKÜLE, INSBESONDERE ZUR VERWENDUNG IN OPTOELEKTRONISCHEN VORRICHTUNGEN**

ORGANIC MOLECULES, ESPECIALLY FOR USE IN OPTOELECTRONIC DEVICES

MOLÉCULES ORGANIQUES POUR UNE UTILISATION EN PARTICULIER DANS DES DISPOSITIFS OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:
**04.05.2016 DE 102016108325**
**04.05.2016 DE 102016108327**
**04.05.2016 DE 102016108329**
**04.05.2016 DE 102016108332**
**04.05.2016 DE 102016108331**
**04.05.2016 DE 102016108330**
**04.05.2016 DE 102016108334**
**04.05.2016 DE 102016108335**
**31.05.2016 DE 102016110004**

(43) Veröffentlichungstag der Anmeldung:
**13.03.2019 Patentblatt 2019/11**

(73) Patentinhaber: **CYNORA GMBH**
**76646 Bruchsal (DE)**

(72) Erfinder:
- **ZINK, Daniel**
  **76646 Bruchsal (DE)**
- **SEIFERMANN, Stefan**
  **77815 Bühl (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2015/111864 US-A1- 2013 056 720**

- **DONG RYUN LEE ET AL: "Design Strategy for 25% External Quantum Efficiency in Green and Blue Thermally Activated Delayed Fluorescent Devices", ADVANCED MATERIALS, Bd. 27, Nr. 39, 26. Oktober 2015 (2015-10-26), Seiten 5861-5867, XP055328577, DE ISSN: 0935-9648, DOI: 10.1002/adma.201502053**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 452 471 B1

**Beschreibung**

Die Erfindung betrifft rein organische Moleküle und deren Verwendung in organischen lichtemittierenden Dioden (OLEDs) und in anderen organischen optoelektronischen Vorrichtungen.

Aus der WO 2015/111864 A1 sind organische Verbindungen sowie diese Verbindungen enthaltende elektrolumineszente Vorrichtungen bekannt.

**Beschreibung**

Der vorliegenden Erfindung lag die Aufgabe zu Grunde, Moleküle bereitzustellen, die sich zur Verwendung in optoelektronischen Vorrichtungen eignen.

Die Erfindung stellt eine neue Klasse von organischen Molekülen bereit, die sich zur Verwendung in organischen optoelektronischen Vorrichtungen eignen.

Die erfindungsgemäßen organischen Moleküle sind rein organische Moleküle, weisen also keine Metallionen auf und grenzen sich so von den zur Verwendung in organischen optoelektronischen Vorrichtungen bekannten Metallkomplexverbindungen ab.

Die erfindungsgemäßen organischen Moleküle zeichnen sich durch Emissionen im blauen, himmelblauen oder grünen Spektralbereich aus. Die Photolumineszenzquantenausbeuten der erfindungsgemäßen organischen Moleküle betragen insbesondere 20 % und mehr. Die erfindungsgemäßen Moleküle zeigen insbesondere thermisch aktivierte verzögerte Fluoreszenz (TADF). Die Verwendung der erfindungsgemäßen Moleküle in einer optoelektronischen Vorrichtung, beispielsweise einer organischen lichtemittierenden Diode (OLED), führt zu höheren Effizienzen der Vorrichtung. Entsprechende OLEDs weisen eine höhere Stabilität auf als OLEDs mit bekannten Emittermaterialien und vergleichbarer Farbe.

Unter dem blauen Spektralbereich wird hier der sichtbare Bereich von kleiner als 470 nm verstanden. Unter dem himmelblauen Spektralbereich wird hier der Bereich von 470 nm bis 499 nm verstanden. Unter dem grünen Spektralbereich wird hier der Bereich von 500 nm bis 599 nm verstanden. Dabei liegt das Emissionsmaximum im jeweiligen Bereich.

Die organischen Moleküle der Beschreibung weisen eine Struktur der Formel A1 auf oder bestehen aus einer Struktur gemäß Formel A1:

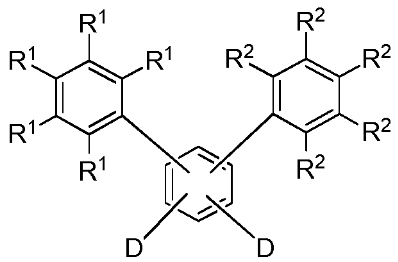

Formel A1

Erfindungsgemäß weisen die organischen Moleküle eine Struktur der Formel Ia auf oder bestehen aus einer Struktur gemäß Formel Ia:

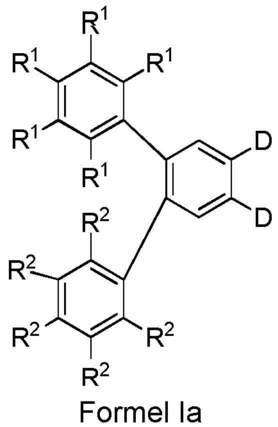

Formel Ia

In einer weiteren nicht erfindungsgemäßen Offenbarung der Beschreibung weisen die organischen Moleküle eine Struktur der Formel Ib auf oder bestehen aus einer Struktur gemäß Formel Ib:

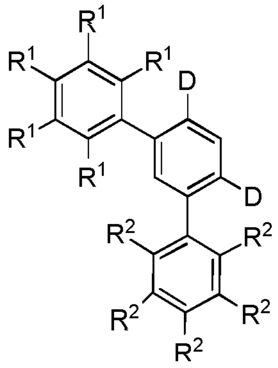

Formel Ib

In einer weiteren nicht erfindungsgemäßen Offenbarung der Beschreibung weisen die organischen Moleküle eine Struktur der Formel Ic auf oder bestehen aus einer Struktur gemäß Formel Ic:

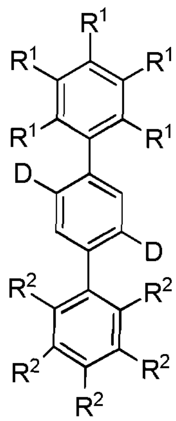

Formel Ic

In einer weiteren nicht erfindungsgemäßen Offenbarung der Beschreibung weisen die organischen Moleküle eine Struktur der Formel Id auf oder bestehen aus einer Struktur gemäß Formel Id:

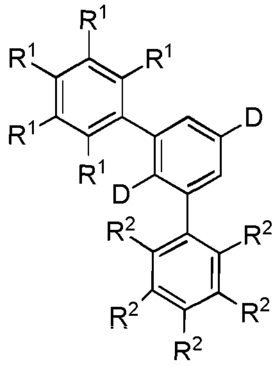

Formel Id

In einer weiteren nicht erfindungsgemäßen Offenbarung der Beschreibung weisen die organischen Moleküle eine Struktur der Formel Ie auf oder bestehen aus einer Struktur gemäß Formel Ie:

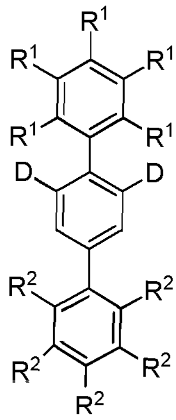

Formel Ie

In einer weiteren nicht erfindungsgemäßen Offenbarung der Beschreibung weisen die organischen Moleküle eine Struktur der Formel If auf oder bestehen aus einer Struktur gemäß Formel If:

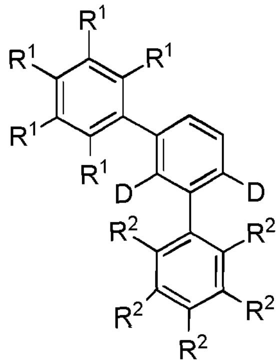

Formel If

In einer weiteren nicht erfindungsgemäßen Offenbarung der Beschreibung weisen die organischen Moleküle eine Struktur der Formel Ig auf oder bestehen aus einer Struktur gemäß Formel Ig:

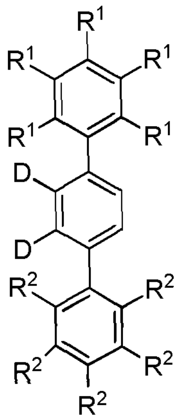

Formel Ig

In einer weiteren nicht erfindungsgemäßen Offenbarung der Beschreibung weisen die organischen Moleküle eine Struktur der Formel Ih auf oder bestehen aus einer Struktur gemäß Formel Ih:

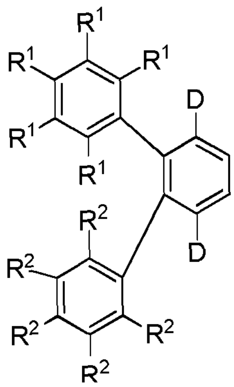

Formel Ih

In den oben genannten Formeln gilt für die verwendeten Symbole Folgendes:
D =

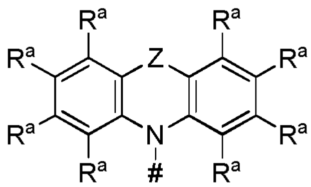

Formel I-1

Darin gilt:

# ist jeweils der Anknüpfungspunkt der Einheit D an den zentralen (mittleren) Phenylring in den Strukturen der Formeln A1 und Ia bis Ih;

Z ist eine direkte Bindung oder ausgewählt aus der Gruppe bestehend aus $CR^3R^4$, $C=CR^3R^4$, C=O, $C=NR^3$, $NR^3$, O, $SiR^3R^4$, S, S(O) und $S(O)_2$.

$R^1$ und $R^2$ ist bei jedem Auftreten gleich oder verschieden Deuterium, eine lineare Alkylgruppe mit 1 bis 5 C-Atomen, eine lineare Alkenyl- oder Alkinylgruppe mit 2 bis 8 C-Atomen, eine verzweigte oder cyclische Alkyl-, Alkenyl- oder Alkinylgruppe mit 3 bis 10 C-Atomen, wobei ein oder mehrere H-Atome durch Deuterium, CN, $CF_3$ oder $NO_2$ ersetzt sein können, oder ein aromatisches oder heteroaromatisches Ringsystem mit 5 bis 15 aromatischen Ringatomen, das jeweils mit einem oder mehreren Resten $R^6$ substituiert sein kann, $CF_3$ oder CN.

$R^a$, $R^3$ und $R^4$ ist bei jedem Auftreten gleich oder verschieden H, Deuterium, $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehrerem Resten $R^5$ substituiert sein kann, oder eine Aryloxy- oder Heteroaryloxygruppe mit 5 bis 60 aromatischen Ringatomen, die mit einem oder mehreren Resten $R^5$ substituiert sein kann, oder eine Diarylaminogruppe, Diheteroarylaminogruppe oder Arylheteroarylaminogruppe mit 10 bis 40 aromatischen Ringatomen, welche mit einem oder mehrerne Resten $R^5$ substituiert sein kann.

$R^5$ ist bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann,

wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^6$ substituiert sein kann, oder eine Aryloxy- oder Heteroaryloxygruppe mit 5 bis 60 aromatischen Ringatomen, die mit einem oder mehreren Resten $R^6$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^6$ substituiert sein kann.

$R^6$ ist bei jedem Auftreten gleich oder verschieden H, Deuterium, OH, $CF_3$, CN, F, Br, I, eine lineare Alkyl-, Alkoxy- oder Thioalkoxygruppe mit 1 bis 5 C-Atomen oder eine lineare Alkenyl- oder Alkinylgruppe mit 2 bis 5 C-Atomen oder eine verzweigte oder cyclische Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen oder eine Aryloxy- oder Heteroaryloxygruppe mit 5 bis 60 aromatischen Ringatomen oder eine Diarylaminogruppe, Diheteroarylaminogruppe oder Arylheteroarylaminogruppe mit 10 bis 40 aromatischen Ringatomen.

Dabei kann jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoannelliertes Ringsystem bilden.

Erfindungsgemäß sind hierbei genau ein $R^1$ und genau ein $R^2$ gleich $CF_3$ oder CN. In einer Ausführungsform der Erfindung sind genau ein $R^1$ und ein $R^2$ CN. In einer weiteren Ausführungsform sind genau ein $R^1$ und ein $R^2$ CN und die weiteren $R^1$und $R^2$ sind gewählt aus der Gruppe bestehend aus H und Alkyl, insbesondere sind die weiteren $R^1$und $R^2$ gewählt aus der Gruppe bestehend aus H, Methyl und t-Butyl ($C(CH_3)_3$); in einer Ausführungsform sind genau ein weiterer Rest $R^1$und genau ein weiterer Rest $R^2$ Methyl und die weiteren $R^1$ und $R^2$ H. In einer Ausführungsform sind genau ein $R^1$ und genau ein $R^2$ gleich CN und die weiteren $R^1$und $R^2$ sind H.

In einer Ausführungsform ist Z gleich einer direkten Bindung oder ausgewählt aus der Gruppe bestehend aus $CR^3R^4$, $C=CR^3R^4$, $C=NR^3$, $NR^3$, O, $SiR^3R^4$, S, S(O) und $S(O)_2$.

In einer weiteren Ausführungsform der organischen Moleküle weist die Gruppe D eine Struktur der Formel II auf bzw. besteht aus einer Struktur der Formel II:

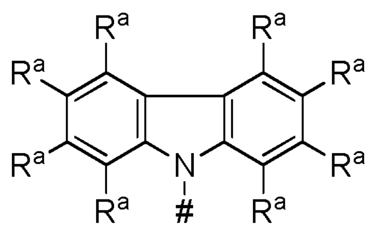

Formel II

wobei für # und $R^a$ die oben genannten Definitionen gelten.

In einer weiteren Ausführungsform der erfindungsgemäßen organischen Moleküle weist die Gruppe D eine der Formel IIa oder der Formel IIb auf oder besteht daraus:

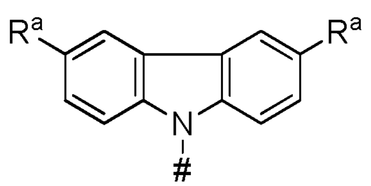

Formel IIa

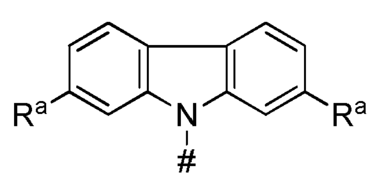

Formel IIb

wobei für #, und $R^a$ die oben genannten Definitionen gelten.

Im Folgenden sind beispielhaft Ausführungsformen der Gruppe D gezeigt:

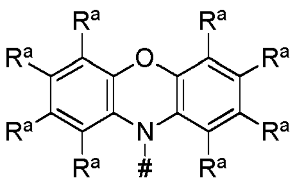
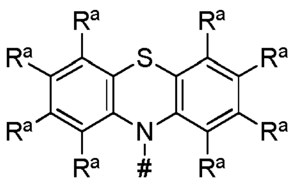
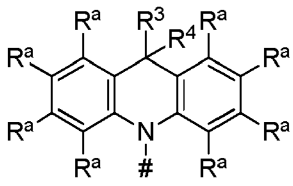
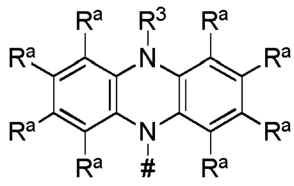

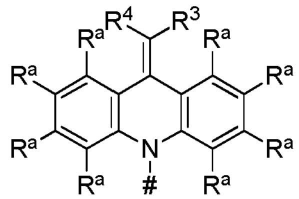
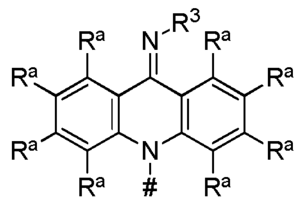
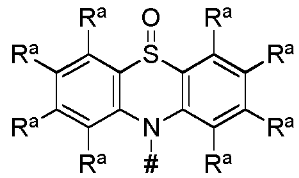

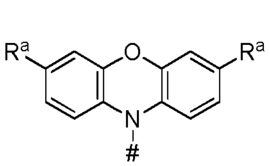
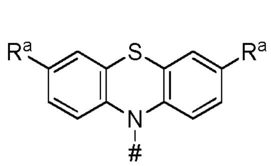
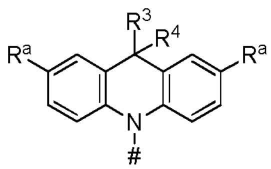
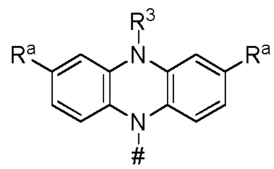

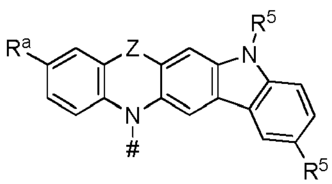
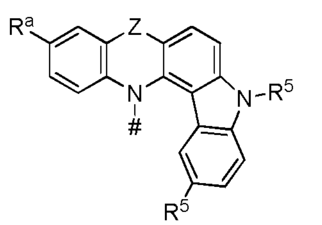
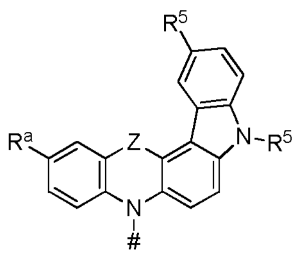

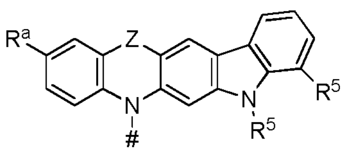
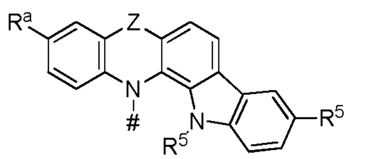
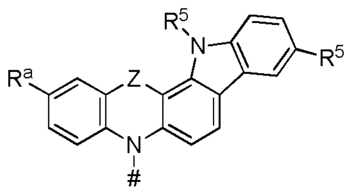

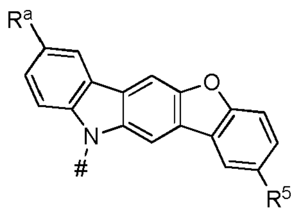
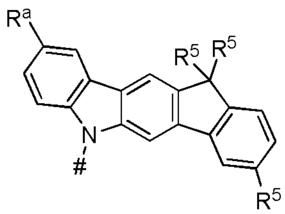
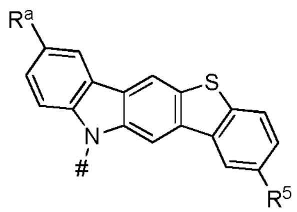

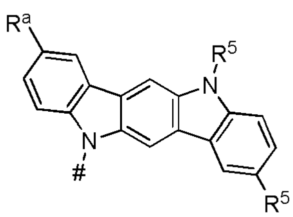

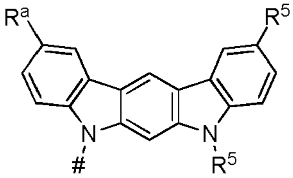
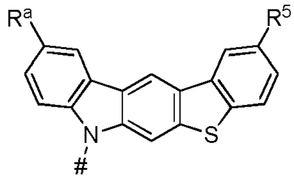
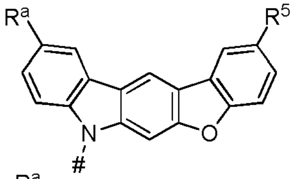
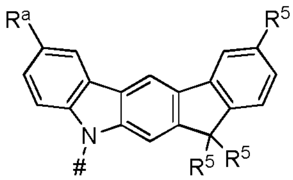
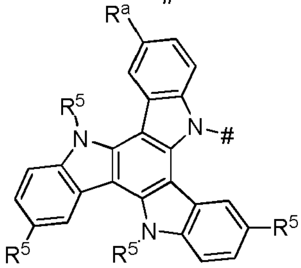

wobei für #, $R^a$ und $R^5$ die oben genannten Definitionen gelten. In einer Ausführungsform ist der Rest $R^5$ bei jedem Auftreten gleich oder verschieden ausgewählt aus der Gruppe bestehend aus H, Methyl, Ethyl, Phenyl und Mesityl. In einer Ausführungsform ist $R^a$ bei jedem Auftreten gleich oder verschieden ausgewählt aus der Gruppe bestehend aus H, Methyl (Me), i-Propyl ($CH(CH_3)_2$) ($^i$Pr), t-Butyl ($^t$Bu), Phenyl (Ph) und Diphenylamin ($NPh_2$).

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen Ringatome oder der enthaltenen Heteroatome, so gelten diese.

Unter einer Arylgruppe bzw. Heteroarylgruppe wird ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1 -Dimethyl-n-octadec-1 -yl-, 1,1 -Diethyl-n-hex-1 -yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- und 1-(n-Decyl)-cyclohex-1-yl verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Eine Ausführungsform der Erfindung betrifft organische Moleküle, welche einen $\Delta E(S_1-T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ aufweisen und/oder eine Emissionslebensdauer von höchstens 150 $\mu$s, insbesondere von höchstens 100 $\mu$s, von höchsten 50 $\mu$s, oder von höchstens 10 $\mu$s aufweisen und/oder eine Hauptemissionsbande mit einer Halbwertsbreite kleiner als 120 nm, insbesondere kleiner als 100 nm, kleiner als 80 nm, oder kleiner als 60 nm aufweisen.

Die Bestimmung des $\Delta E(S_1-T_1)$-Wertes kann sowohl durch quantenmechanische Berechnungen mittels im Stand der Technik bekannten Computerprogrammen durchgeführt werden (z. B. mittels Turbomole-Programmen unter Ausführung von TD-DFT- und unter Berücksichtigung von CC2-Rechnungen) oder — wie weiter unten erläutert wird - experimentell bestimmt werden.

Die Energiedifferenz $\Delta E(S_1-T_1$ lässt sich näherungsweise quantenmechanisch durch das mit dem Faktor 2 multiplizierte sogenannte Austauschintegral beschreiben. Dessen Wert hängt direkt ab von der Überlappung der Molekülorbitale. Diese Molekülorbitale sind über unterschiedliche Raumbereiche verteilt (teil-delokalisiert über $\pi$- bzw. $\pi^*$-Molekülorbitale). Das heißt, ein elektronischer Übergang zwischen den verschiedenen Molekülorbitalen repräsentiert einen sogenannten Charge-Transfer (CT)-Übergang. Je geringer die Überlappung der oben beschriebenen Molekülorbitale ist, desto ausgeprägter ist der elektronische Charge-Transfer Charakter. Das ist dann mit einer Abnahme des Austausch-Integrals und somit einer Abnahme der Energiedifferenz $\Delta E(S_1-T_1)$ verbunden.

Die Bestimmung des $\Delta E(S_1-T_1)$-Wertes kann experimentell folgendermaßen erfolgen:

Für ein vorgegebenes organisches Molekül lässt sich der Energieabstand $\Delta E(S_1-T_1) = \Delta E$ unter Verwendung der oben angegebenen Gleichung (1) einfach bestimmen. Eine Umformung ergibt:

$$\ln\{Int(S_1\rightarrow S_0)/Int(T_1\rightarrow S_0)\} = \ln\{k(S_1)/k(T_1)\} - (\Delta E/k_B)(1/T) \qquad (3)$$

Für die Messung der Intensitäten $Int(S_1\rightarrow S_0)$ und $Int(T_1\rightarrow S_0)$ kann jedes handelsübliche Spektralphotometer verwendet werden. Eine graphische Auftragung der bei verschiedenen Temperaturen gemessenen (logarithmierten) Intensitätsverhältnisse $\ln\{Int(S_1\rightarrow S_0)/Int(T_1\rightarrow S_0)\}$ gegen den Kehrwert der absoluten Temperatur T ergibt in der Regel eine Gerade. Die Messung wird in einem Temperaturbereich von Raumtemperatur (300 K) bis 77 K oder bis 4,2 K durchgeführt, wobei die Temperatur mittels eines Kryostaten eingestellt wird. Die Intensitäten werden aus den (korrigierten) Spektren bestimmt, wobei $Int(S_1\rightarrow S_0)$ bzw. $Int(T_1\rightarrow S_0)$ die integrierten Fluoreszenz- bzw. Phosphoreszenz-Bandenintensitäten repräsentieren, welche sich mittels der zum Spektralphotometer gehörenden Programme bestimmen lassen. Die jeweiligen Übergänge (Bandenintensitäten) lassen sich leicht identifizieren, da die Triplett-Bande bei niedrigerer Energie liegt als die Singulett-Bande und mit sinkender Temperatur an Intensität gewinnt. Dabei werden die Messungen in sauerstofffreien verdünnten Lösungen (ca. $10^{-2}$ mol/L) oder an dünnen Filmen aus den entsprechenden Molekülen oder an mit den entsprechenden Molekülen dotierten Filmen durchgeführt. Verwendet man als Probe eine Lösung, so empfiehlt es sich, ein Lösemittel bzw. Lösemittelgemisch zu verwenden, das bei tiefen Temperaturen Gläser bildet, wie 2-Methyl-THF, THF (Tetrahydrofuran) oder aliphatische Kohlenwasserstoffe. Verwendet man als Probe einen Film, so eignet sich die Verwendung einer Matrix mit einer deutlich größeren Singulettsowie Triplett-Energie als die der organischen Emittermoleküle, z. B. PMMA (Polymethylmethacrylat). Dieser Film kann aus Lösung aufgebracht werden.

Die Geradensteigung beträgt—$\Delta E/k_B$. Mit $k_B = 1{,}380\ 10^{-23}\ JK^{-1} = 0{,}695\ cm^{-1}\ K^{-1}$ lässt sich der Energieabstand direkt bestimmen.

Eine äquivalente Betrachtungsweise zeigt, dass mittels der Messung der Temperaturabhängigkeit der Emissionsabklingzeit der $\Delta E(S_1-T_1)$-Wert auch bestimmt werden kann.

Eine einfache, näherungsweise Abschätzung des $\Delta E(S_1-T_1)$-Wertes kann auch dadurch vorgenommen werden, dass bei tiefer Temperatur (z. B. 77 K oder 4,2 K unter Verwendung eines Kryostaten) die Fluoreszenz- und Phosphoreszenz-Spektren registriert werden. Der $\Delta E(S_1-T_1)$-Wert entspricht dann in Näherung der Energiedifferenz zwischen den hochenergetischen Anstiegsflanken der Fluoreszenz- bzw. Phosphoreszenz-Bande.

Je ausgeprägter der CT-Charakter eines organischen Moleküls ist, desto stärker verändern sich die elektronischen Übergangsenergien als Funktion der Lösungsmittelpolarität. So gibt bereits eine ausgeprägte Polaritätsabhängigkeit der Emissionsenergien einen Hinweis auf das Vorliegen kleiner $\Delta E(S_1-T_1)$-Werte.

In einem weiteren Aspekt betrifft die Erfindung ein Verfahren zur Herstellung eines erfindungsgemäßen organischen Moleküls der hier beschriebenen Art (mit einer eventuellen Folgeumsetzung), wobei ein Dibromdifluorbenzol als Edukt eingesetzt wird. Erfindungsgemäße Dibromdifluor-benzole sind 1,2-Dibrom-4,5-difluorbenzol, 1,3-Dibrom-4,6-difluorbenzol, 1,5-Dibrom-2,4-difluorbenzol, 1,4-Dibrom-2,5-difluorbenzol, 1,3-Dibrom-2,5-difluorbenzol, 1,4-Dibrom-2,6-difluorbenzol, 1,3-Dibrom-2,4-difluorbenzol, 1,4-Dibrom-2,3-difluorbenzol oder 1,2-Dibrom-3,6-difluorbenzol.

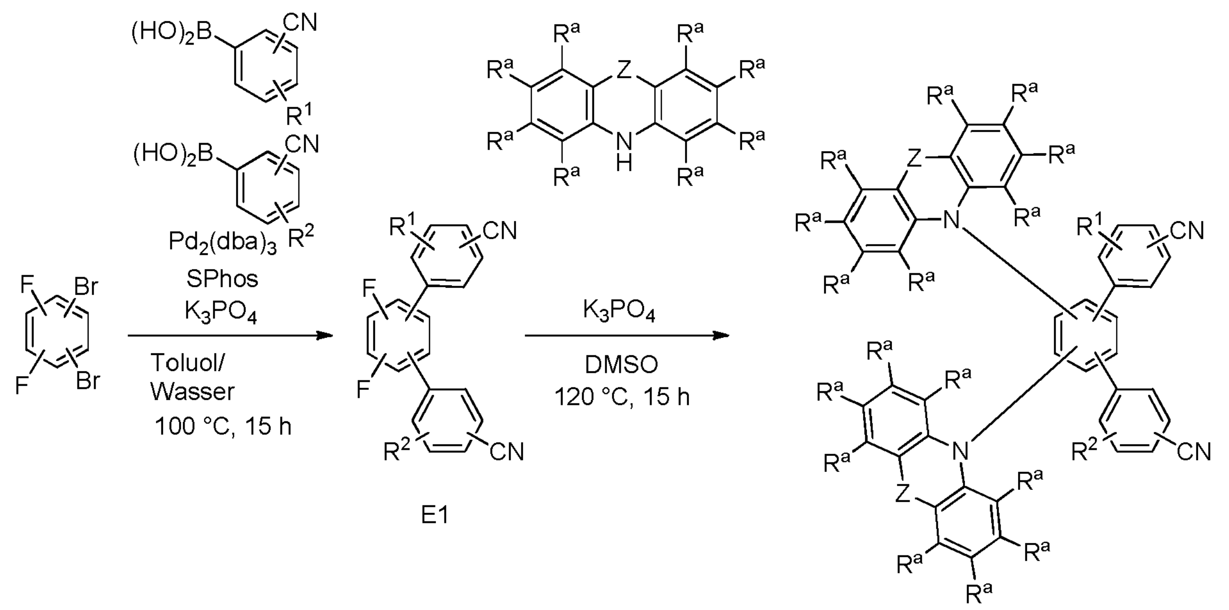


In einer Ausführungsform wird das Dibromdifluorbenzol mit einer Benzonitrilboronsäure in einer Palladium-katalysierten Kreuzkupplungsreaktion umgesetzt. Das Produkt wird durch Deprotonierung des der Formel I-1 entsprechenden Amins und anschließender nukleophiler Substitution der Fluorgruppen erhalten. Hierbei wird ein Stickstoffheterozyklus im Sinne einer nukleophilen aromatischen Substitution mit einem Edukt E1 umgesetzt. Typische Bedingungen beinhalten die Verwendung einer Base wie beispielweise tribasisches Kaliumphosphat oder Natriumhydrid in einem aprotisch polarem Lösungsmittel wie beispielweise Dimetylsulfoxid (DMSO) oder N,N-Dimethylformamid (DMF). Durch Wahl des Dibromdifluorbenzol und der relativen Position der Boronsäure- und der Cyanogruppe und des Restes $R^1$ am Phenylring können unterschiedliche Substitutionsmuster erhalten werden.

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und
(b) mindestens ein, d. h. ein oder mehrere Emitter- und/oder Hostmaterialien, die von dem erfindungsgemäßen organischen Molekül verschiedenen ist bzw. sind und
(c) optional eine oder mehreren Farbstoffen und/ oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett (T1)- und Singulett (S1)- Energieniveaus auf, die energetisch höher liegen als die Triplett (T1)- und Singulett (S1)- Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das des elektronendominanten Hostmaterials. Das HOMO des lochdominanten Hostmaterials liegt energetisch unter dem HOMO des erfindungsgemäßen organischen Moleküls,

während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen dem Fachmann bekannten invertierten Aufbau auf.

Gemäß einer weiteren Ausführungsform weist das organische Bauelement, insbesondere eine OLED, einen dem Fachmann bekannten gestapelten Aufbau auf. Hierdurch kann die Erzeugung von Mischlicht ermöglicht werden. Weiterhin können bei praktisch gleicher Effizienz und identischer Leuchtdichte signifikant längere Lebensdauern im Vergleich zu üblichen OLEDs erzielt werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen. Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie

beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm.

Desweiteren können kleine Moleküle können verwendet werden (z. B. Kupfer-Phthalocyanin (CuPc z. B. 10 nm dick)) oder Metalloxide wie beispielhaft $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N*,*N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10-100 nm.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan) oder DPEPO (Bis[2-((oxo)diphenylphosphino)phenyl]ether). Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl) ), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

Dem Fachmann ist hierbei bekannt, welche Kombinationen der Materialien für eine optoelektronische Vorrichtung enthaltend ein erfindungsgemäßes organisches Molekül zu nutzen sind.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Der Massenanteil des erfindungsgemäßen organischen Moleküls an der Emitter-Schicht EML beträgt in einer

weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierender Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett (T1)- und Singulett (S1)-Energieniveaus energetisch höher liegen als die Triplett (T1)- und Singulett (S1)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierender Schicht aufgebracht ist, und die lichtemittierende Schicht zwischen löcher- und elektronentransportierender Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

**Beispiele**

Allgemeines Syntheseschema

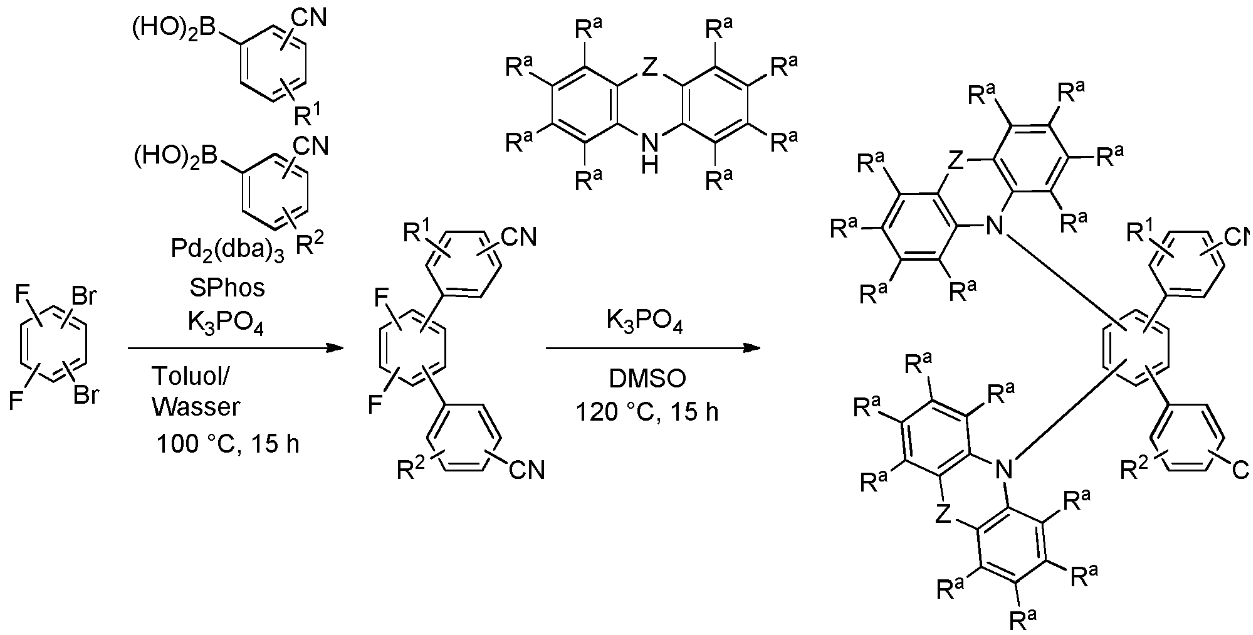


*Allgemeine Synthesevorschrift **AAV1**:*

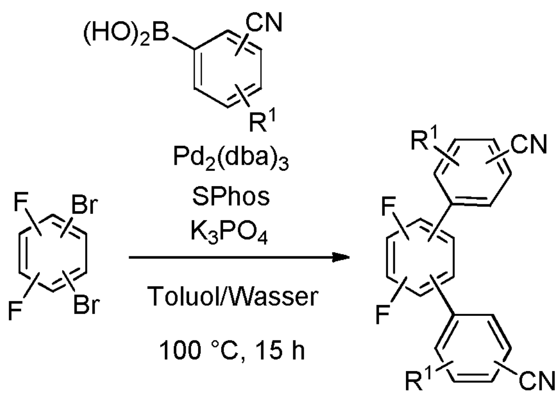


Das entsprechende Difluor-dibrom-benzol (2,00 mmol, 1,00 Aquiv.), die Boronsäure (7,20 mmol, 3,6 Äquiv.), Tris(dibenzylideneacetone)dipalladium (0,09 mmol, 0,04 Äquiv.), 2-Dicyclohexylphosphino-2',6'-dimethoxybiphenyl (0,35 mmol, 0,16 Äquiv.) und tribasisches Kaliumphosphat (11,0 mmol, 5 Äquiv.) werden unter Stickstoff in Toluol (40 mL) und Wasser (8 mL) für 12-24 h bei 100 °C gerührt. Anschließend wird die Reaktionsmischung auf 400 mL gesättigte NaCl-Lösung gegeben und mit Essigsäureethylester extrahiert (2 x 200 mL). Die vereinigten organischen Phasen werden mit gesättigter NaCl-Lösung (200 mL) gewaschen, getrocknet über $MgSO_4$ und das Lösemittel im Vakuum entfernt. Das erhaltene Rohprodukt wird durch Flash-Chromatographie oder durch Umkristallisation gereinigt.

Allgemeine Synthesevorschrift ***AAV2***:

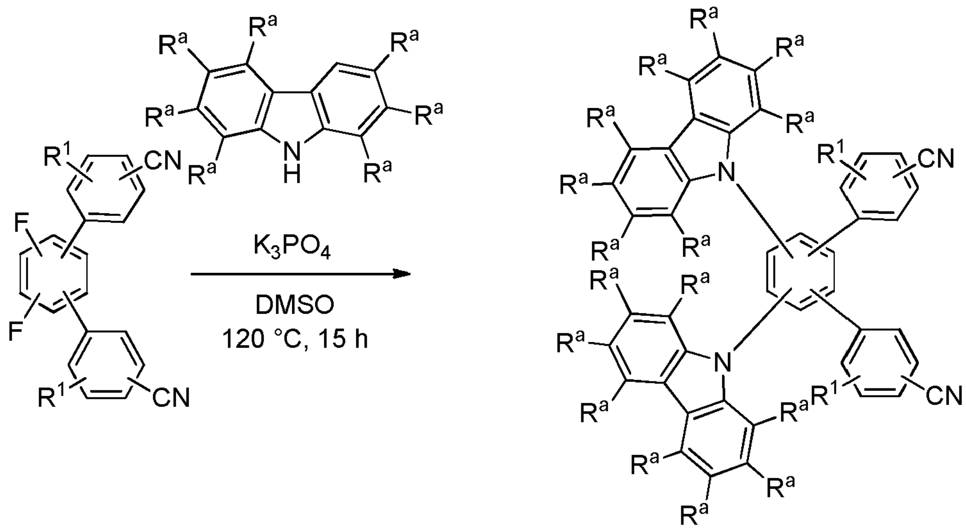


Das entsprechende Difluor- -dibenzonitril-benzol (10,0 mmol, 1,00 Aquiv.), ein entsprechendes Carbazolderivat (20,0 mmol, 2,00 Aquiv.) und tribasisches Kaliumphosphat (40,0 mmol, 4,00 Aquiv.) werden unter Stickstoff in DMSO (30 mL) suspendiert und bei 120 °C für 12 bis 24 h gerührt. Anschließend wird die Reaktionsmischung auf 400 mL gesättigte NaCl-Lösung gegeben und mit Essigsäureethylester extrahiert (2 x 200 mL). Die vereinigten organischen Phasen werden mit gesättigter NaCl-Lösung (200 mL) gewaschen, getrocknet über $MgSO_4$ und das Lösemittel im Vakuum entfernt. Das erhaltene Rohprodukt wird durch Flash-Chromatographie oder durch Umkristallisation gereinigt.

Photophysikalische Messungen

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

*Probenvorbereitung, Film: Spin-Coating*

Gerät: Spin150, SPS euro.

Die Probenkonzentration entsprach 10 mg/ml, angesetzt in Toluol oder Chlorbenzol. Programm: 1) 3 s bei 400 U/min; 2) 20 s bei 1000 U/min bei 1000 Upm/ s. 3) 10 s bei 4000 U/min bei 1000 Upm/s. Die Filme wurden nach dem Beschichten für 1 min bei 70 °C an Luft auf einer Präzisionsheizplatte von LHG getrocknet.

Photolumineszenzspektroskopie und TCSPC

Steady-state Emissionsspektroskopie wurde mit einem Fluoreszenzspektrometer der Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer "zeit-korrelierten Einphotonzähl" (*Time-correlated single-photon counting*, TCSPC)-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen:

NanoLED 370 (Wellenlänge: 371 nm, Pulsdauer: 1,1 ns)
NanoLED 290 (Wellenlänge: 294 nm, Pulsdauer: <1 ns)
SpectraLED 310 (Wellenlänge: 314 nm)
SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: gemessenen Größe.

Quanteneffizienzbestimmung

Die Messung der Photolumineszenzquantenausbeute (PLQY) erfolgte mittels eines *Absolute PL Quantum Yield Measurement* C9920-03G-Systems der *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- (*back thinned*-) CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0.

Das Emissionsmaximum wird in nm, die Quantenausbeute $\Phi$ in % und die CIE-Farbkoordinaten als x,y-Werte angegeben.

Die Photolumineszenzquantenausbeute wurde nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.
3) Durchführung der Probenmessung:
Es wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

Herstellung und Charakterisierung von organischen Elektrolumineszenzvorrichtungen aus der Gasphase

Mit den erfindungsgemäßen organischen Molekülen können OLED-Devices mittels Vakuum-Sublimationstechnik erstellt werden.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, den Elektrolumineszenzspektren und dem Strom aufgenommen.

Die am Device anliegende Spannung beträgt z. B. 2,5 V bis 15 V.

Synthese von Vorstufen nach AAV1 zur Darstellung der erfindungsgemäßen Moleküle ausgehend von kommerziell erhältlichen Verbindungen

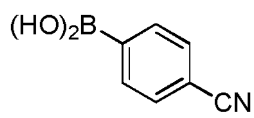

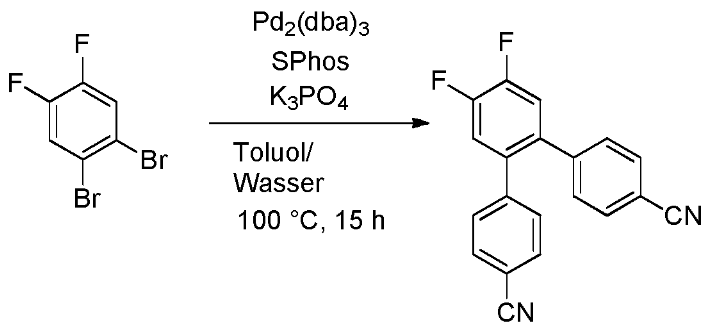
Pd2(dba)3
SPhos
K3PO4
Toluol/
Wasser
100 °C, 15 h

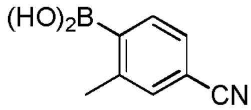

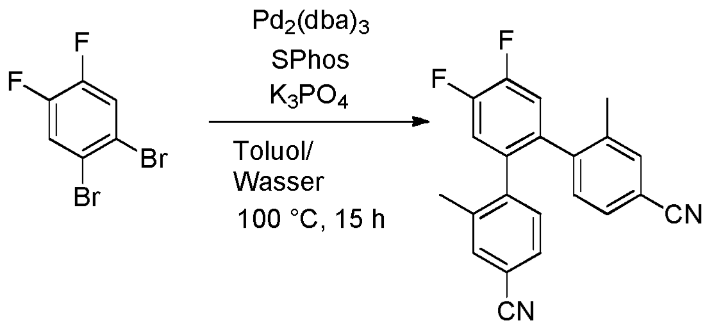
Pd2(dba)3
SPhos
K3PO4
Toluol/
Wasser
100 °C, 15 h

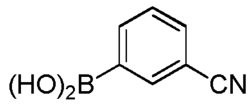

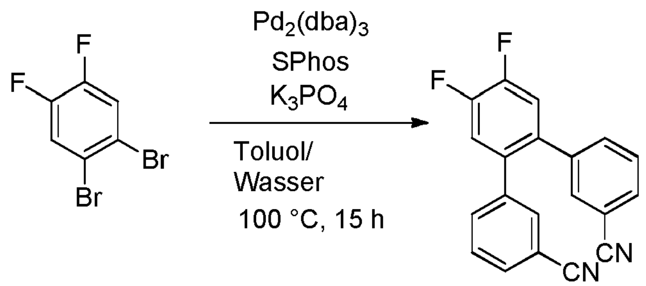
Pd2(dba)3
SPhos
K3PO4
Toluol/
Wasser
100 °C, 15 h

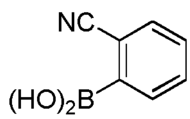

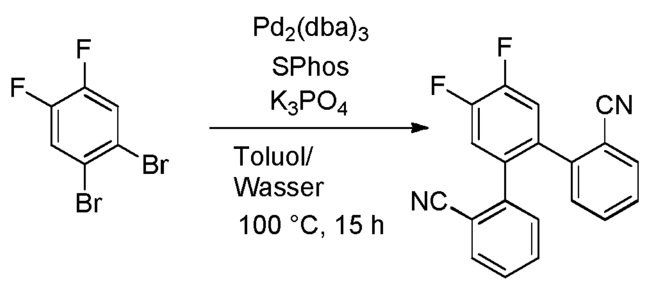
Pd2(dba)3
SPhos
K3PO4
Toluol/
Wasser
100 °C, 15 h

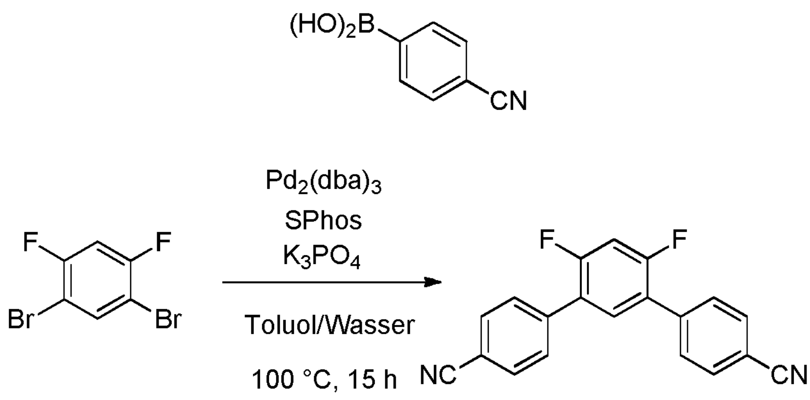
Pd$_2$(dba)$_3$
SPhos
K$_3$PO$_4$
Toluol/Wasser
100 °C, 15 h

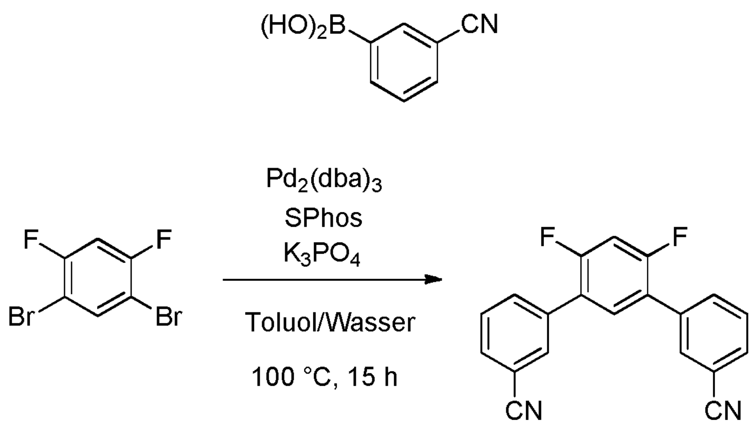
Pd$_2$(dba)$_3$
SPhos
K$_3$PO$_4$
Toluol/Wasser
100 °C, 15 h

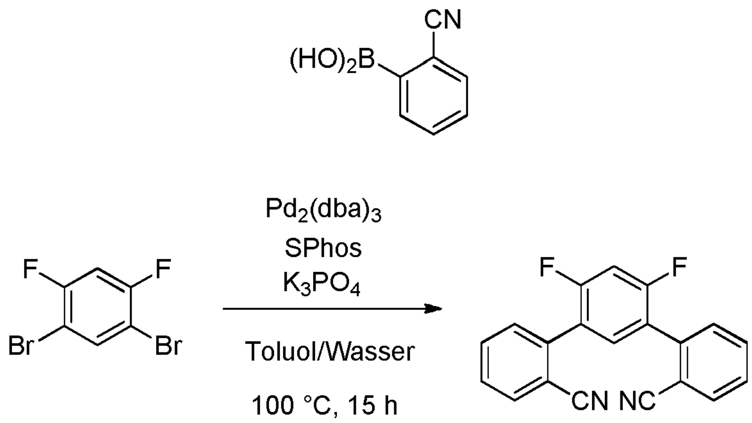
Pd$_2$(dba)$_3$
SPhos
K$_3$PO$_4$
Toluol/Wasser
100 °C, 15 h

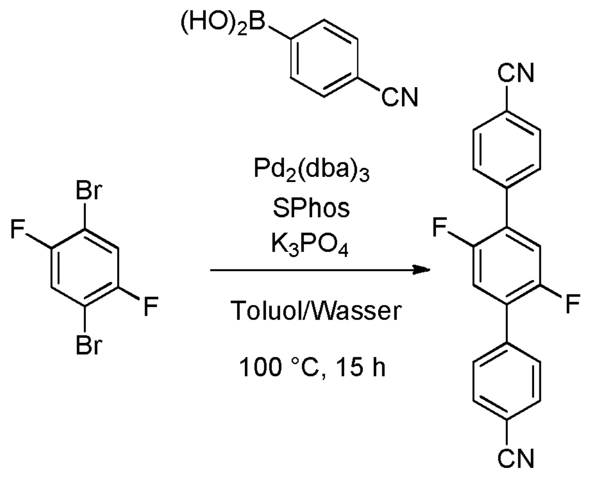
Pd2(dba)3
SPhos
K3PO4
Toluol/Wasser
100 °C, 15 h

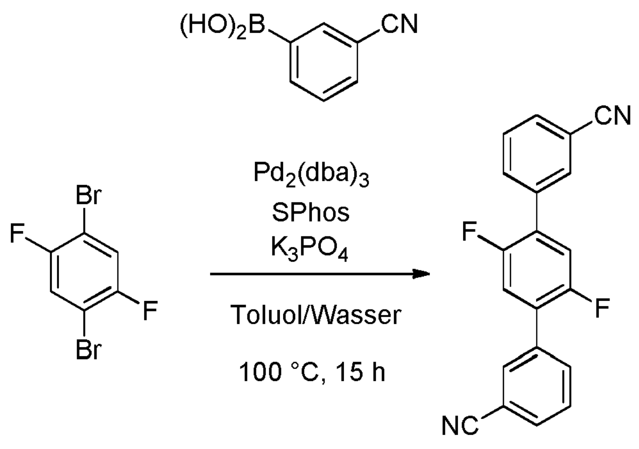
Pd2(dba)3
SPhos
K3PO4
Toluol/Wasser
100 °C, 15 h

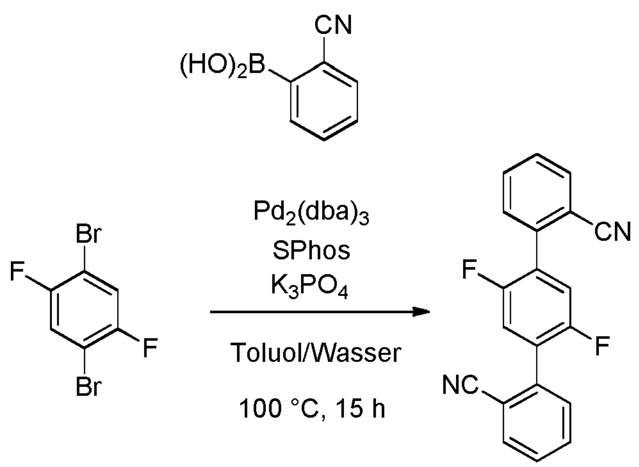
Pd2(dba)3
SPhos
K3PO4
Toluol/Wasser
100 °C, 15 h

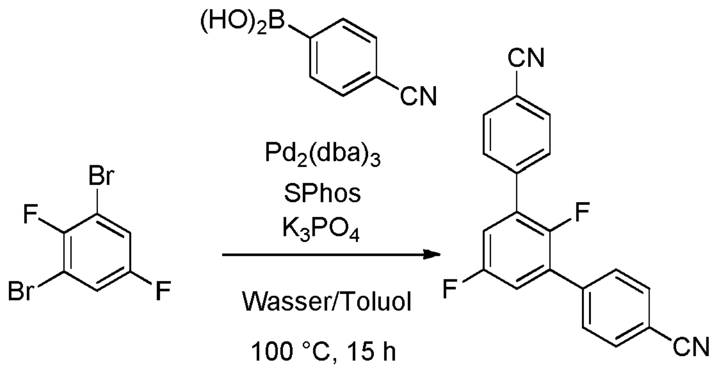
Pd2(dba)3
SPhos
K3PO4
Wasser/Toluol
100 °C, 15 h

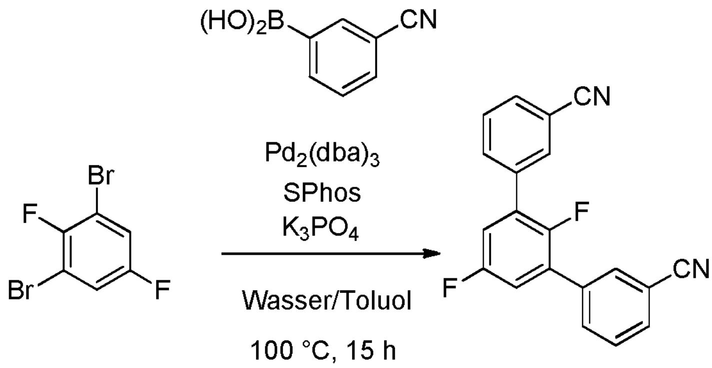
Pd2(dba)3
SPhos
K3PO4
Wasser/Toluol
100 °C, 15 h

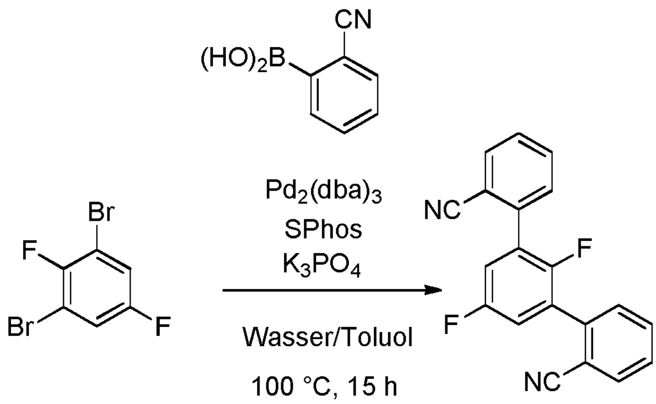
Pd2(dba)3
SPhos
K3PO4
Wasser/Toluol
100 °C, 15 h

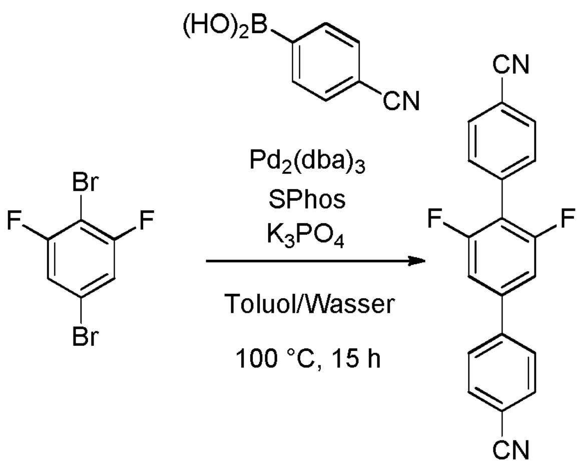
Pd2(dba)3
SPhos
K3PO4
Toluol/Wasser
100 °C, 15 h

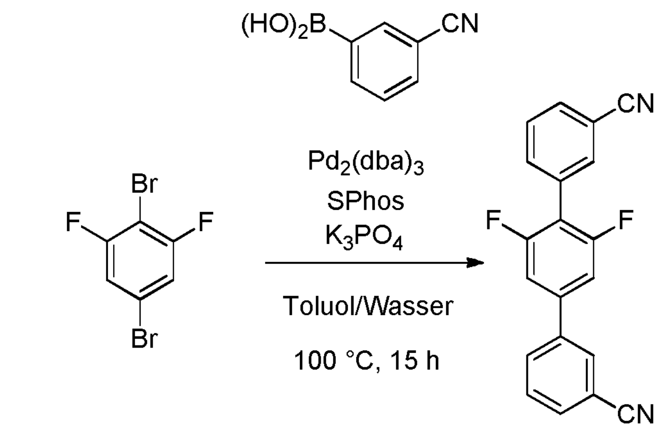
Pd2(dba)3
SPhos
K3PO4
Toluol/Wasser
100 °C, 15 h

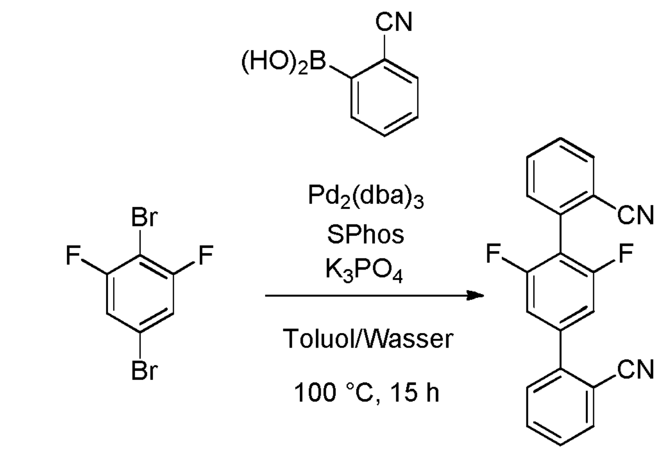
Pd2(dba)3
SPhos
K3PO4
Toluol/Wasser
100 °C, 15 h

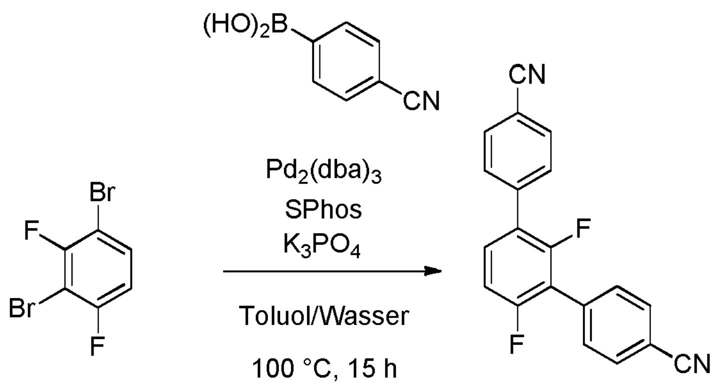
Pd2(dba)3
SPhos
K3PO4
Toluol/Wasser
100 °C, 15 h

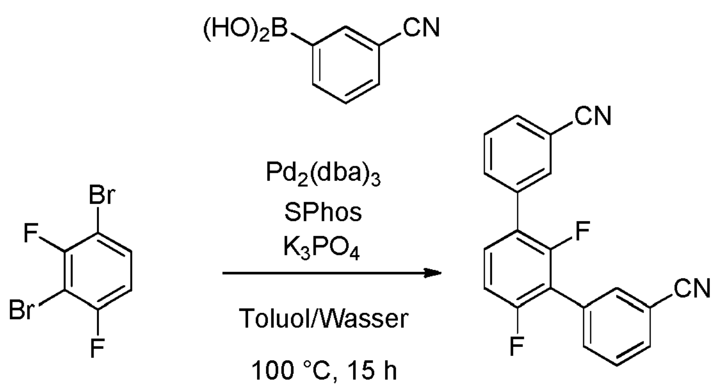
Pd2(dba)3
SPhos
K3PO4
Toluol/Wasser
100 °C, 15 h

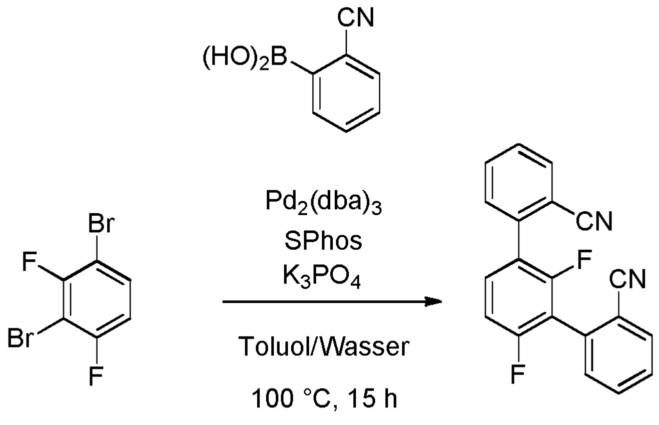

$Pd_2(dba)_3$
SPhos
$K_3PO_4$
Toluol/Wasser
100 °C, 15 h


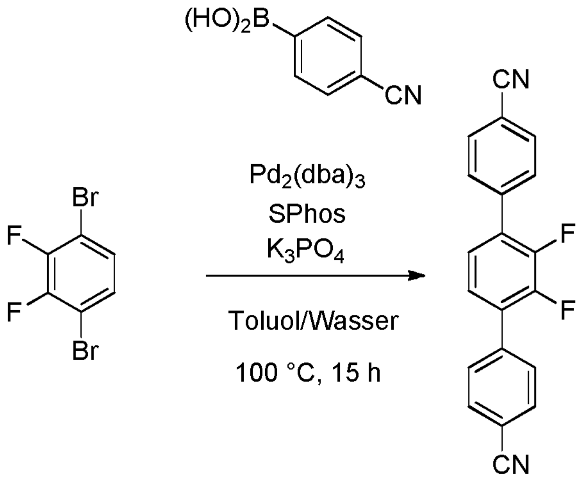

$Pd_2(dba)_3$
SPhos
$K_3PO_4$
Toluol/Wasser
100 °C, 15 h


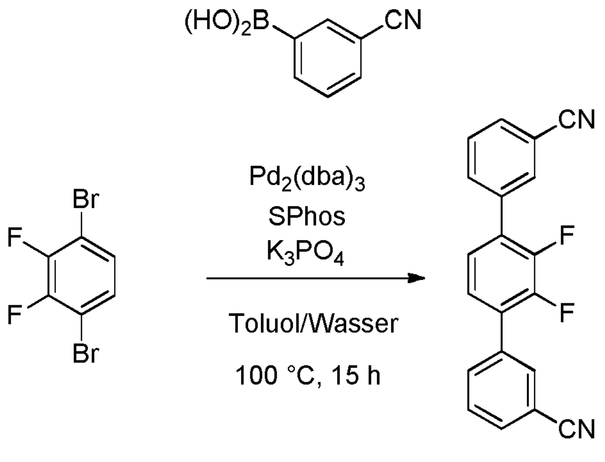

$Pd_2(dba)_3$
SPhos
$K_3PO_4$
Toluol/Wasser
100 °C, 15 h

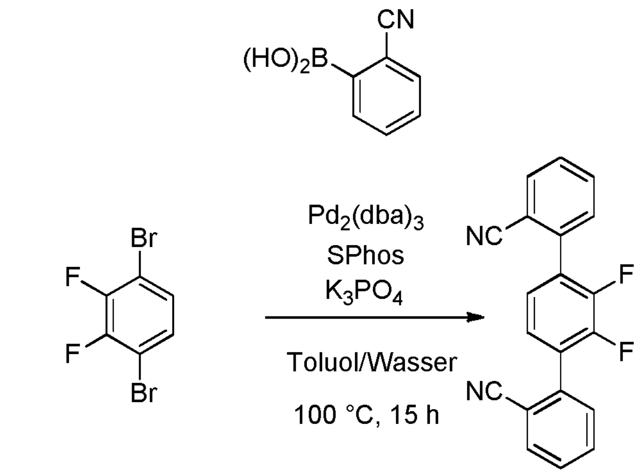
Pd2(dba)3
SPhos
K3PO4
Toluol/Wasser
100 °C, 15 h

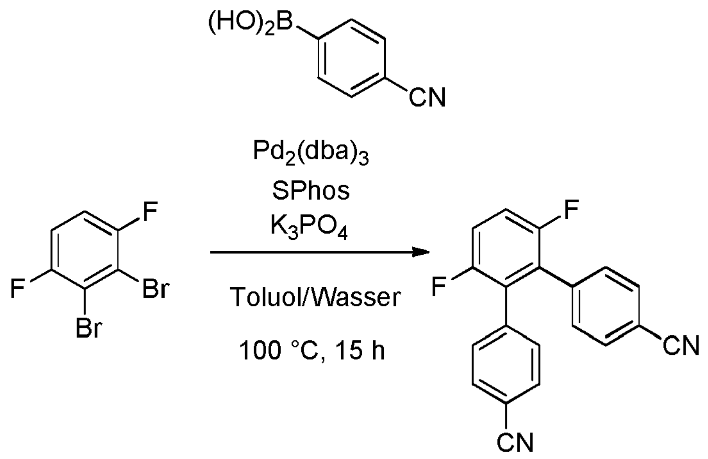
Pd2(dba)3
SPhos
K3PO4
Toluol/Wasser
100 °C, 15 h

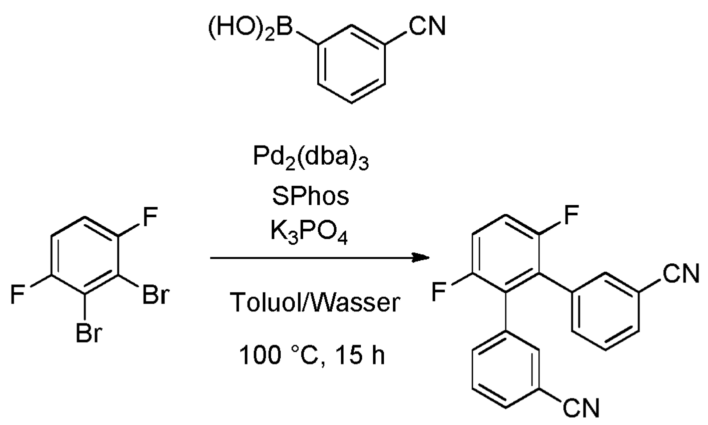
Pd2(dba)3
SPhos
K3PO4
Toluol/Wasser
100 °C, 15 h

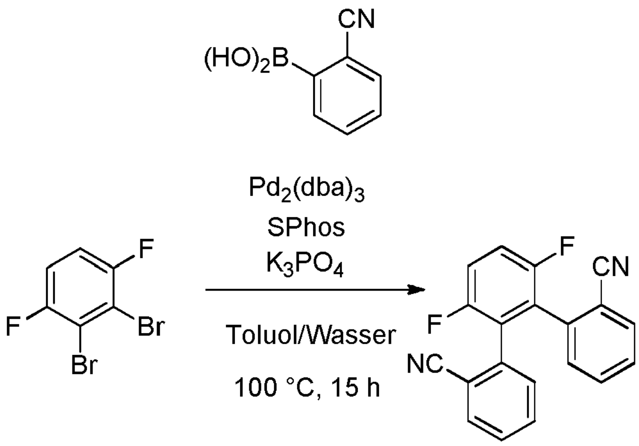


## Beispiel 1

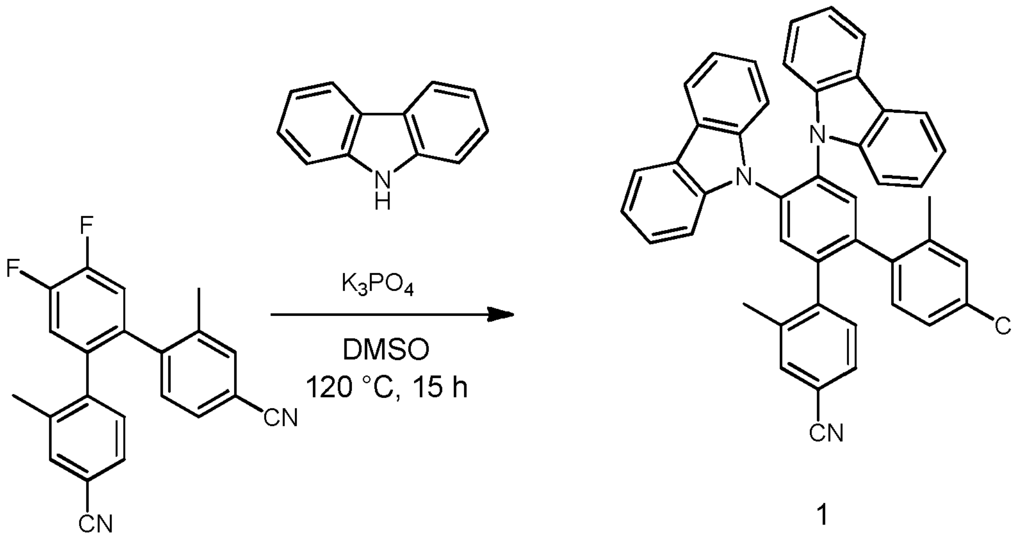


1

Beispiel 1 wurde nach AAV2 hergestellt.

Dünnschichtchromatografie: $R_f$ = 0,3 (Cyclohexan/Ethylacetat 5:1)

Figur 1 zeigt das Emissionsspektrum von Beispiel 1 (10 % in PMMA). Das Emissionsmaximum liegt bei 392 nm. Die Photolumineszenzquantenausbeute (PLQY) beträgt 34 %.

## Beispiel 2

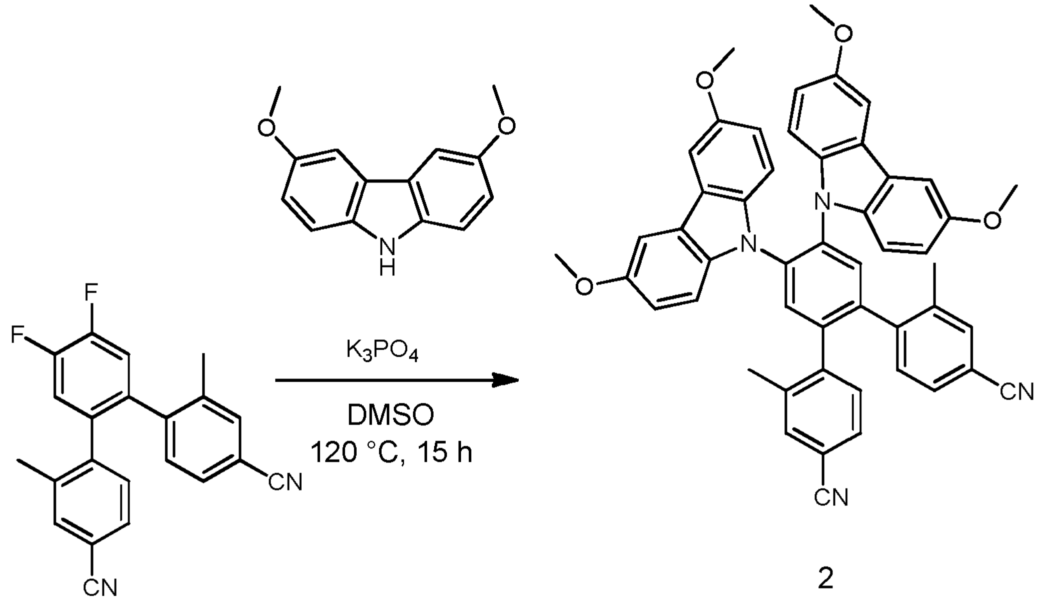


2

Beispiel **2** wurde nach AAV2 hergestellt.

Dünnschichtchromatografie: $R_f$ = 0,3 (Cyclohexan/Ethylacetat 5:1)

Figur 2 zeigt das Emissionsspektrum von Beispiel **2** (10 % in PMMA). Das Emissionsmaximum liegt bei 451 nm. Die Photolumineszenzquantenausbeute (PLQY) beträgt 52 %.

## Beispiel 3

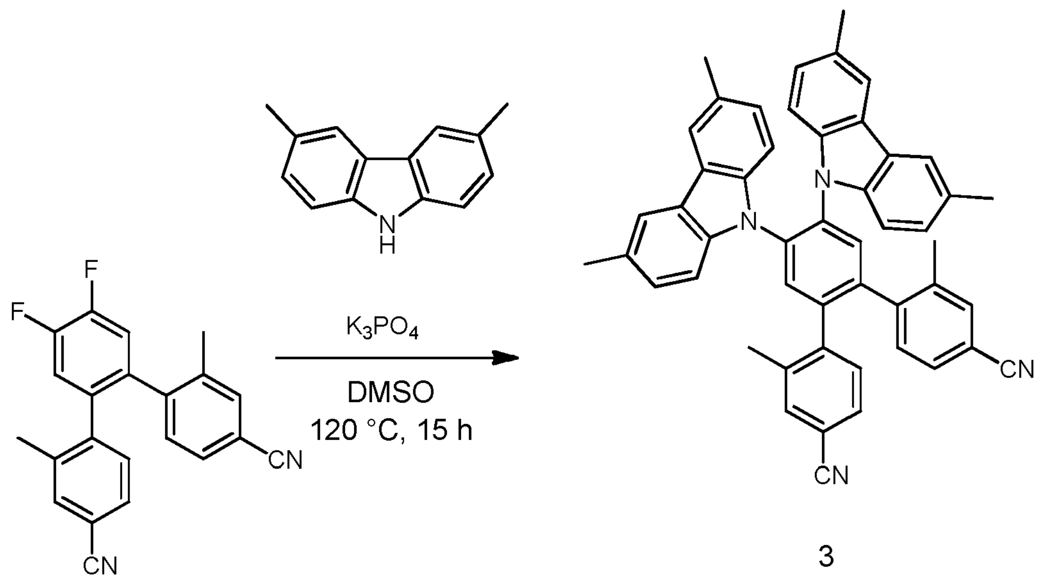


3

Beispiel **3** wurde nach AAV2 hergestellt.

Dünnschichtchromatografie: $R_f$ = 0,5 (Cyclohexan/Ethylacetat 5:1)

Figur 3 zeigt das Emissionsspektrum von Beispiel 3 (10 % in PMMA). Das Emissionsmaximum liegt bei 416 nm. Die Photolumineszenzquantenausbeute (PLQY) beträgt 35 %.

## Beispiel 4

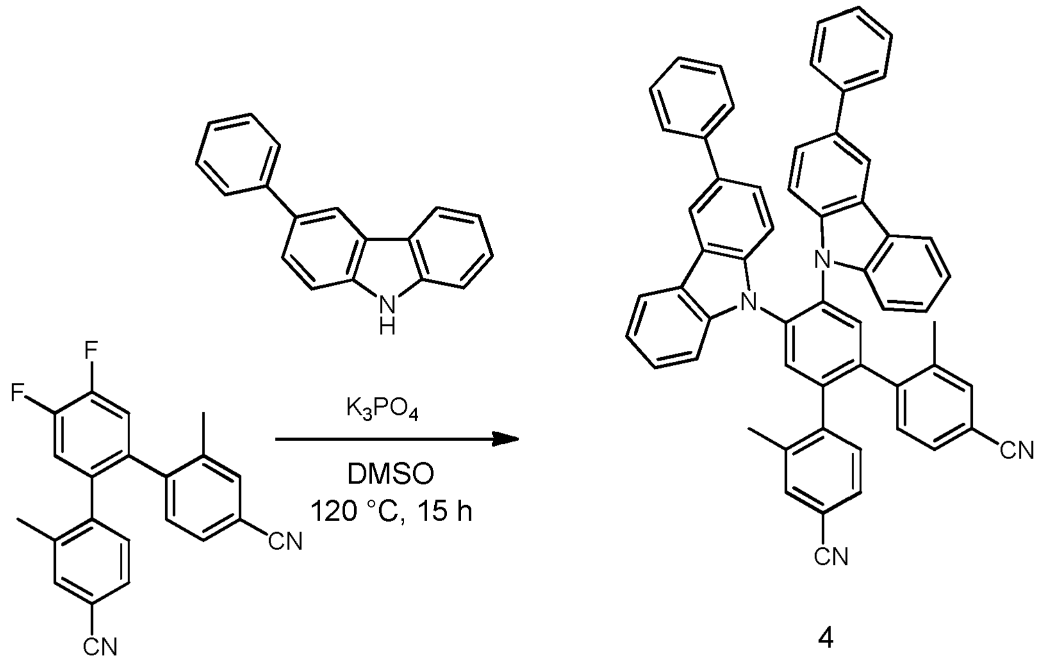


4

Beispiel 4 wurde nach AAV2 hergestellt.

Dünnschichtchromatografie: $R_f$ = 0,3 (Cyclohexan/Ethylacetat 5:1)

Figur 4 zeigt das Emissionsspektrum von Beispiel **4** (10 % in PMMA). Das Emissionsmaximum liegt bei 406 nm. Die Photolumineszenzquantenausbeute (PLQY) beträgt 32 %.

## Beispiel 5

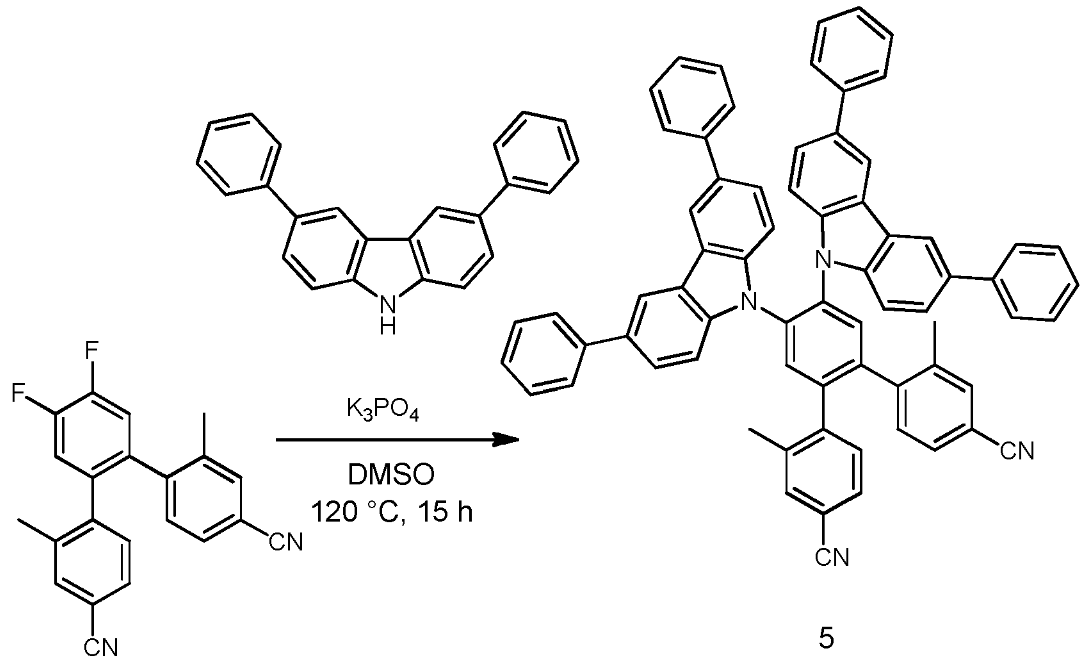


5

Beispiel 5 wurde nach AAV2 hergestellt.

Dünnschichtchromatografie: $R_f$ = 0,1 (Cyclohexan/Ethylacetat 10:1)

Figur 5 zeigt das Emissionsspektrum von Beispiel **5** (10 % in PMMA). Das Emissionsmaximum liegt bei 407 nm. Die Photolumineszenzquantenausbeute (PLQY) beträgt 31 %.

## Beispiel 6

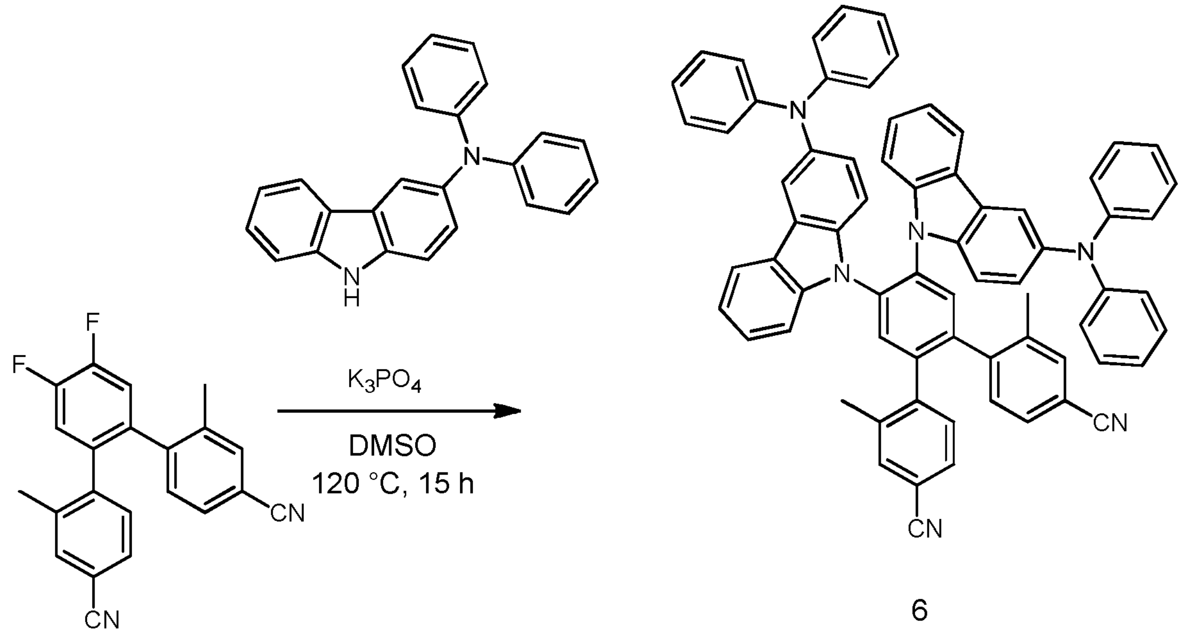


6

Beispiel 6 wurde nach AAV2 hergestellt.

Dünnschichtchromatografie: $R_f$ = 0,3 (Cyclohexan/Ethylacetat 5:1)

Figur 6 zeigt das Emissionsspektrum von Beispiel **6** (10 % in PMMA). Das Emissionsmaximum liegt bei 478 nm. Die Photolumineszenzquantenausbeute (PLQY) beträgt 41 % und die Emissionslebensdauer beträgt 103 µs.

## Beispiel 7

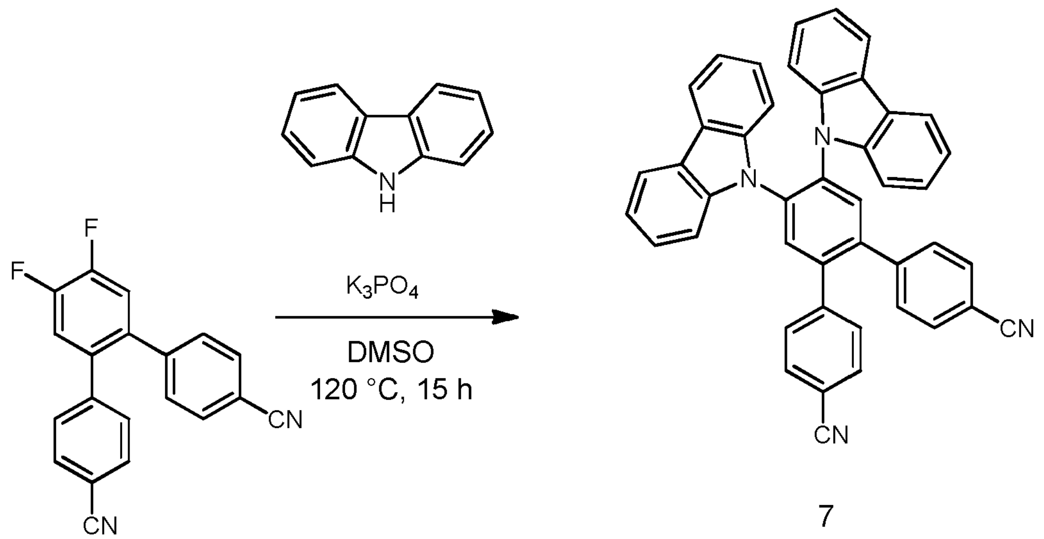


7

Beispiel 7 wurde nach AAV2 hergestellt.

Dünnschichtchromatografie: $R_f$ = 0,3 (Cyclohexan/Ethylacetat 5:1)

Figur 7 zeigt das Emissionsspektrum von Beispiel **7** (10 % in PMMA). Das Emissionsmaximum liegt bei 422 nm. Die Photolumineszenzquantenausbeute (PLQY) beträgt 45 % und die Halbwertsbreite (FWHM) 66 nm.

## Beispiel 8

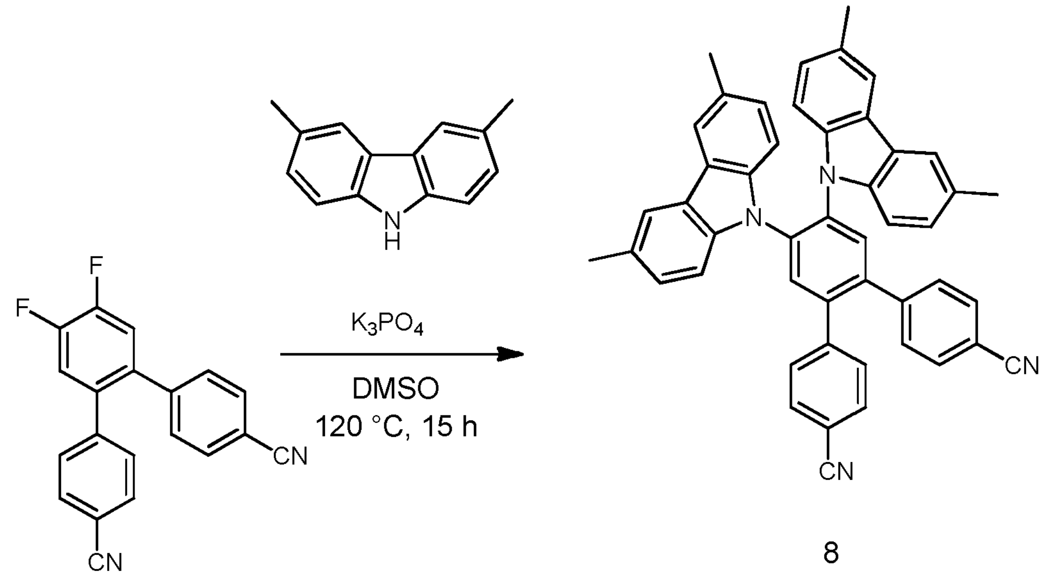


8

Beispiel **8** wurde nach AAV2 hergestellt.

Dünnschichtchromatografie: $R_f$ = 0,6 (Cyclohexan/Ethylacetat 5:1)

Figur 8 zeigt das Emissionsspektrum von Beispiel **8** (10 % in PMMA). Das Emissionsmaximum liegt bei 446 nm. Die Photolumineszenzquantenausbeute (PLQY) beträgt 42 % und die Halbwertsbreite (FWHM) 77 nm.

## Beispiel 9

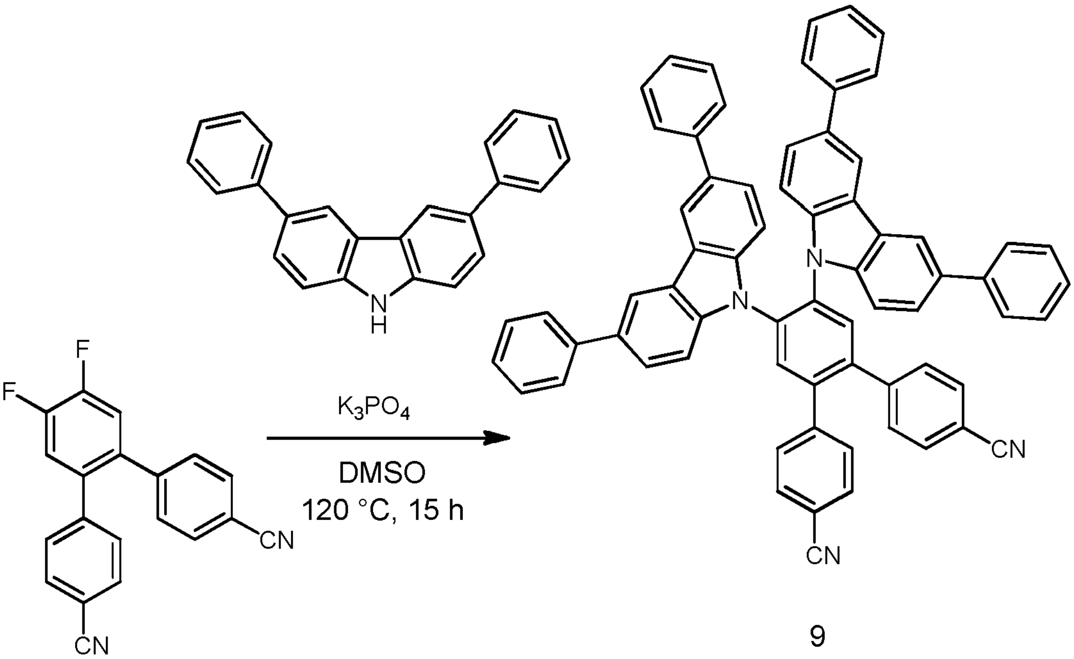


9

Beispiel 9 wurde nach AAV2 hergestellt.

Dünnschichtchromatografie: $R_f$ = 0,4 (Cyclohexan/Ethylacetat 5:1)

Figur 9 zeigt das Emissionsspektrum von Beispiel 9 (10 % in PMMA). Das Emissionsmaximum liegt bei 442 nm. Die Photolumineszenzquantenausbeute (PLQY) beträgt 40 % und die Halbwertsbreite (FWHM) 75 nm.

## Beispiel 10

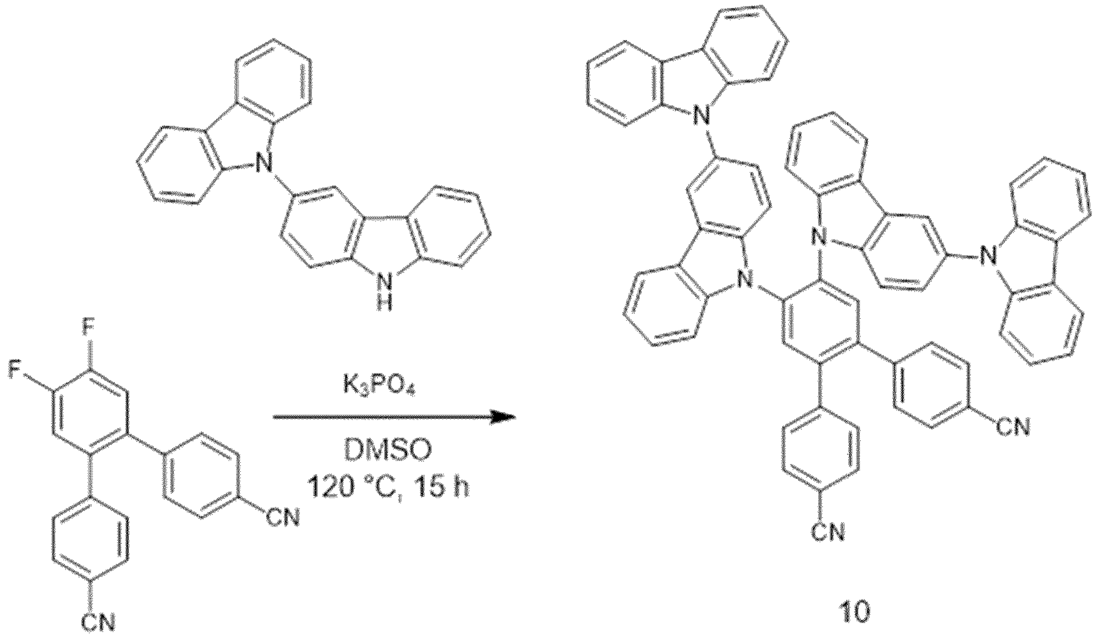


10

Beispiel 10 wurde nach AAV2 hergestellt.

Figur 10 zeigt das Emissionsspektrum von Beispiel 10 (10% in PMMA). Das Emissionsmaximum liegt bei 441 nm. Die Photolumineszenzquantenausbeute (PLQY) beträgt 39 % und die Halbwertsbreite (FWHM) 86 nm.

## Beispiel 11

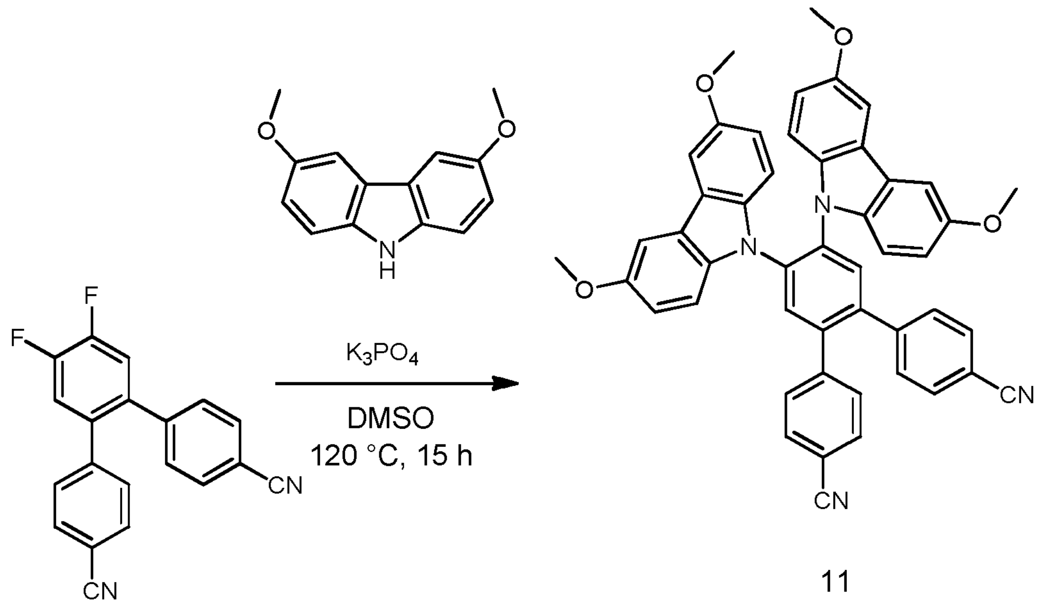


11

Beispiel 11 wurde nach AAV2.

Figur 11 zeigt das Emissionsspektrum von Beispiel **11** (10% in PMMA). Das Emissionsmaximum liegt bei 480 nm. Die Photolumineszenzquantenausbeute (PLQY) beträgt 62 % und die Halbwertsbreite (FWHM) 98 nm.

## Beispiel 12

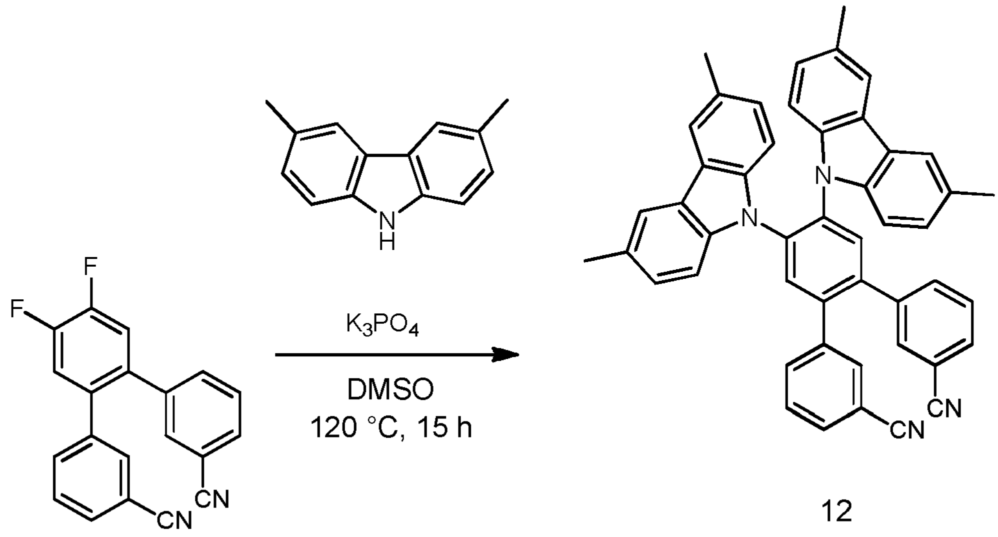


12

Beispiel 12 wurde nach AAV2 hergestellt.

Dünnschichtchromatografie: $R_f$ = 0,3 (Cyclohexan/Ethylacetat 5:1)

Figur 12 zeigt das Emissionsspektrum von Beispiel **12** (10 % in PMMA). Das Emissionsmaximum liegt bei 411 nm. Die Photolumineszenzquantenausbeute (PLQY) beträgt 22 % und die Halbwertsbreite (FWHM) 66 nm.

## Beispiel 13

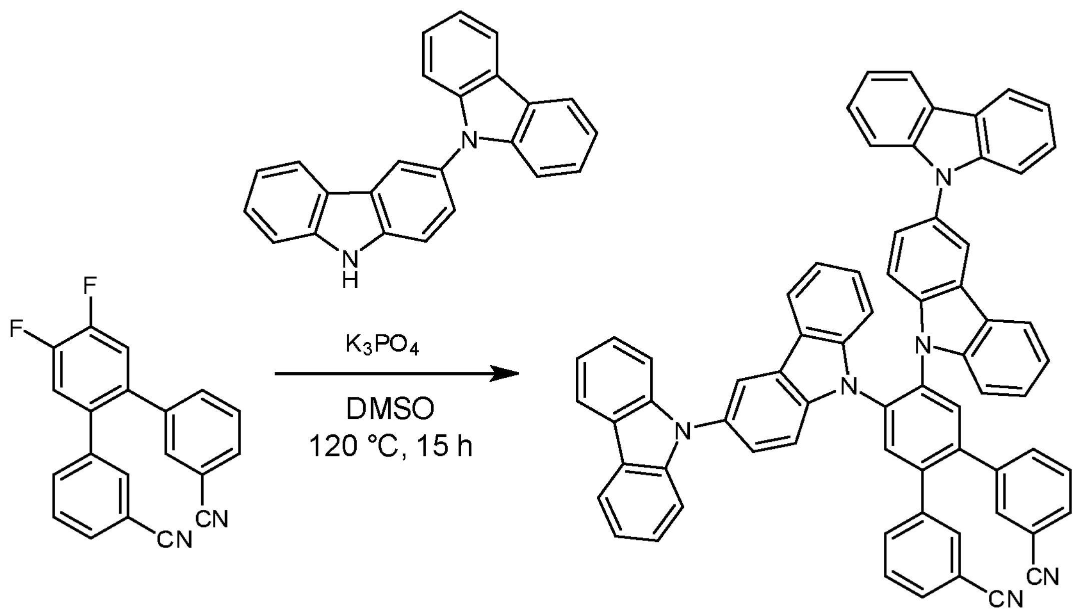


13

Beispiel **13** wurde nach AAV2 hergestellt.

Dünnschichtchromatografie: $R_f$ = 0,3 (Cyclohexan/Ethylacetat 5:1)

Figur 13 zeigt das Emissionsspektrum von Beispiel **13** (10% in PMMA). Das Emissionsmaximum liegt bei 415 nm. Die Photolumineszenzquantenausbeute (PLQY) beträgt 40 % und die Halbwertsbreite (FWHM) 75 nm.

**Beispiel 14** (nicht erfindungsgemäß)

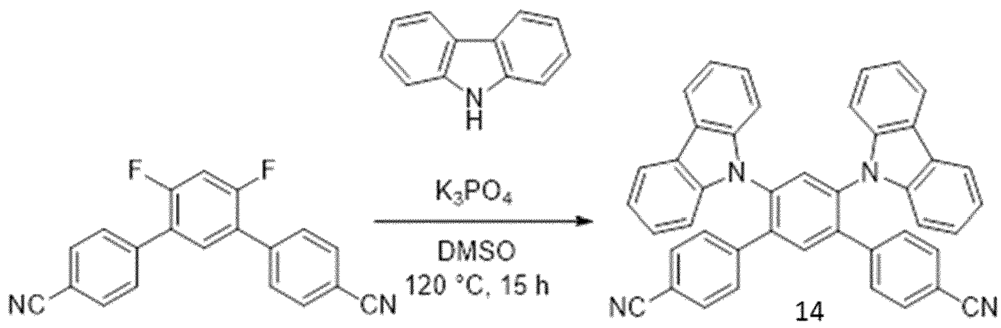


14

Beispiel **14** wurde nach AAV2 hergestellt.

Dünnschichtchromatografie: $R_f$ = 0,3 (Cyclohexan/Ethylacetat 5:1)

Figur 14 zeigt das Emissionsspektrum von Beispiel **14** (10 % in PMMA). Das Emissionsmaximum liegt bei 412 nm. Die Photolumineszenzquantenausbeute (PLQY) beträgt 28 % und die Halbwertsbreite (FWHM) 57 nm.

**Beispiel 15** (nicht erfindungsgemäß)

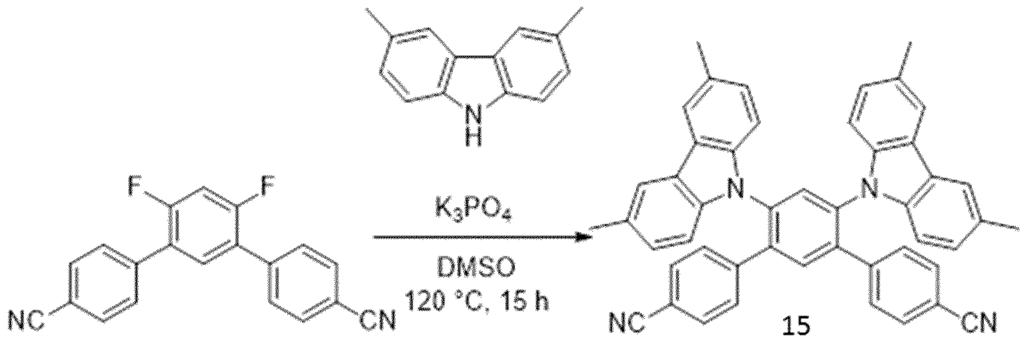


15

Beispiel **15** wurde nach AAV1 hergestellt.

Dünnschichtchromatografie: $R_f$ = 0,3 (Cyclohexan/Ethylacetat 5:1)

Figur 15 zeigt das Emissionsspektrum von Beispiel **15** (10% in PMMA). Das Emissionsmaximum liegt bei 436 nm. Die Photolumineszenzquantenausbeute (PLQY) beträgt 31 % und die Halbwertsbreite (FWHM) 69 nm.

**Beispiel 16** (nicht erfindungsgemäß)

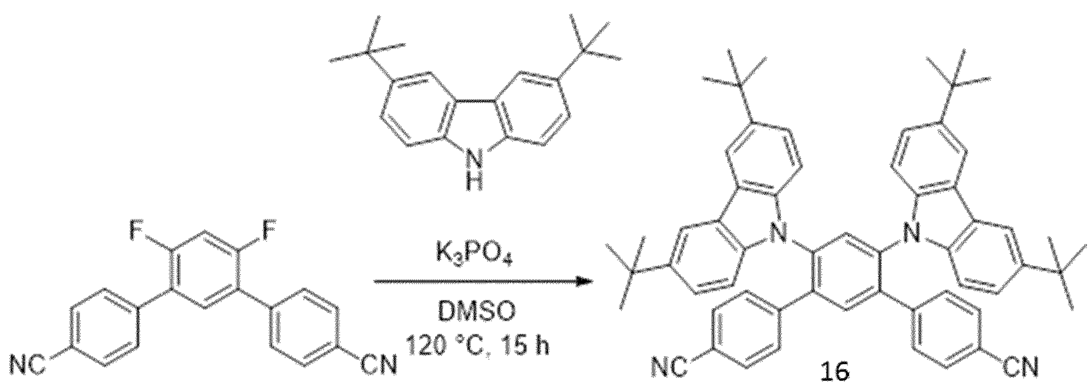


16

Beispiel **16** wurde nach AAV2 hergestellt.

Dünnschichtchromatografie: $R_f$ = 0,5 (Cyclohexan/Ethylacetat 5:1)

Figur 16 zeigt das Emissionsspektrum von Beispiel 16 (10 % in PMMA). Das Emissionsmaximum liegt bei 429 nm. Die Photolumineszenzquantenausbeute (PLQY) beträgt 34 % und die Halbwertsbreite (FWHM) 65 nm.

## **Beispiel 17** (nicht erfindungsgemäß)

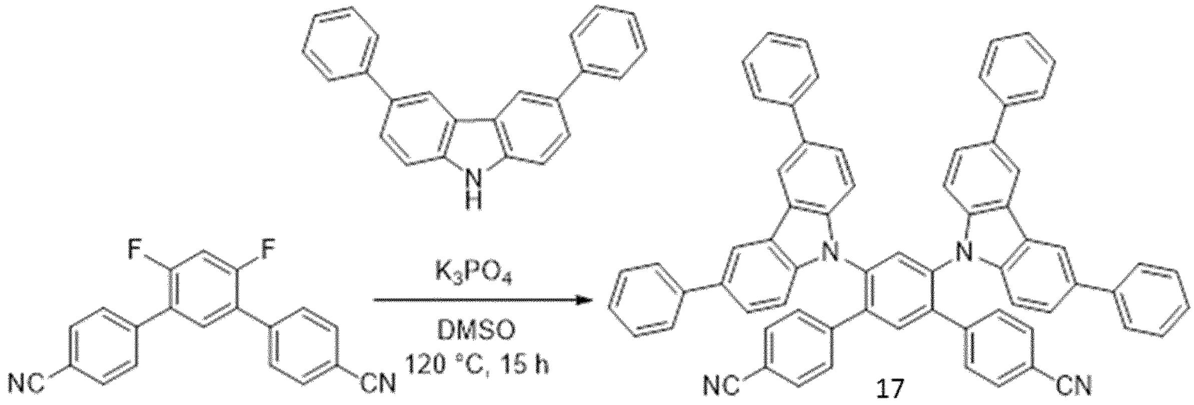


17

Beispiel **17** wurde nach AAV2 hergestellt.

Dünnschichtchromatografie: $R_f$ = 0,3 (Cyclohexan/Ethylacetat 5:1)

Figur 17 zeigt das Emissionsspektrum von Beispiel **17** (10% in PMMA). Das Emissionsmaximum liegt bei 437 nm. Die Photolumineszenzquantenausbeute (PLQY) beträgt 30 % und die Halbwertsbreite (FWHM) 69 nm.

## **Beispiel 18** (nicht erfindungsgemäß)

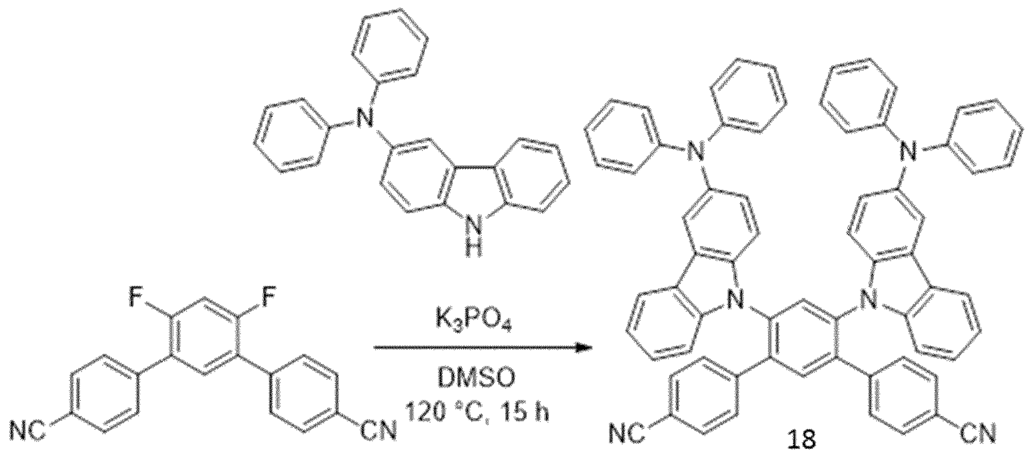


18

Beispiel **18** wurde nach AAV2 hergestellt.

Dünnschichtchromatografie: $R_f$ = 0,4 (Cyclohexan/Ethylacetat 5:1)

Figur 18 zeigt das Emissionsspektrum von Beispiel **18** (10% in PMMA). Das Emissionsmaximum liegt bei 507 nm. Die Photolumineszenzquantenausbeute (PLQY) beträgt 54%.

## **Beispiel 19** (nicht erfindungsgemäß)

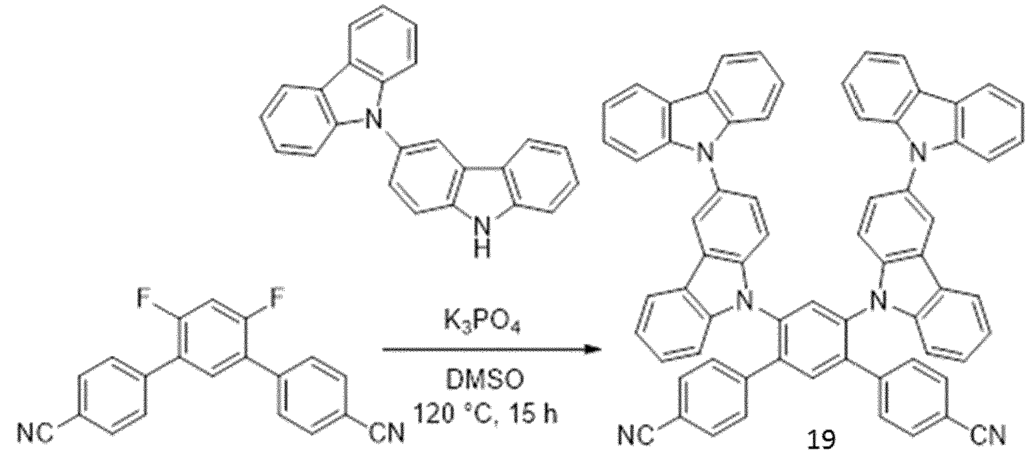


19

Beispiel **19** wurde nach AAV2 hergestellt.

Dünnschichtchromatografie: $R_f$ = 0,4 (Cyclohexan/Ethylacetat 5:1)

Figur 19 zeigt das Emissionsspektrum von Beispiel **19** (10% in PMMA). Das Emissionsmaximum liegt bei 443 nm. Die Photolumineszenzquantenausbeute (PLQY) beträgt 33 % und die Halbwertsbreite (FWHM) 86 nm.

## **Beispiel 20** (nicht erfindungsgemäß)

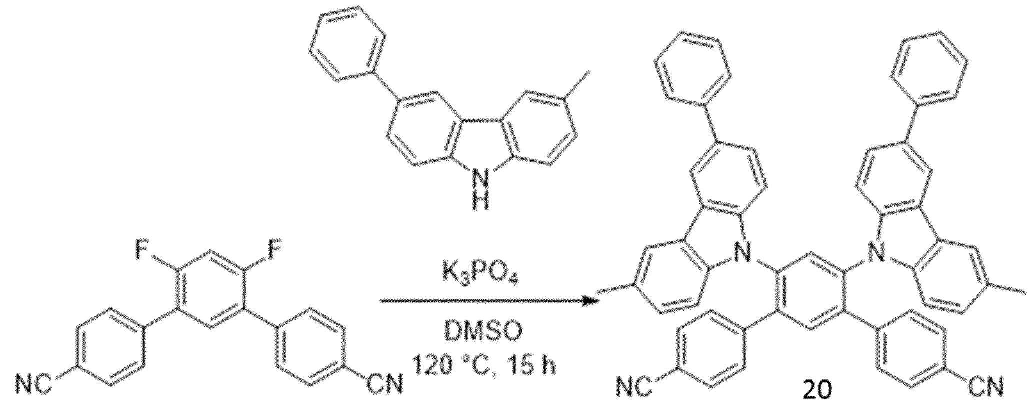


20

Beispiel **20** wurde nach AAV2 hergestellt.

Dünnschichtchromatografie: $R_f$ = 0,3 (Cyclohexan/Ethylacetat 5:1)

Figur 20 zeigt das Emissionsspektrum von Beispiel **20** (10 % in PMMA). Das Emissionsmaximum liegt bei 437 nm. Die Photolumineszenzquantenausbeute (PLQY) beträgt 30 % und die Halbwertsbreite (FWHM) 69 nm.

**Beispiel 21** (nicht erfindungsgemäß)

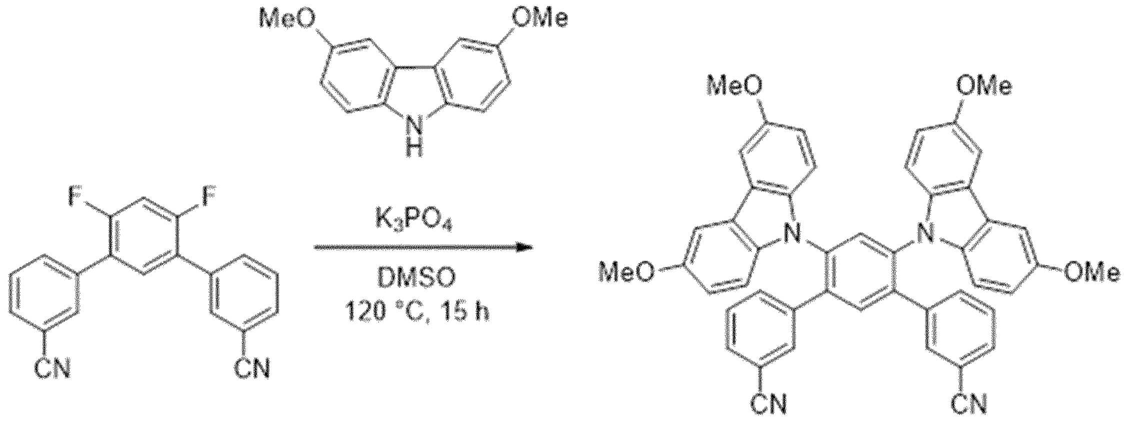


21

Beispiel 21 wurde nach AAV2 hergestellt.

Dünnschichtchromatografie: $R_f$ = 0,2 (Cyclohexan/Ethylacetat 5:1)

**Beispiel 22** (nicht erfindungsgemäß)

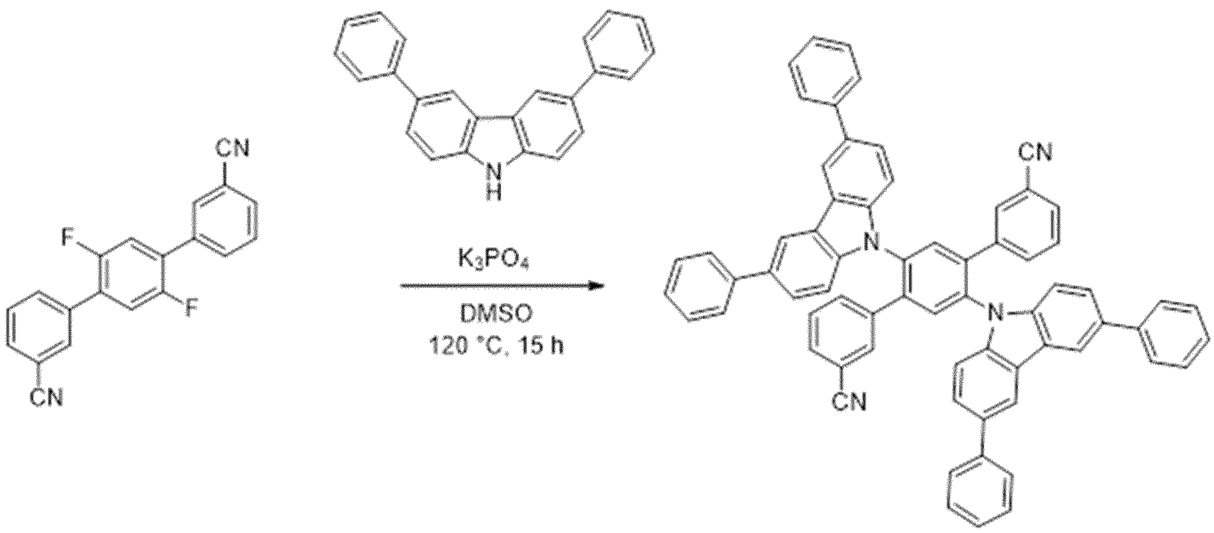


22

Beispiel **22** wurde nach AAV2 hergestellt.

## Beispiel 23 (nicht erfindungsgemäß)

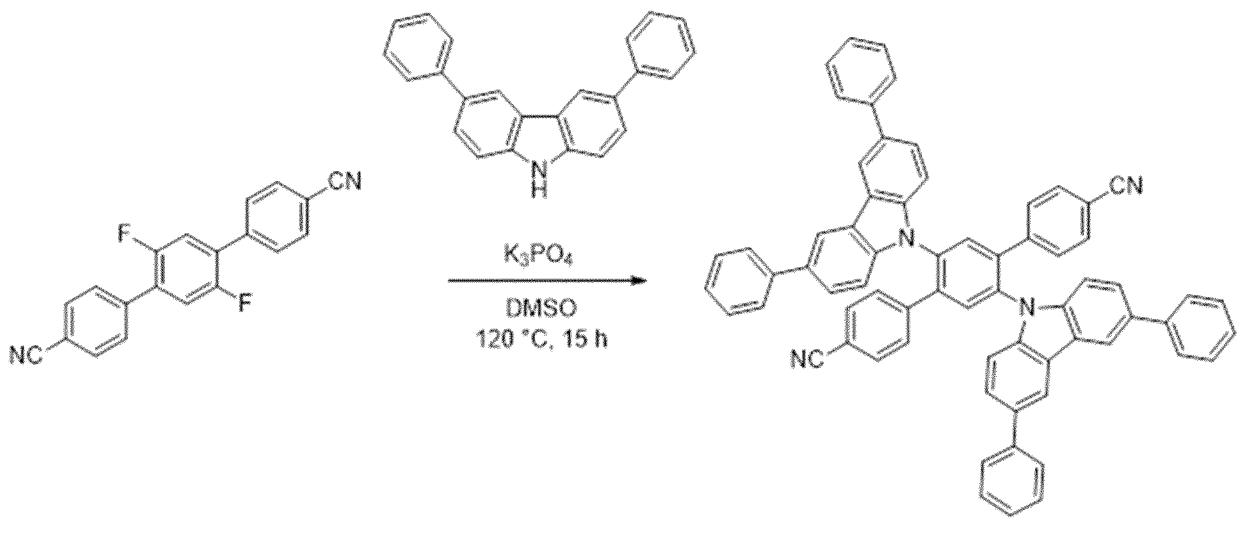


23

Beispiel **23** wurde nach AAV2 hergestellt.

Figur 21 zeigt das Emissionsspektrum von Beispiel **23** (10 % in PMMA). Das Emissionsmaximum liegt bei 455 nm. Die Photolumineszenzquantenausbeute (PLQY) beträgt 60 % und die Halbwertsbreite (FWHM) 73 nm.

## Beispiel 24 (nicht erfindungsgemäß)

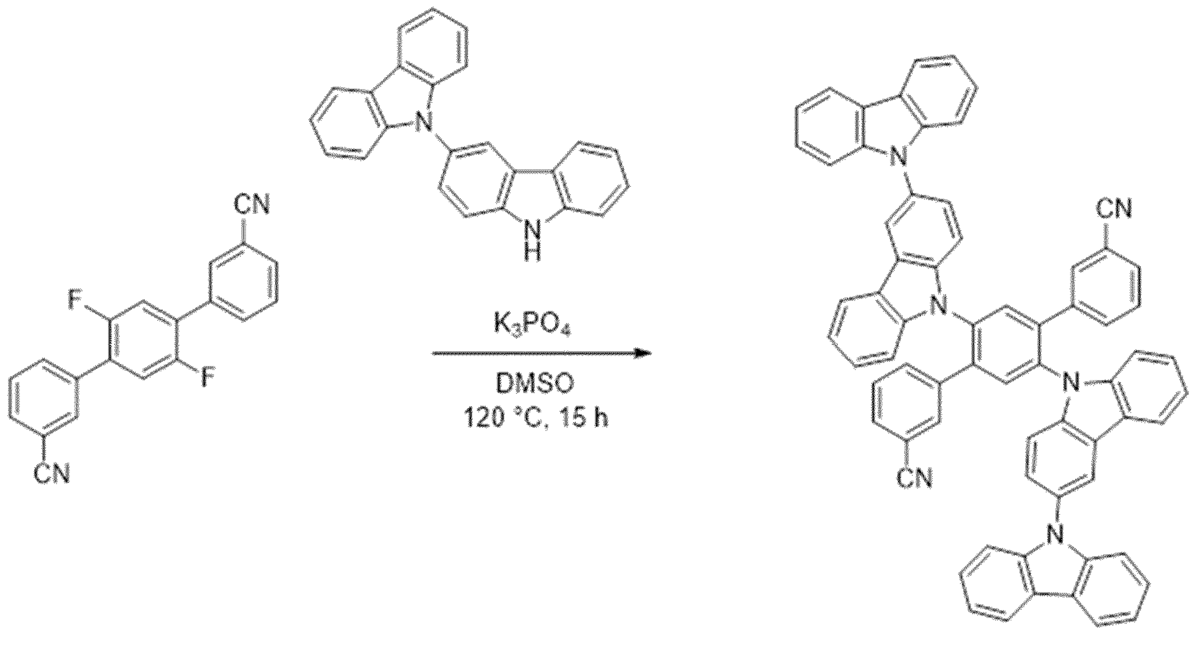


24

Beispiel **24** wurde nach AAV2 hergestellt.

Figur 22 zeigt das Emissionsspektrum von Beispiel **24** (10 % in PMMA). Das Emissionsmaximum liegt bei 423 nm. Die Photolumineszenzquantenausbeute (PLQY) beträgt 32 % und die Halbwertsbreite (FWHM) 77 nm.

**Beispiel 25** (nicht erfindungsgemäß)

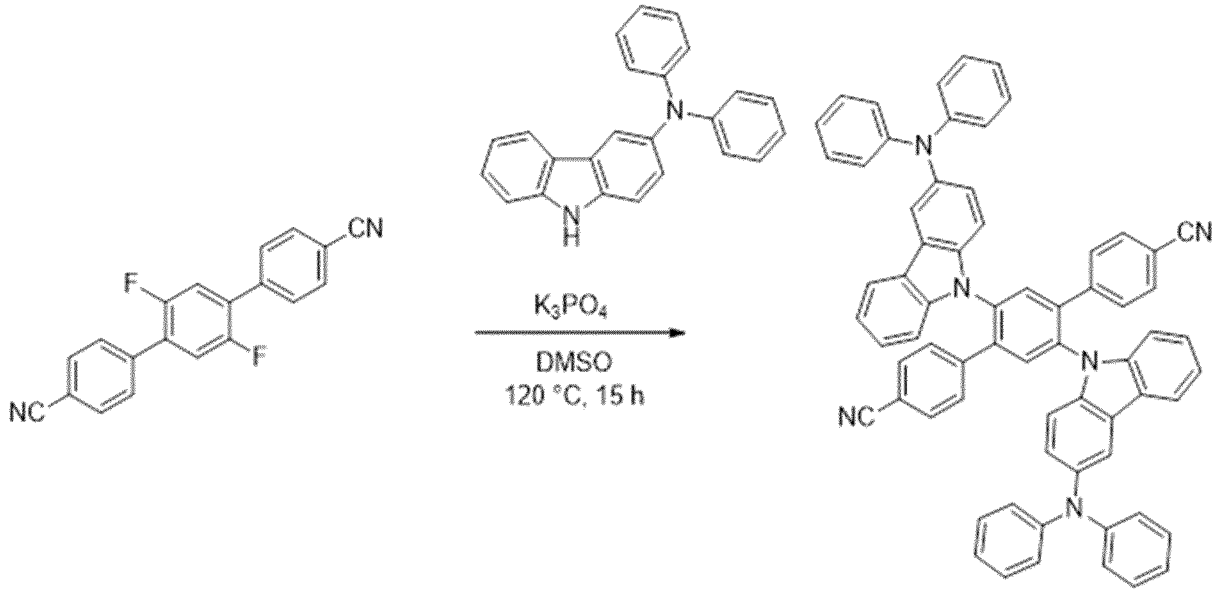


25

Beispiel **25** wurde nach AAV2 hergestellt.

Figur 23 zeigt das Emissionsspektrum von Beispiel **25** (10 % in PMMA). Das Emissionsmaximum liegt bei 524 nm. Die Photolumineszenzquantenausbeute (PLQY) beträgt 44 % und die Halbwertsbreite (FWHM) 111 nm.

**Beispiel 26** (nicht erfindungsgemäß)

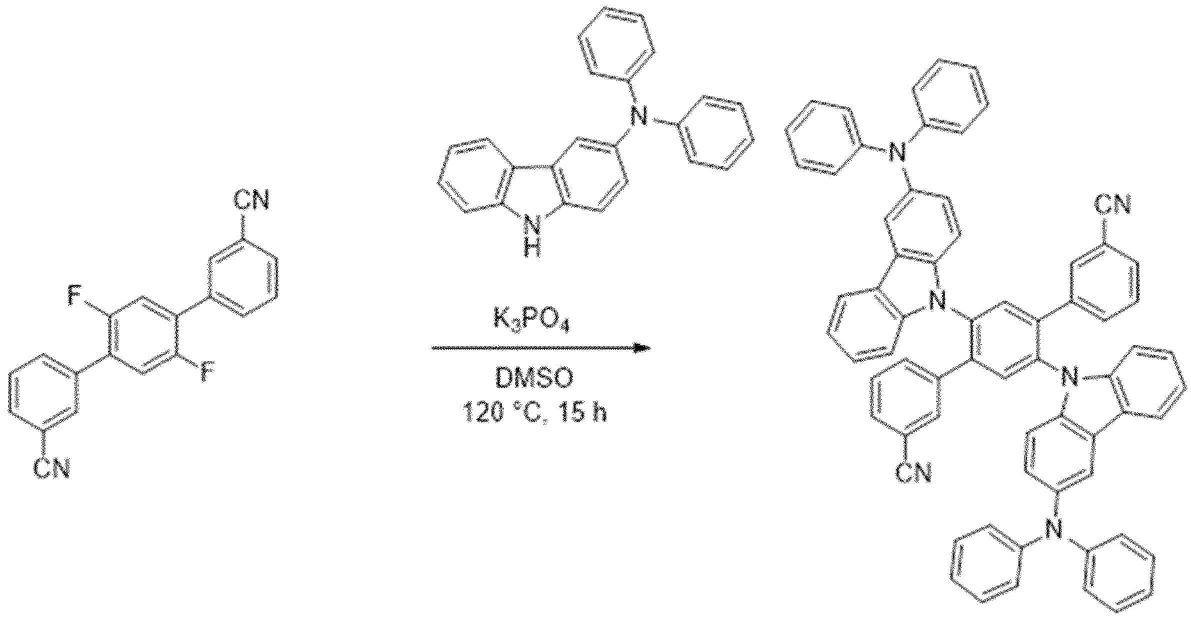


26

Beispiel **26** wurde nach AAV2 hergestellt.

Figur 24 zeigt das Emissionsspektrum von Beispiel **26** (10 % in PMMA). Das Emissionsmaximum liegt bei 495 nm. Die Photolumineszenzquantenausbeute (PLQY) beträgt 38 % und die Halbwertsbreite (FWHM) 104 nm.

Weitere Beispiele organischer Moleküle mit einer Struktur gemäß der Formeln Ia bis Ih, wobei nur die Verbindungen mit der Struktur gemäß Formel Ia erfindungsgemäß sind:

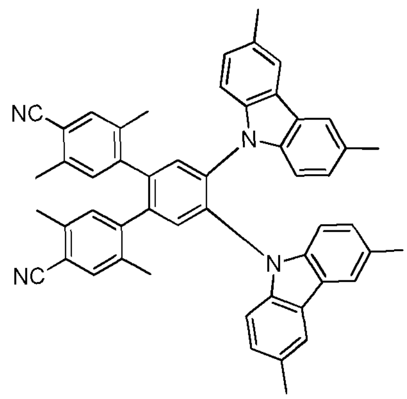
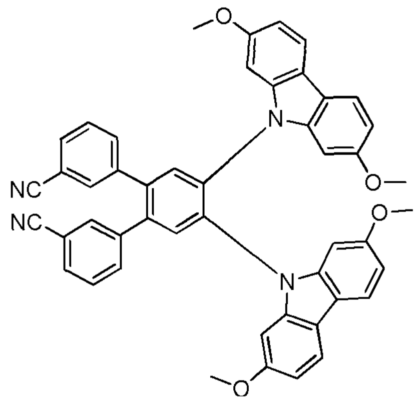
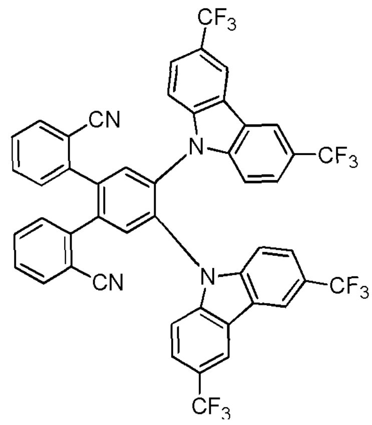
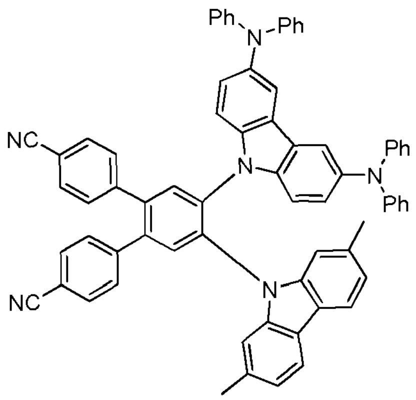
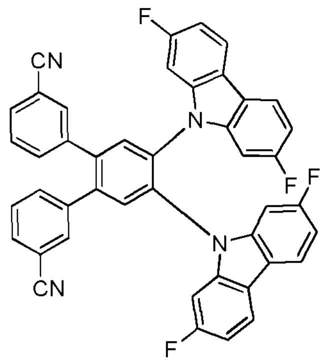
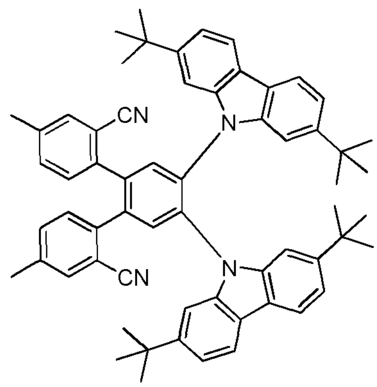
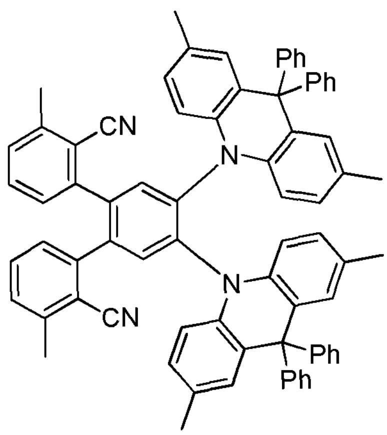
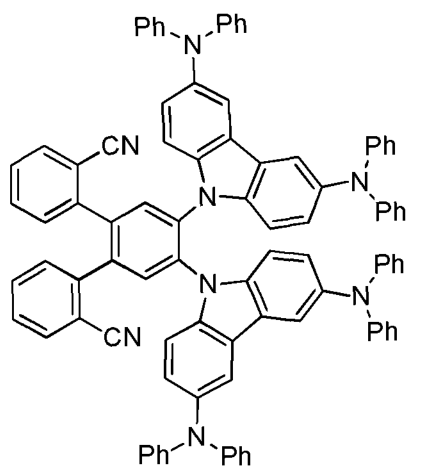

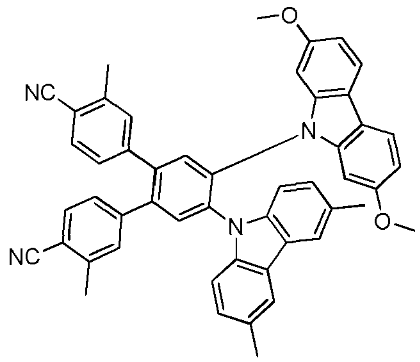

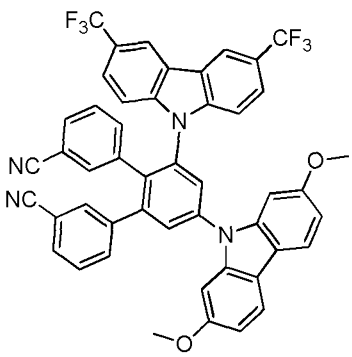

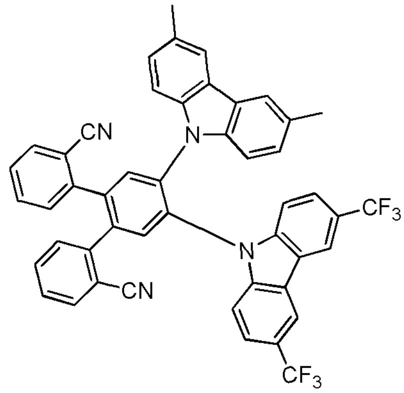

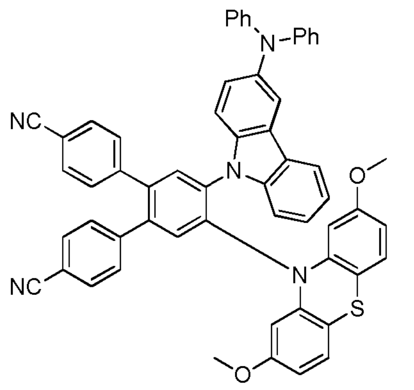

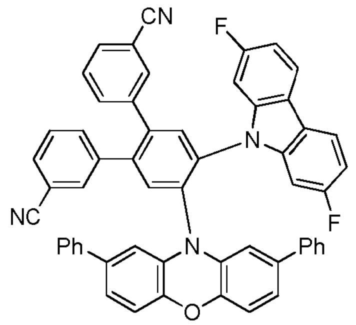

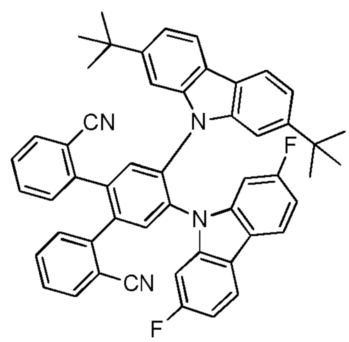

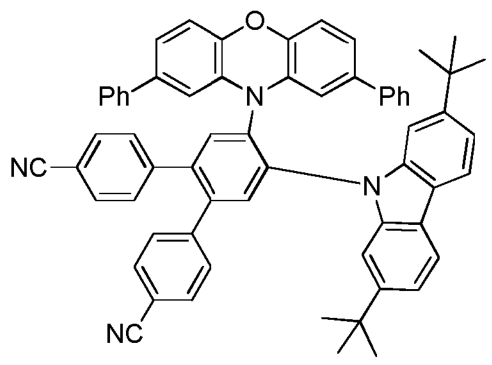

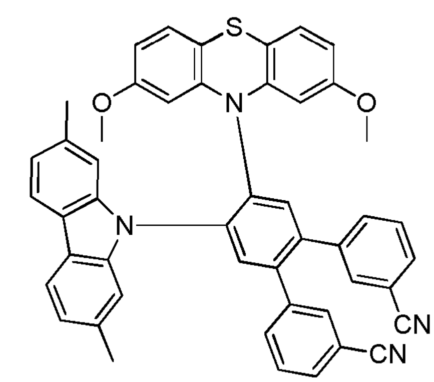

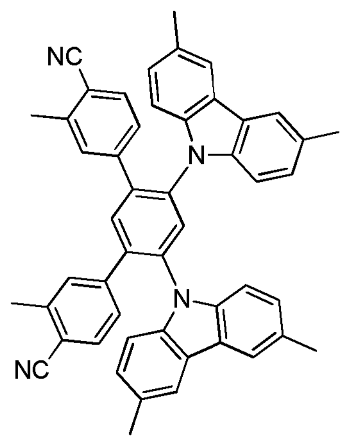

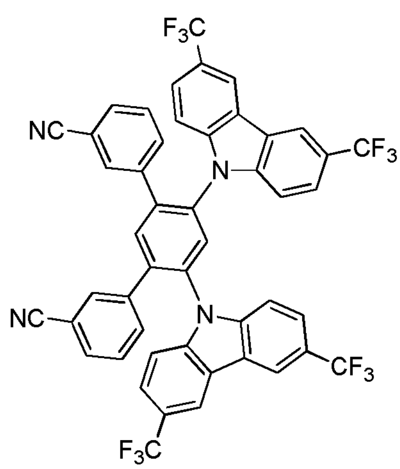

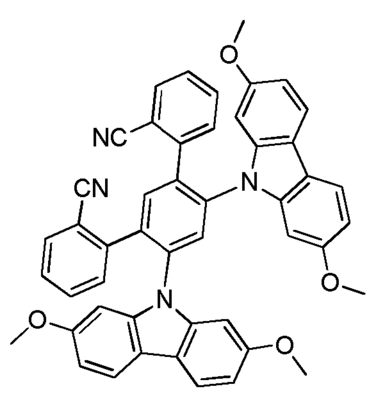

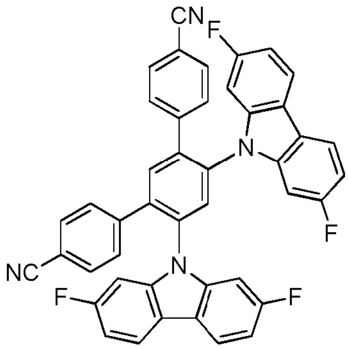

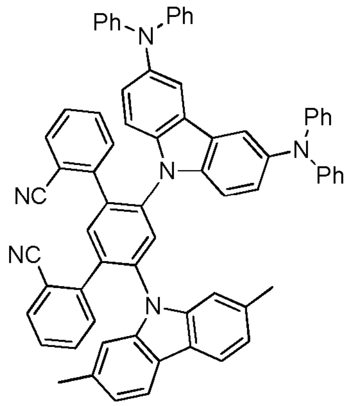

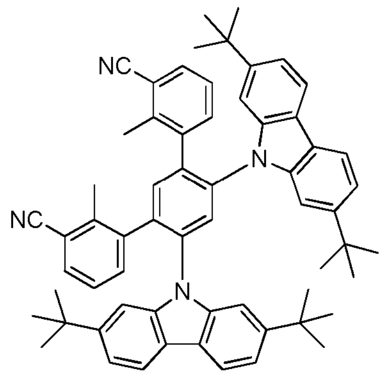

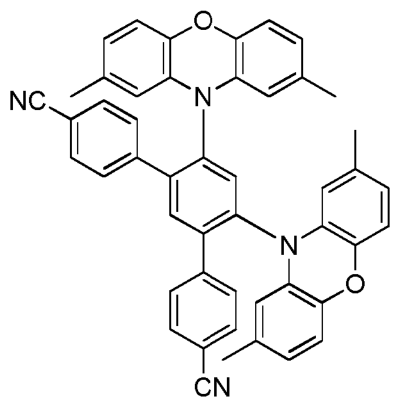

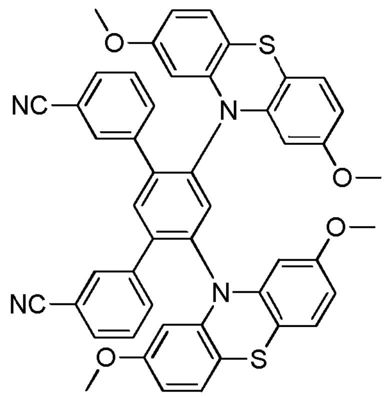

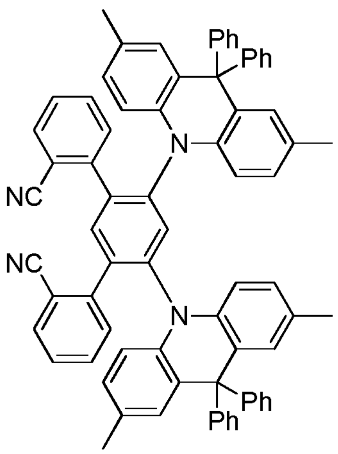

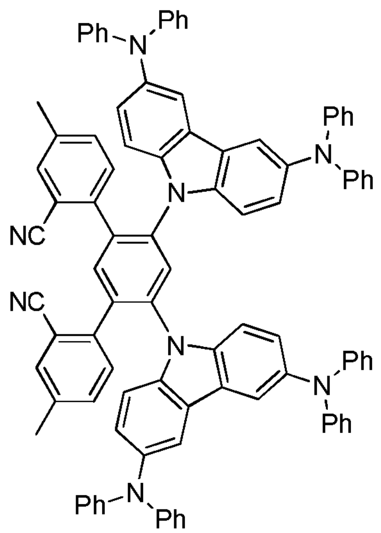

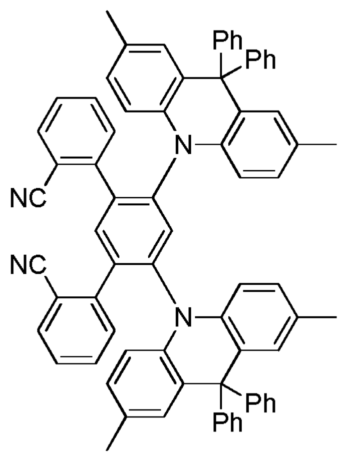

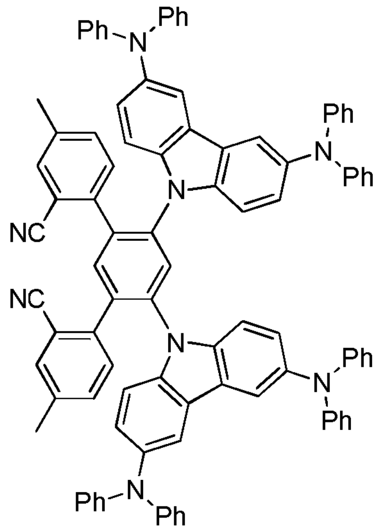

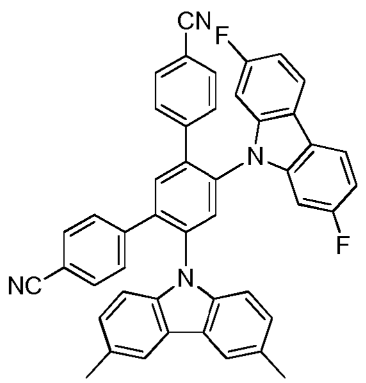

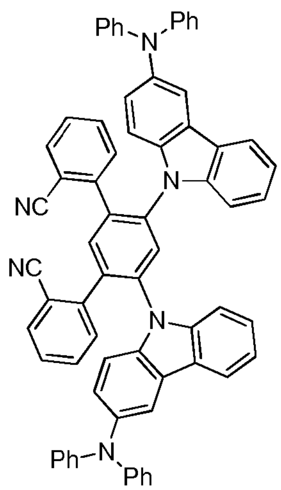

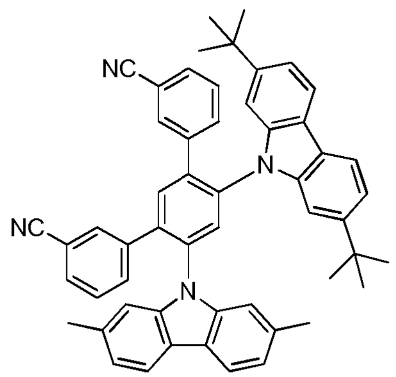

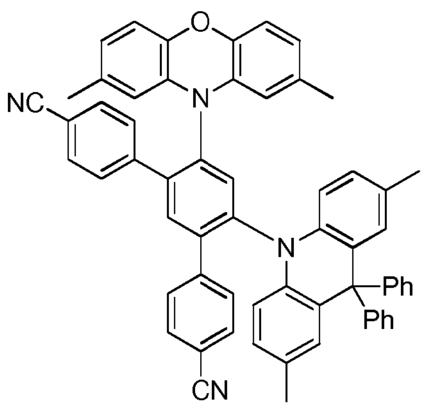

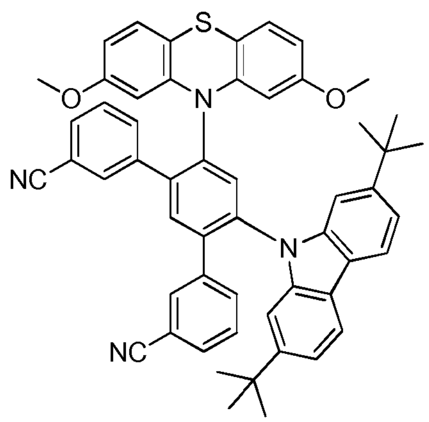

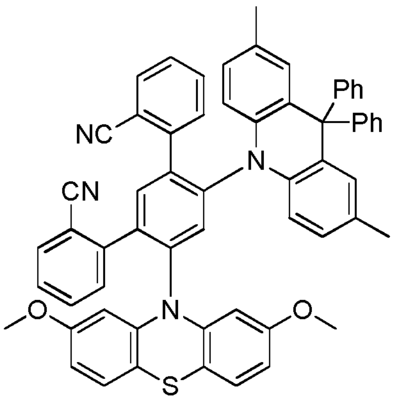

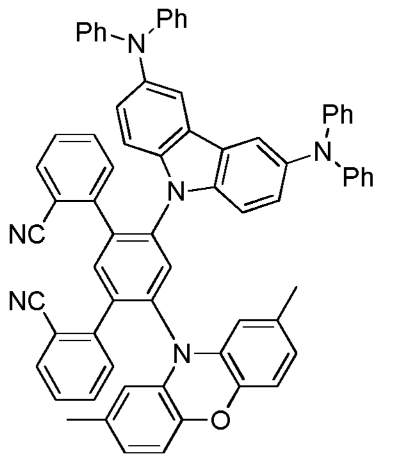

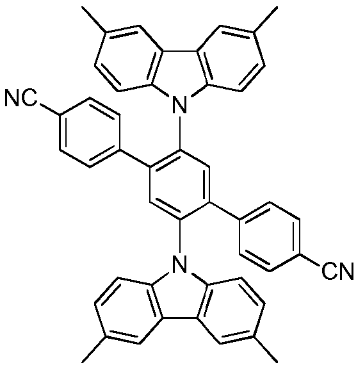

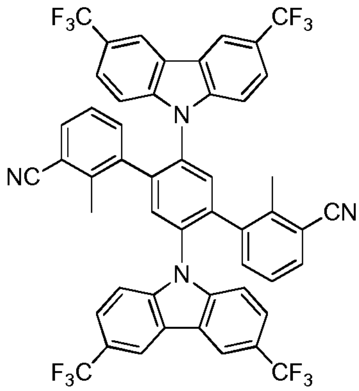

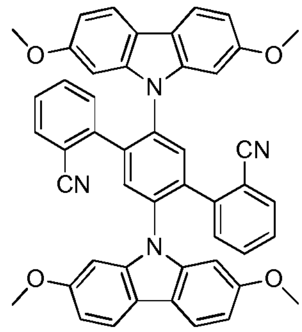

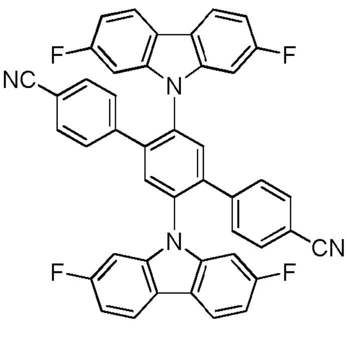

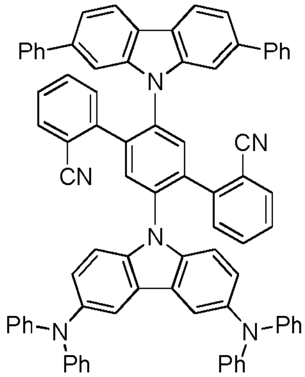

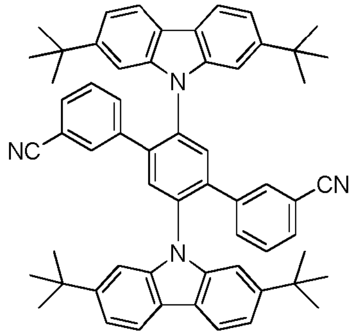

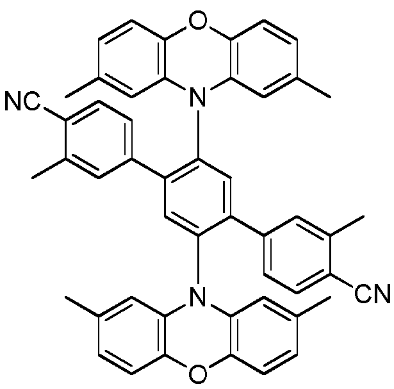

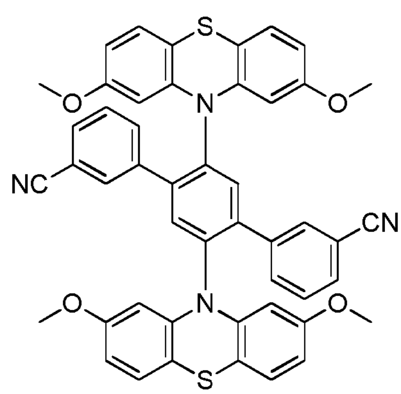

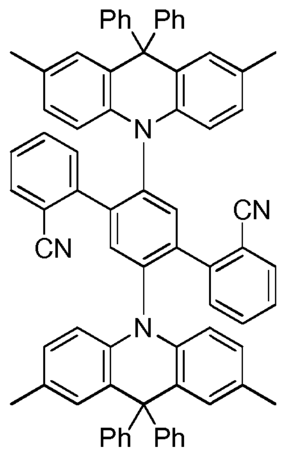

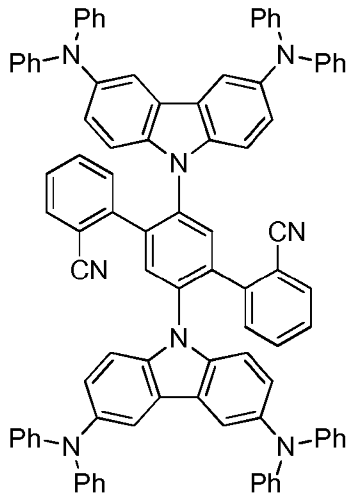

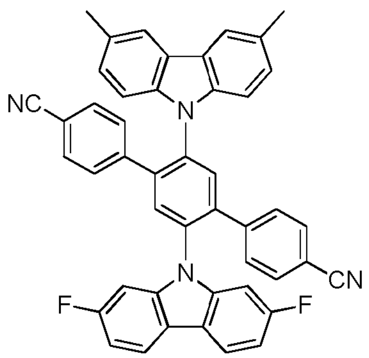

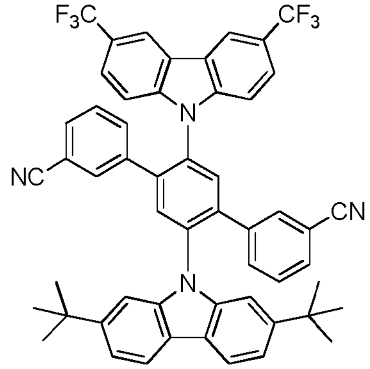

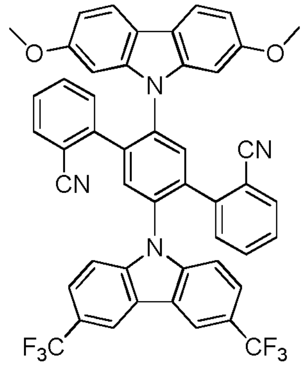

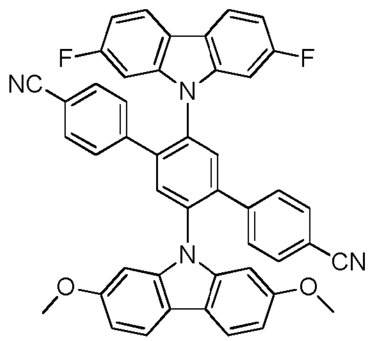

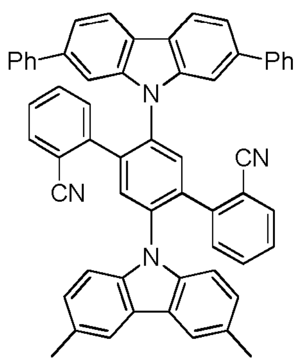

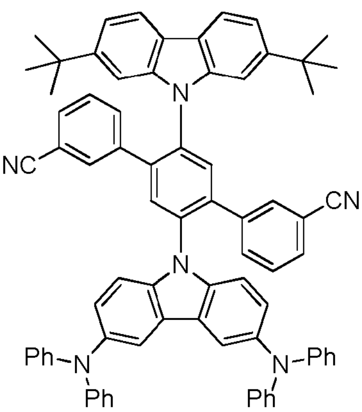

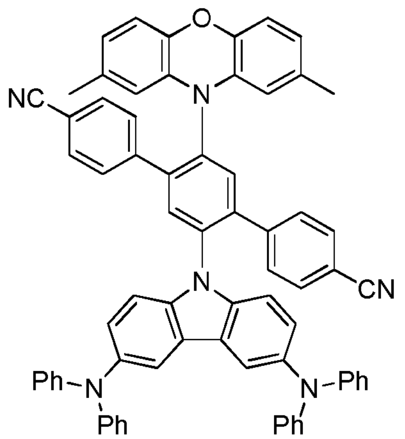

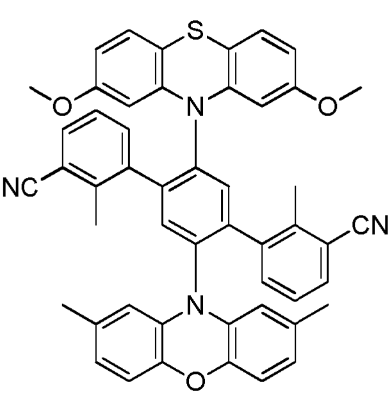

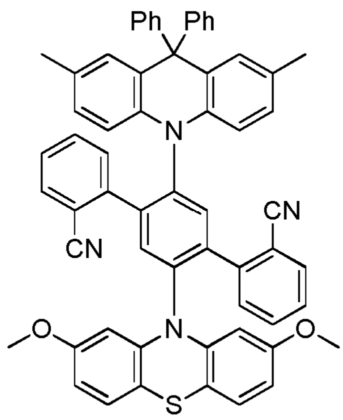

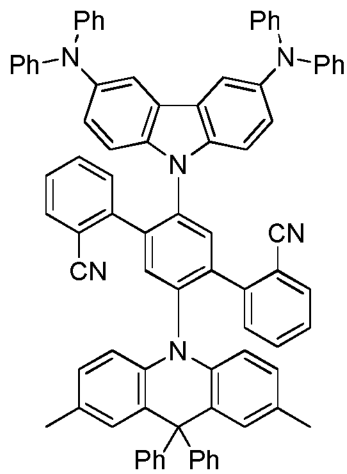

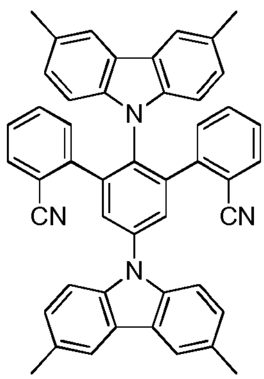

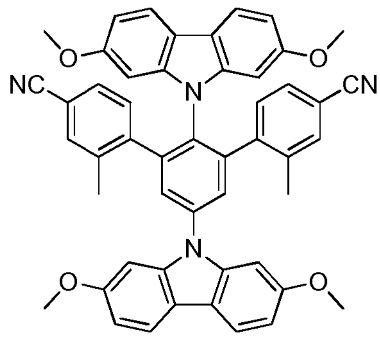

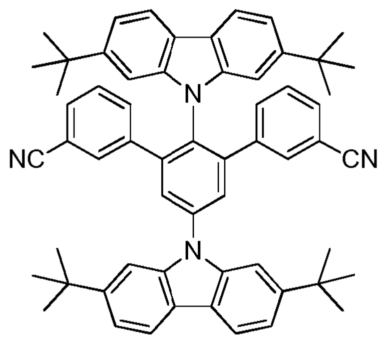

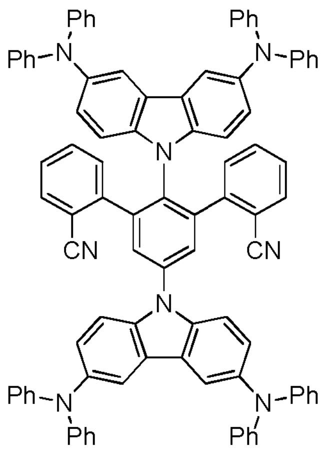

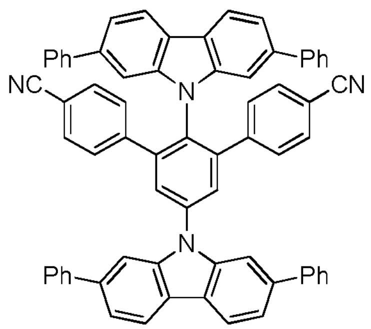

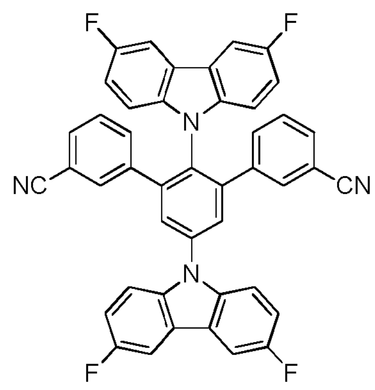

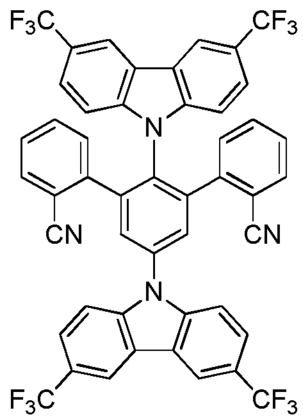

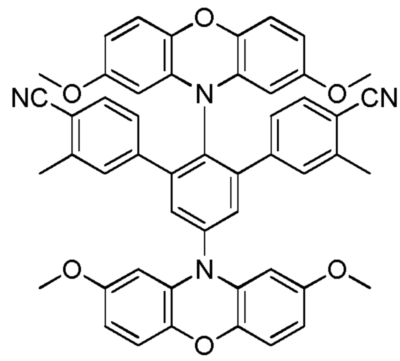

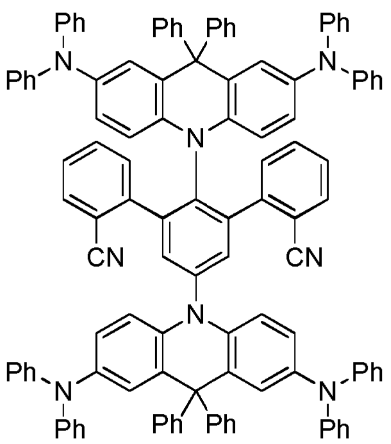

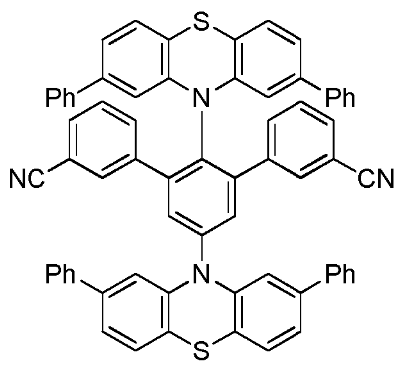

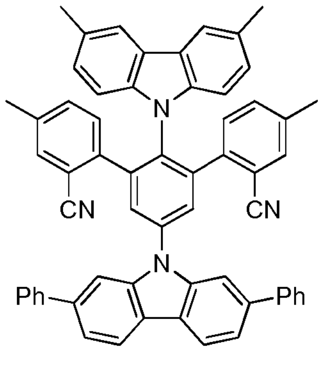

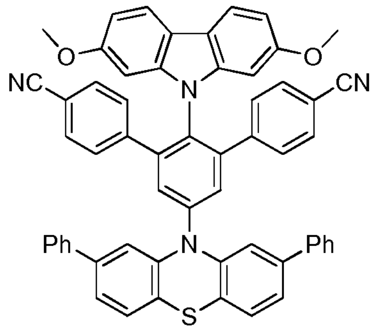

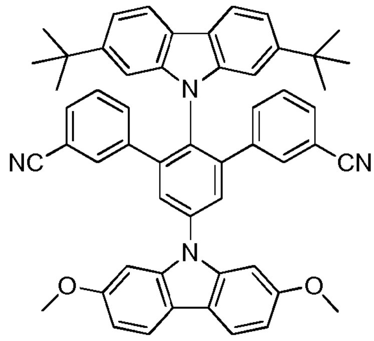

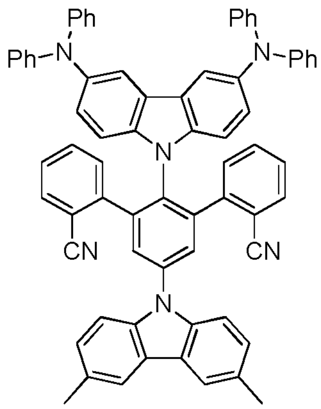

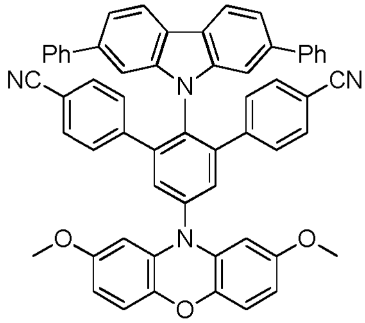

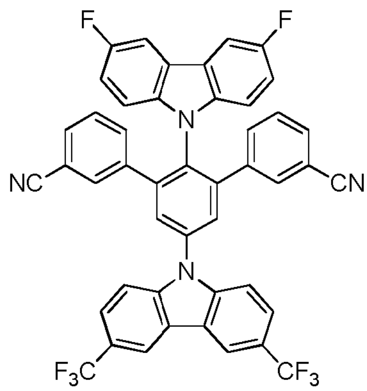

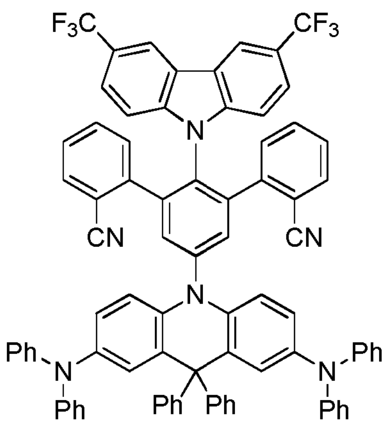

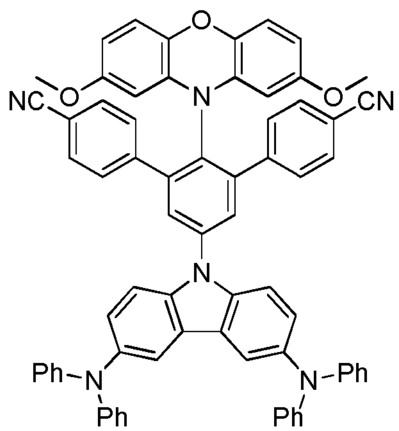

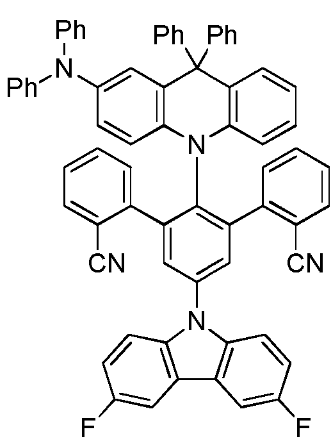

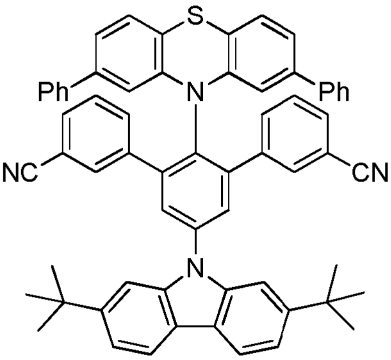

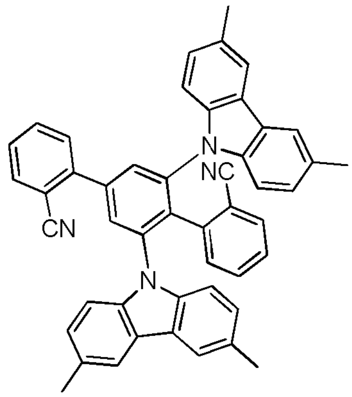

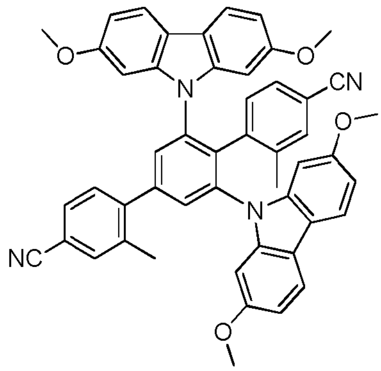

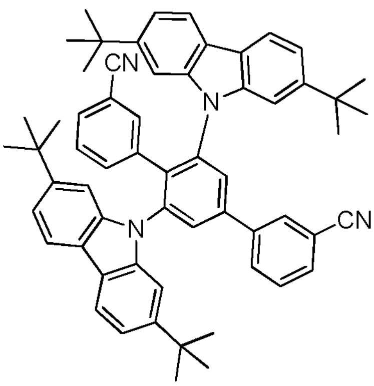

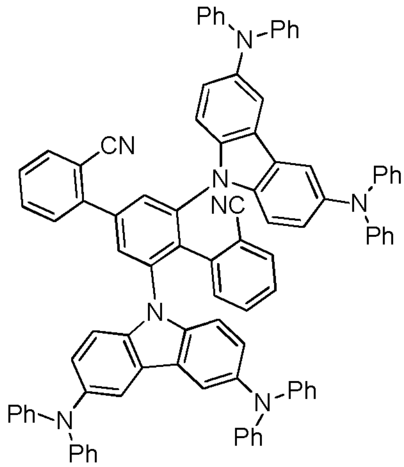

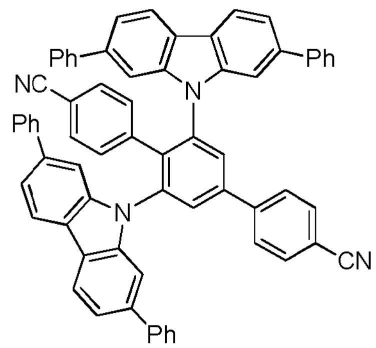

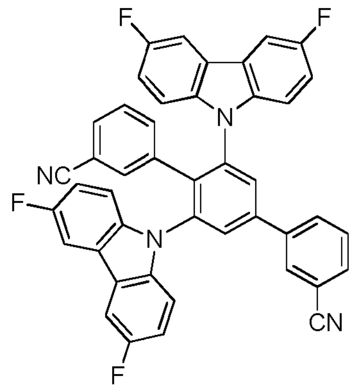

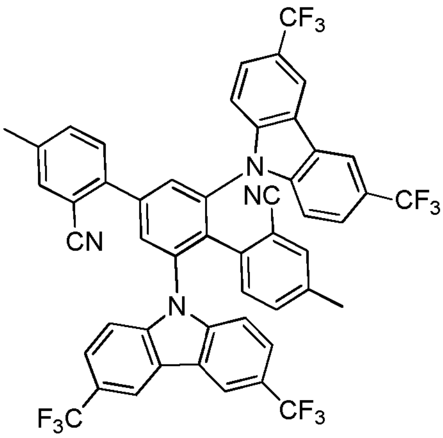

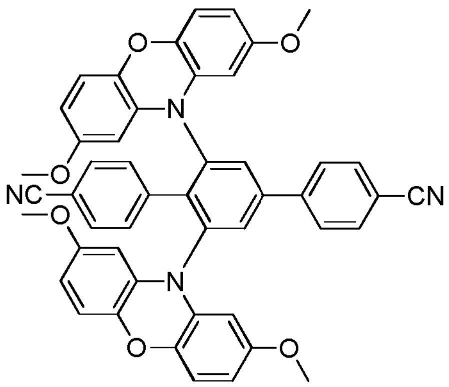

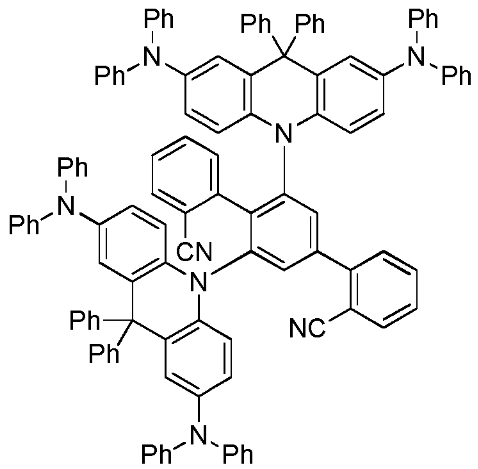

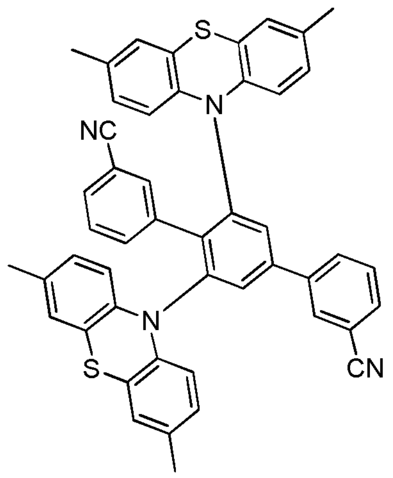

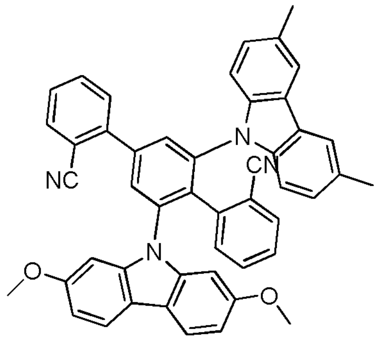

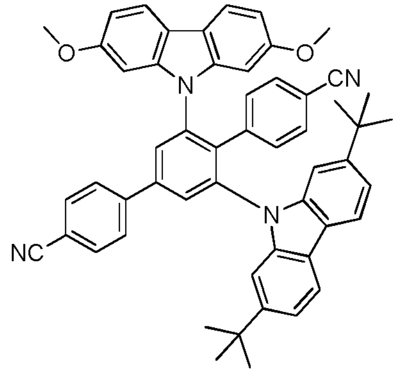

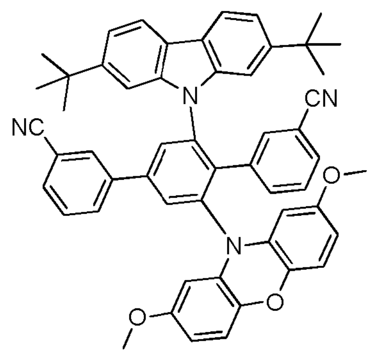

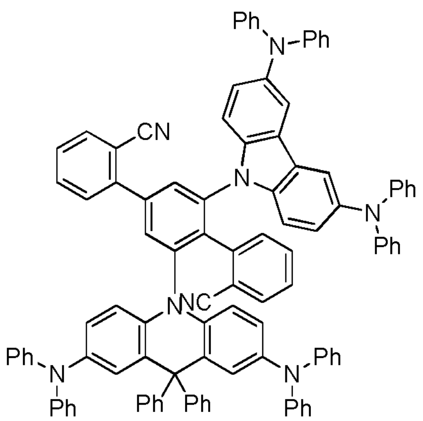

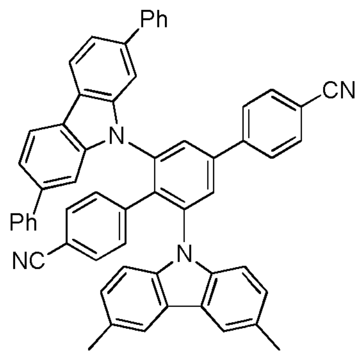

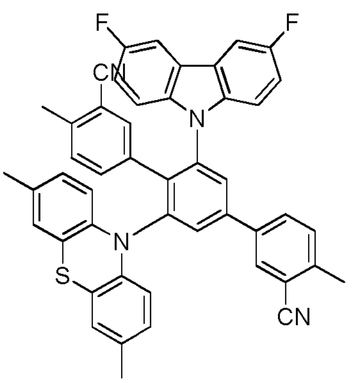

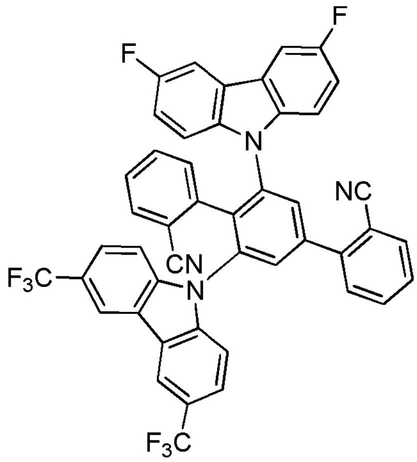

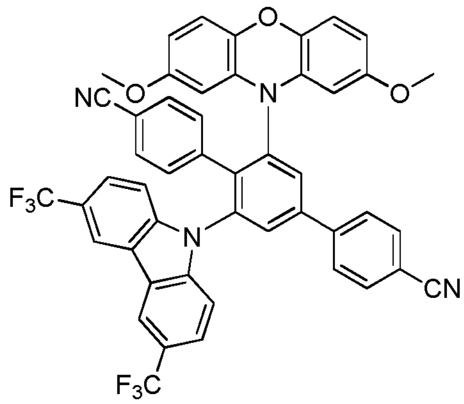

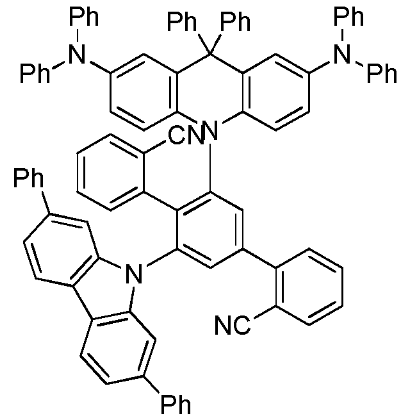

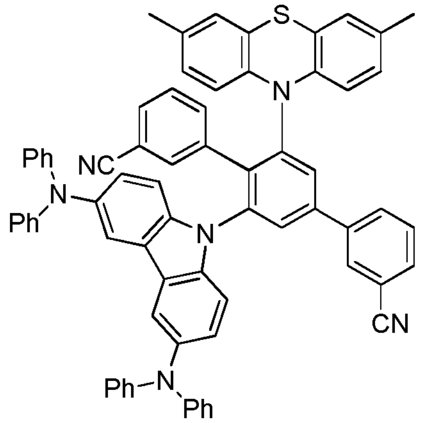

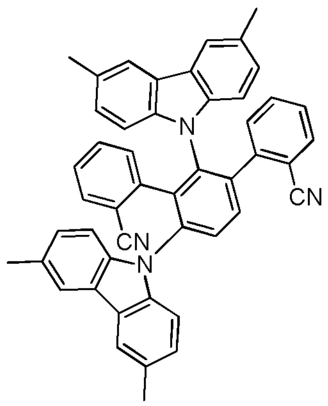

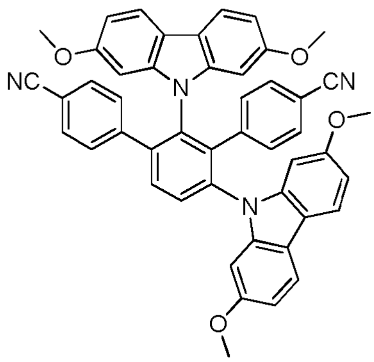

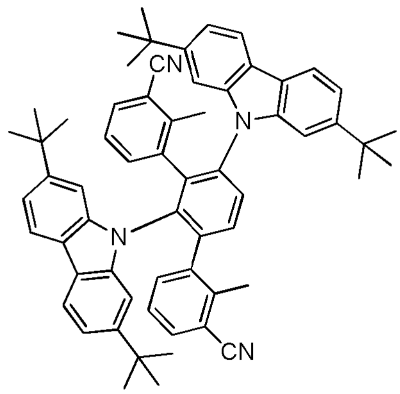

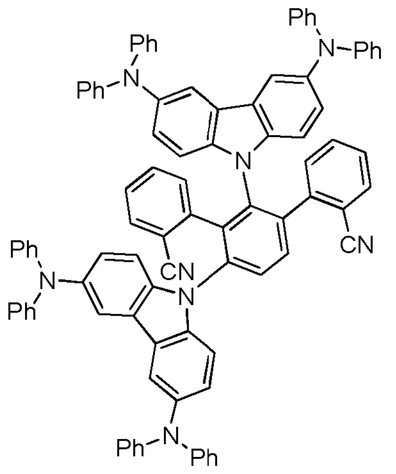

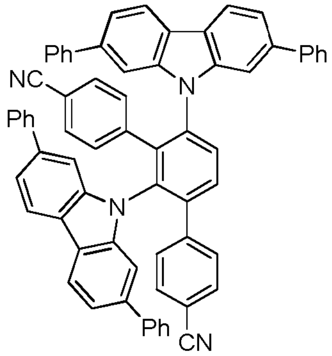

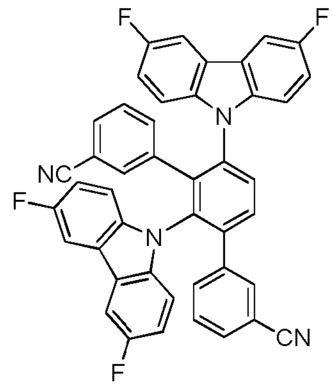

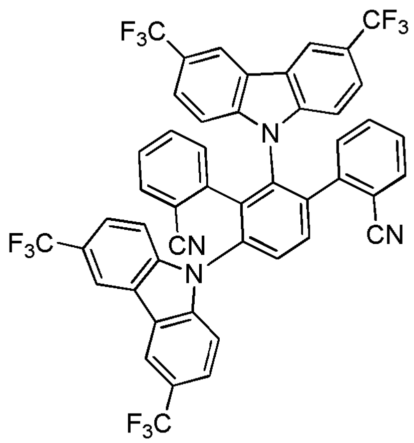

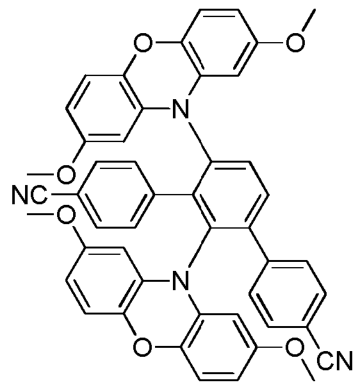

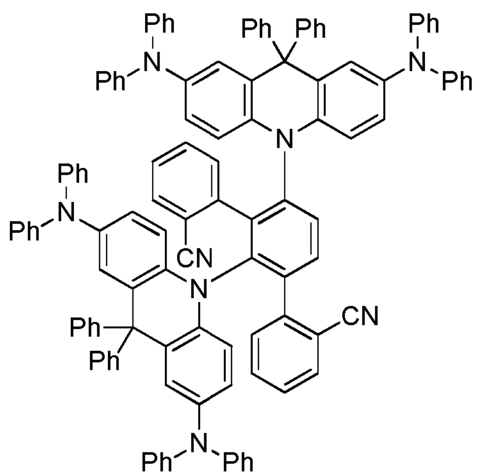

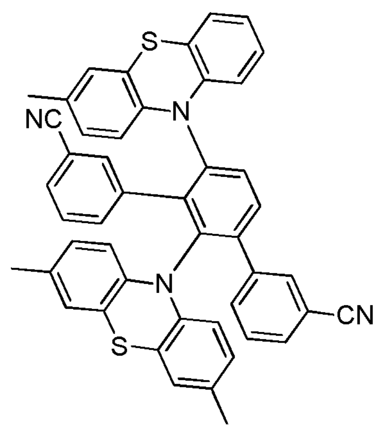

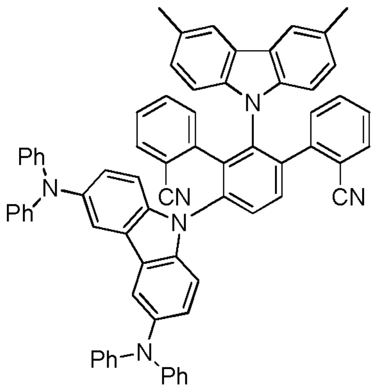

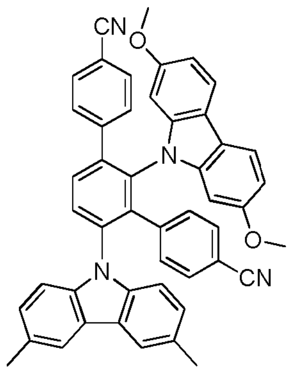

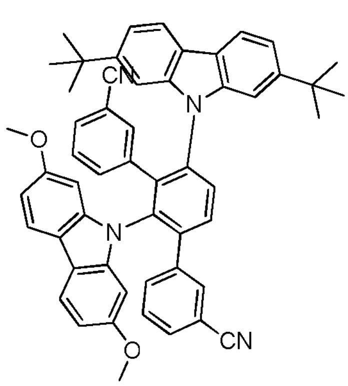

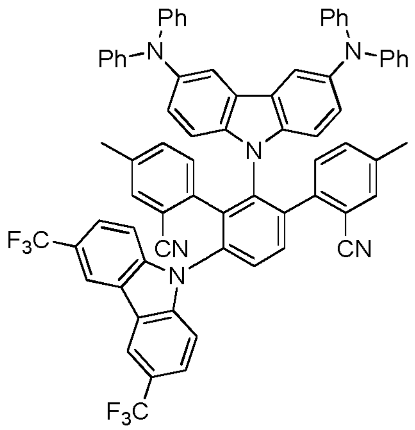

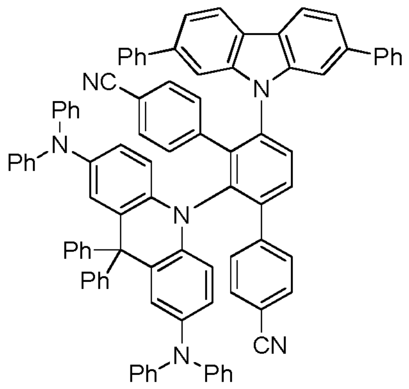

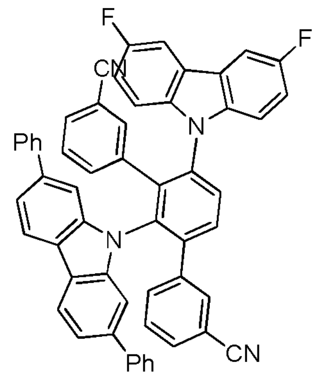

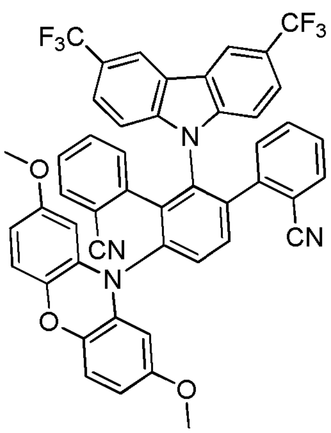

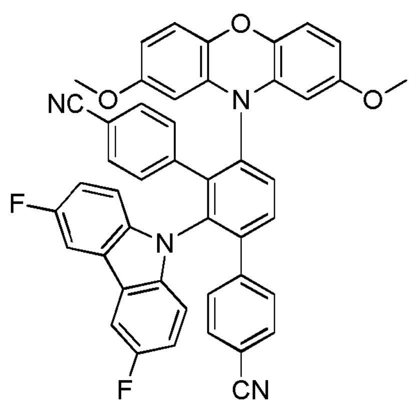

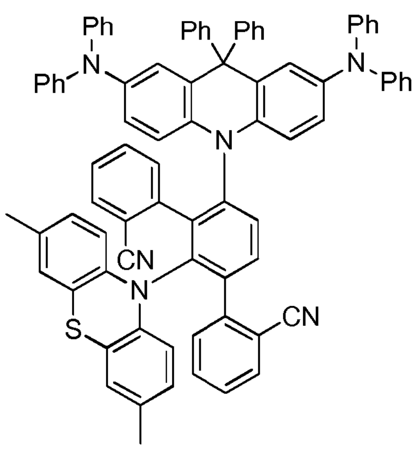

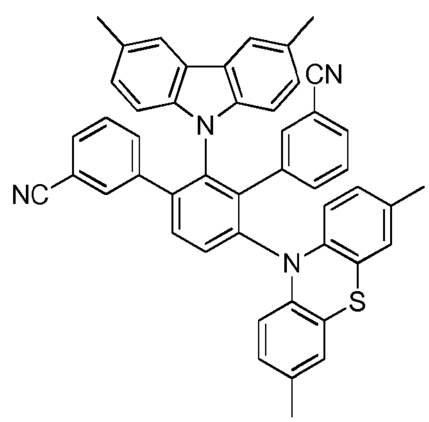

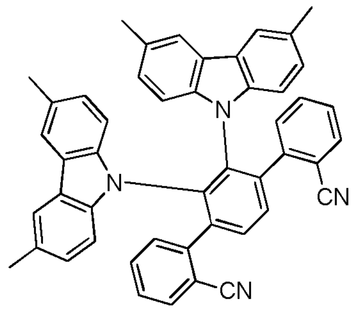
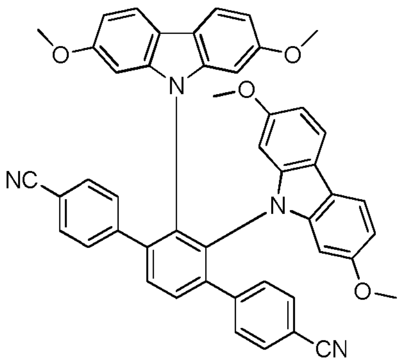
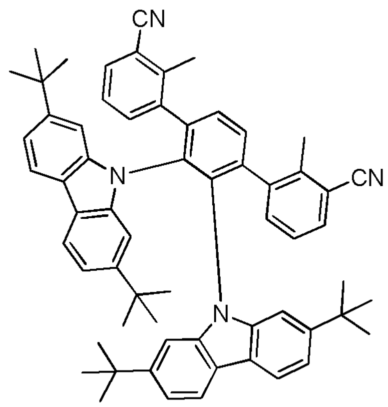

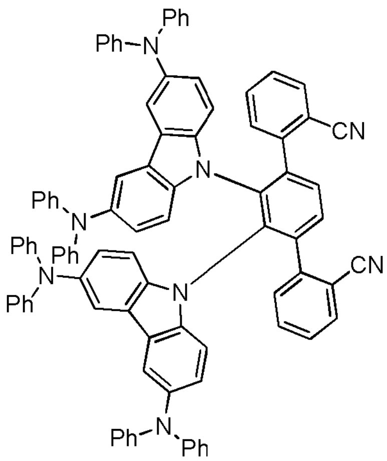
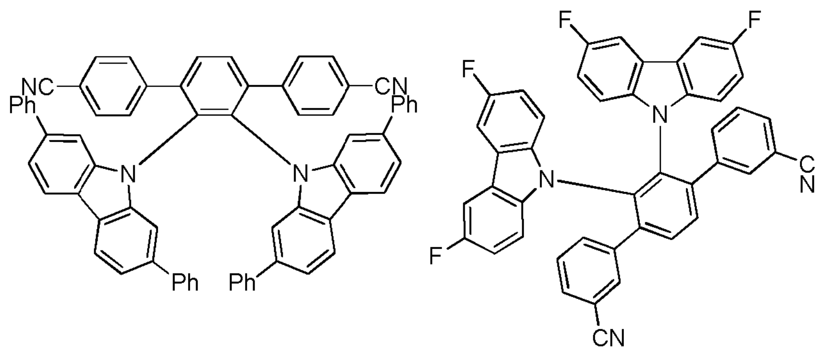

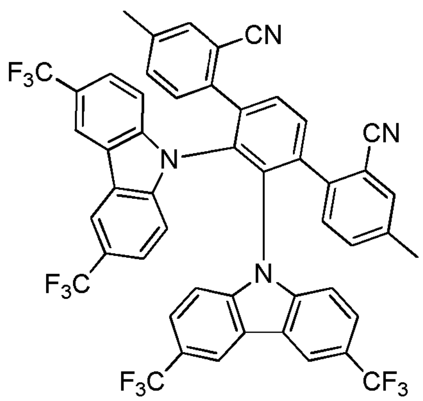
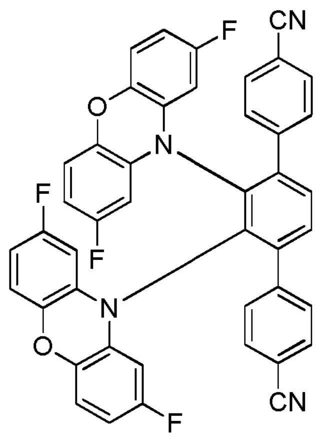

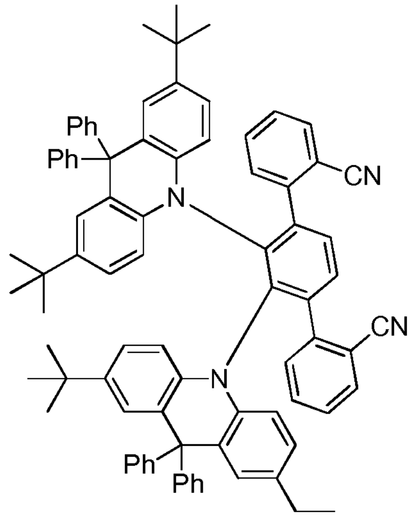

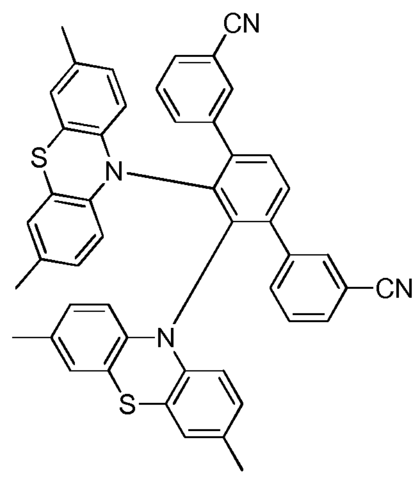

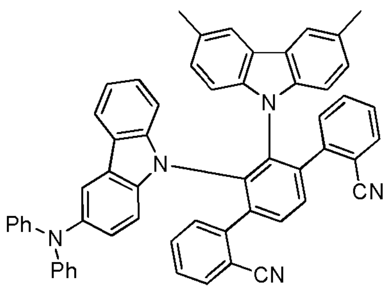

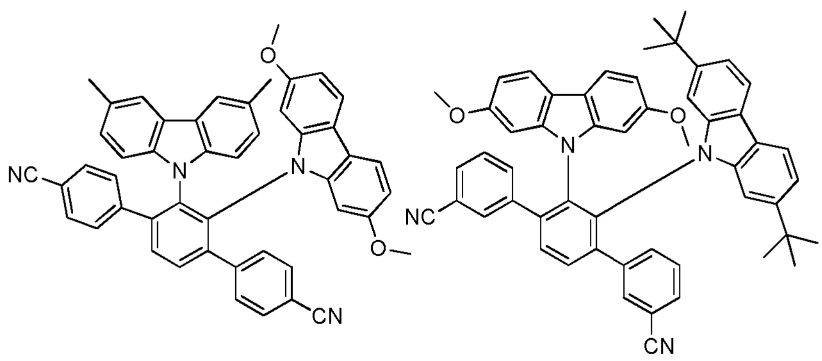

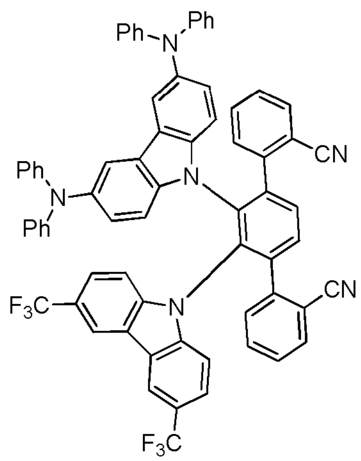

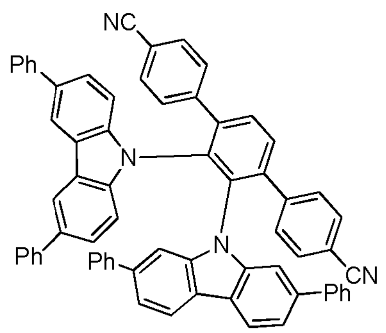

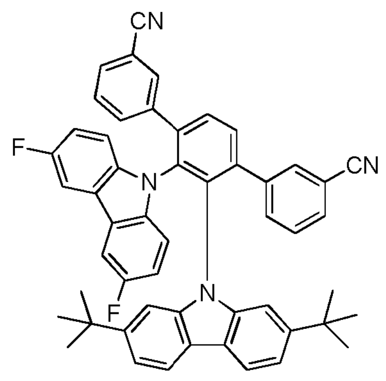

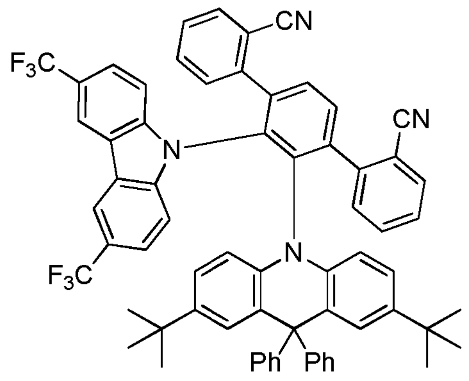

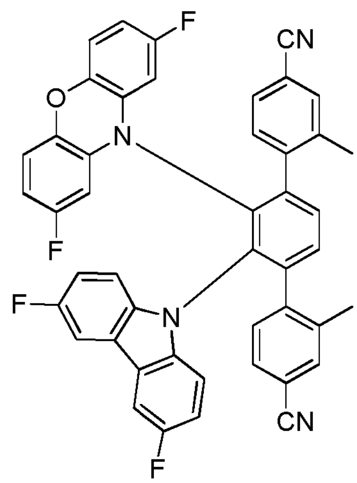

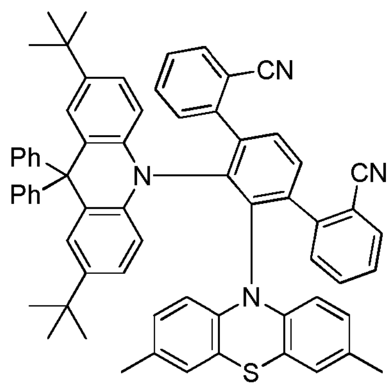

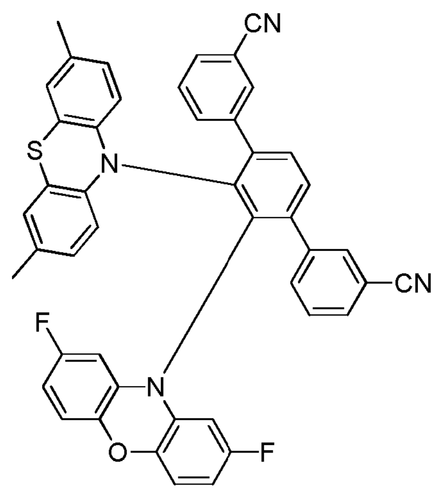

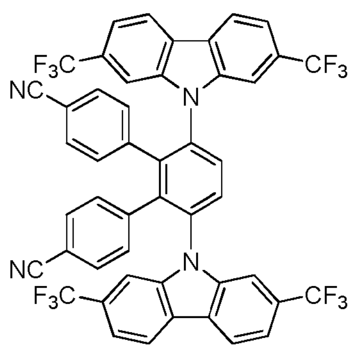

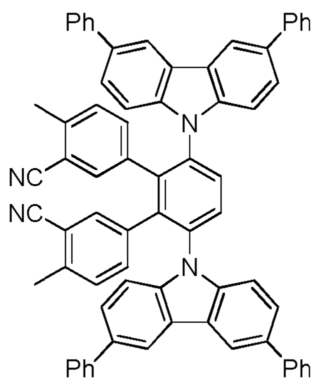

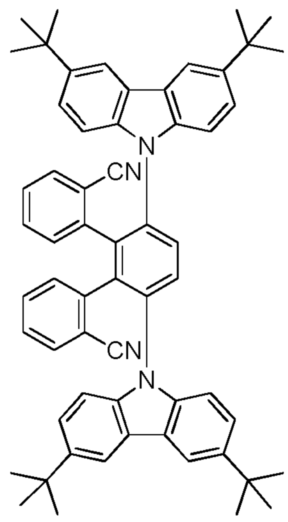

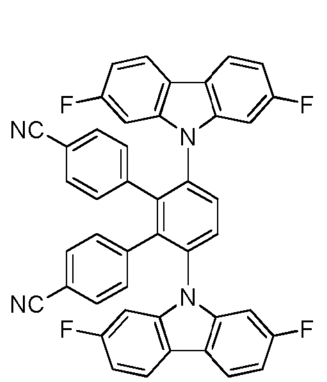

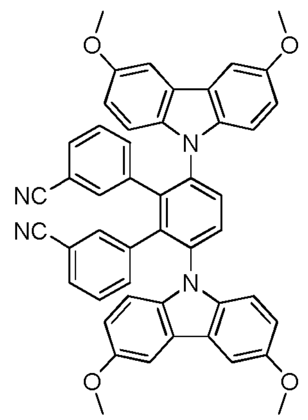

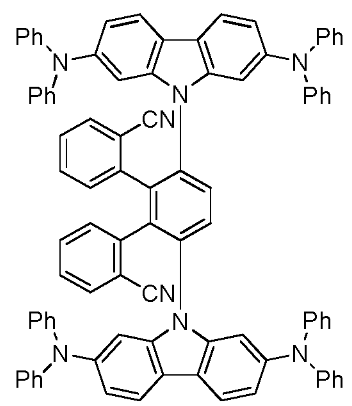

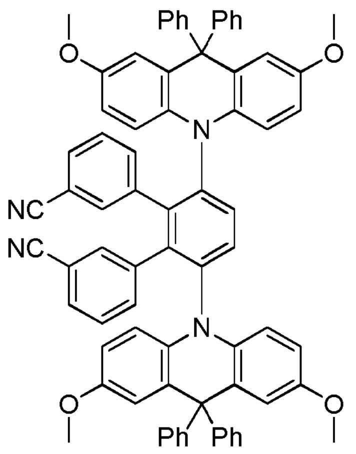

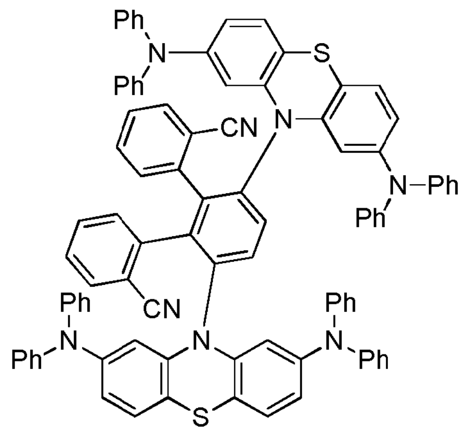

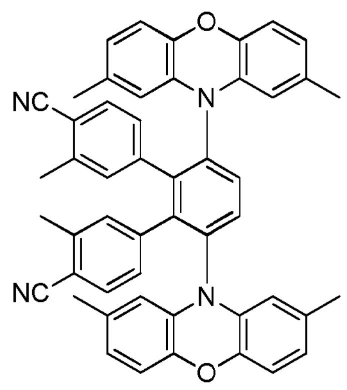

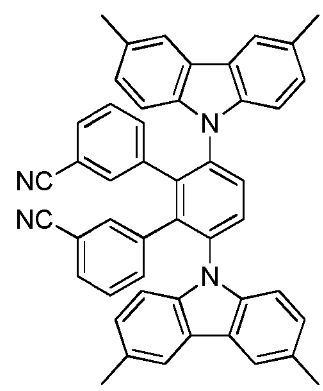

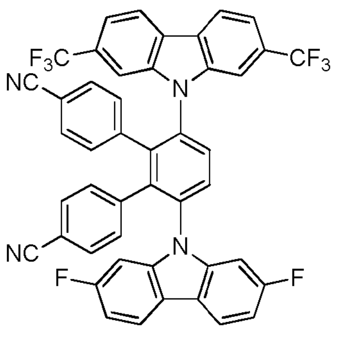

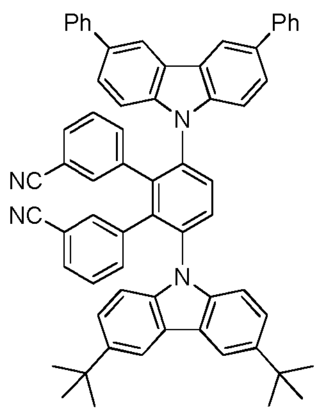

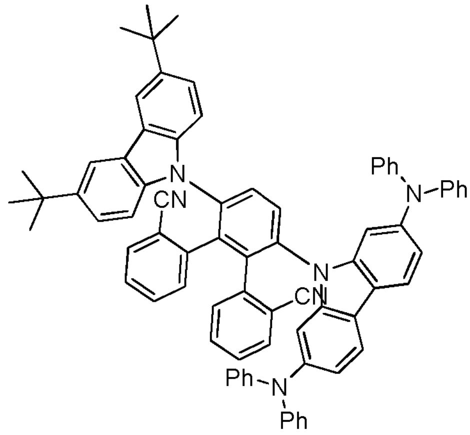

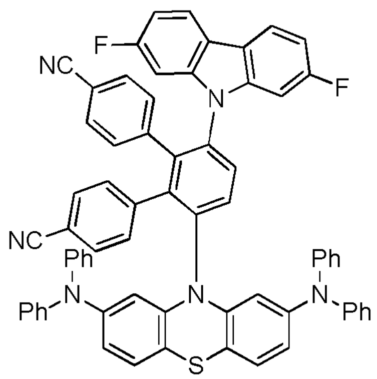

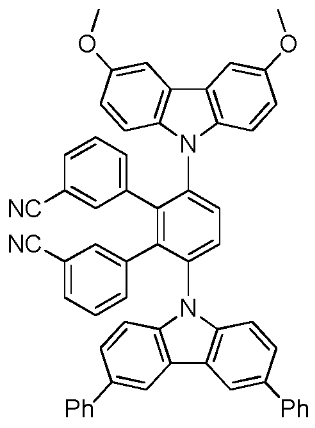

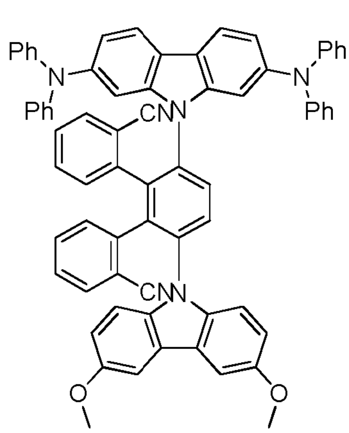

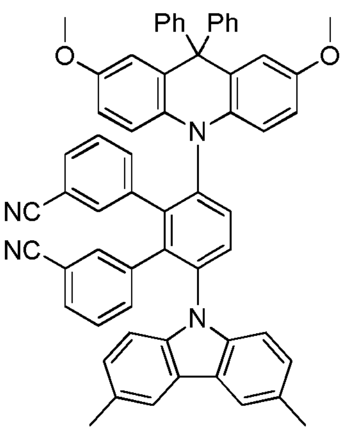

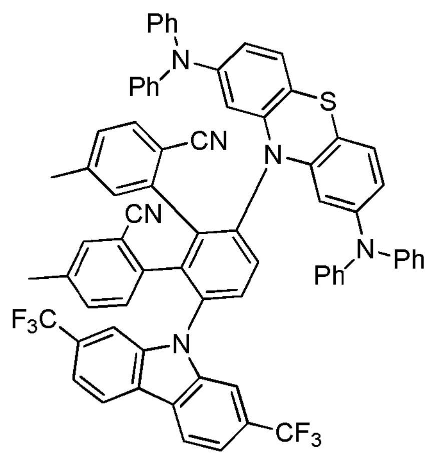

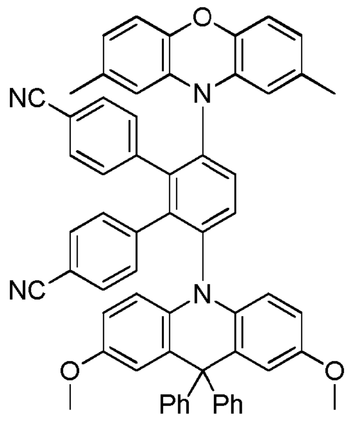 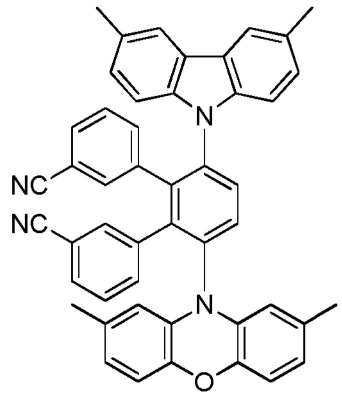

**Figuren**

Figuren 14 bis 24 betreffen Emissionspektren nicht erfindungsgemäß offenbarter Verbindungen. Es zeigen:

Figur 1 Emissionsspektrum von **1** in 10 % PMMA.
Figur 2 Emissionsspektrum von **2** in 10 % PMMA.
Figur 3 Emissionsspektrum von **3** in 10 % PMMA.
Figur 4 Emissionsspektrum von **4** in 10 % PMMA.
Figur 5 Emissionsspektrum von **5** in 10 % PMMA.
Figur 6 Emissionsspektrum von **6** in 10 % PMMA.
Figur 7 Emissionsspektrum von **7** in 10 % PMMA.
Figur 8 Emissionsspektrum von **8** in 10 % PMMA.
Figur 9 Emissionsspektrum von **9** in 10 % PMMA.
Figur 10 Emissionsspektrum von **10** in 10 % PMMA.
Figur 11 Emissionsspektrum von **11** in 10 % PMMA.
Figur 12 Emissionsspektrum von **12** in 10 % PMMA.
Figur 13 Emissionsspektrum von **13** in 10 % PMMA.
Figur 14 Emissionsspektrum von **14** in 10 % PMMA.
Figur 15 Emissionsspektrum von **15** in 10 % PMMA.
Figur 16 Emissionsspektrum von **16** in 10 % PMMA.
Figur 17 Emissionsspektrum von **17** in 10 % PMMA.
Figur 18 Emissionsspektrum von **18** in 10 % PMMA.
Figur 19 Emissionsspektrum von **19** in 10 % PMMA.
Figur 20 Emissionsspektrum von **20** in 10 % PMMA.
Figur 21 Emissionsspektrum von **23** in 10 % PMMA.
Figur 22 Emissionsspektrum von **24** in 10 % PMMA.
Figur 23 Emissionsspektrum von **25** in 10 % PMMA.
Figur 24 Emissionsspektrum von **26** in 10 % PMMA.

**Patentansprüche**

**1.** Organisches Molekül, aufweisend eine Struktur der Formel I

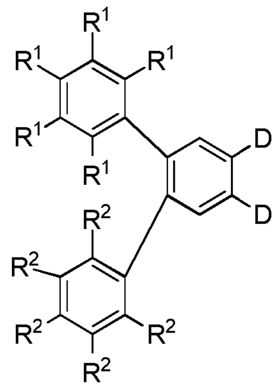

Formel Ia

mit
D =

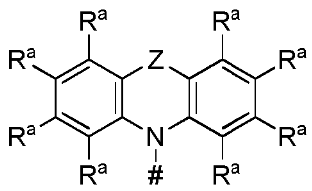

wobei

# ist Anknüpfungspunkt der Einheit D an den zentralen Phenylring der Struktur gemäß Formel Ia;
Z ist eine direkte Bindung oder ausgewählt aus der Gruppe bestehend aus $CR^3R^4$, $C=CR^3R^4$, $C=NR^3$, $NR^3$, O, $SiR^3R^4$, S, S(O) und $S(O)_2$;
$R^1$ und $R^2$ bei jedem Auftreten gleich oder verschieden ausgewählt ist aus der Gruppe bestehend aus

- H, Deuterium;
- einer linearen Alkylgruppe mit 1 bis 5 C-Atomen, wobei ein oder mehrere H-Atome durch Deuterium, CN, $CF_3$ oder $NO_2$ ersetzt sein können;
- einer linearen Alkenyl- oder Alkinylgruppe mit 2 bis 8 C-Atomen, wobei ein oder mehrere H-Atome durch Deuterium, CN, $CF_3$ oder $NO_2$ ersetzt sein können;
- einer verzweigten oder cyclischen Alkyl-, Alkenyl- oder Alkinylgruppe mit 3 bis 10 C-Atomen, wobei ein oder mehrere H-Atome durch Deuterium, CN, $CF_3$ oder $NO_2$ ersetzt sein können;
- einem aromatischen oder heteroaromatischen Ringsystem mit 5 bis 15 aromatischen Ringatomen, das jeweils mit einem oder mehreren Resten $R^6$ substituiert sein kann;
- $CF_3$ und
- CN;

$R^a$, $R^3$ und $R^4$ bei jedem Auftreten gleich oder verschieden ausgewählt ist aus der Gruppe bestehend aus

- H, Deuterium, $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt

sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^5$ substituiert sein kann;

$R^5$ bei jedem Auftreten gleich oder verschieden ausgewählt ist aus der Gruppe bestehend aus

- H, Deuterium, $N(R^6)_2$, OH, $Si(R^6)_3$, $B(OR^6)_2$, $OSO_2R^6$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^6$ substituiert sein kann;
- einer Aryloxy- oder Heteroaryloxygruppe mit 5 bis 60 aromatischen Ringatomen, die mit einem oder mehreren Resten $R^6$ substituiert sein kann; und
- einer Diarylaminogruppe, Diheteroarylaminogruppe oder Arylheteroarylaminogruppe mit 10 bis 40 aromatischen Ringatomen, welche mit einem oder mehreren Resten $R^6$ substituiert sein kann;

$R^6$ bei jedem Auftreten gleich oder verschieden ausgewählt ist aus der Gruppe bestehend aus

- H, Deuterium, OH, $CF_3$, CN, F, Br, I;
- einer linearen Alkyl-, Alkoxy- oder Thioalkoxygruppe mit 1 bis 5 C-Atomen, wobei ein oder mehrere H-Atome durch Deuterium, CN, $CF_3$ oder $NO_2$ ersetzt sein können;
- einer linearen Alkenyl- oder Alkinylgruppe mit 2 bis 5 C-Atomen, wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen;
- einer Aryloxy- oder Heteroaryloxygruppe mit 5 bis 60 aromatischen Ringatomen; und
- einer Diarylaminogruppe, Diheteroarylaminogruppe oder Arylheteroarylaminogruppe mit 10 bis 40 aromatischen Ringatomen;

wobei jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoannelliertes Ringsystem bilden kann; und wobei unabhängig voneinander genau ein $R^1$ und genau ein $R^2$ gleich $CF_3$ oder CN ist.

**2.** Organisches Molekül nach Anspruch 1, wobei genau ein $R^1$ und genau ein $R^2$ gleich CN sind und optional die übrigen Reste $R^1$ und $R^2$ bei jedem Auftreten gleich oder verschieden H oder Methyl sind.

**3.** Organisches Molekül nach Anspruch 1 oder 2, wobei genau zwei $R^1$ oder zwei $R^2$ gleich Methyl und die übrigen $R^1$ und ein $R^2$ gleich H sind.

**4.** Organisches Molekül nach Anspruch 1 oder 2, wobei genau ein weiterer Rest $R^1$ und genau ein weiterer Rest $R^2$ Methyl und die weiteren Reste $R^1$ und $R^2$ H sind.

**5.** Organisches Molekül nach Anspruch 1 bis 4, wobei D eine Struktur der Formel II aufweist:

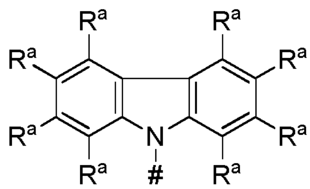

Formel II

wobei

# jeweils der Anknüpfungspunkt der Einheit D an den zentralen Phenylring in der Struktur der Formel Ia ist; und wobei $R^a$ wie in Anspruch 1 definiert ist.

**6.** Verfahren zur Herstellung eines Organischen Moleküls nach Anspruch 1 bis 5, wobei ein 1,2-Dibrom-4,5-difluorbenzol als Edukt eingesetzt wird.

**7.** Verwendung eines organischen Moleküls nach Anspruch 1 bis 5 als lumineszierender Emitter und/oder als Hostmaterial und/oder als Elektronentransportmaterial und/oder als Lochinjektionsmaterial und/oder als Lochblockiermaterial in einer organischen optoelektronischen Vorrichtung.

**8.** Verwendung nach Anspruch 7, wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

**9.** Zusammensetzung aufweisend oder bestehend aus:

(a) mindestens einem organischen Molekül nach einem der Ansprüche 1 bis 5, insbesondere als Emitter und/oder Host, und
(b) ein oder mehrere von dem Molekül nach einem der Ansprüche 1 bis 5 verschiedenen Emitter- und/oder Hostmaterialien
(c) optional einem oder mehreren Farbstoffen und/ oder einem oder mehreren Lösungsmitteln.

**10.** Organische optoelektronische Vorrichtung, aufweisend ein organisches Molekül nach Anspruch 1 bis 5 oder eine Zusammensetzung nach Anspruch 9, insbesondere ausgeformt als eine Vorrichtung ausgewählt aus der Gruppe bestehend ausorganischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**11.** Organische optoelektronische Vorrichtung nach Anspruch 10, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die das organische Molekül nach Anspruch 1 bis 5 aufweist.

**12.** Verfahren zur Herstellung eines optoelektronischen Bauelements, wobei ein organisches Molekül nach Anspruch 1 bis 5 verwendet wird.

**13.** Verfahren nach Anspruch 12, umfassend die Verarbeitung des organischen Moleküls mittels eines Vakuumverdampfungsverfahrens oder aus einer Lösung.

**Claims**

**1.** Organic molecule comprising a structure of formula I

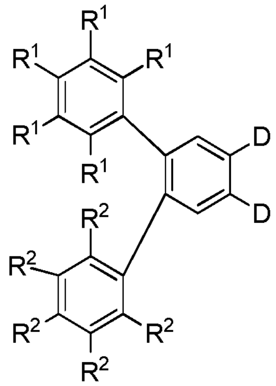

Formula Ia

with
D =

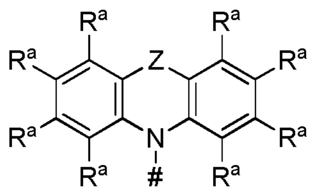

wherein

# is an attachment point of the moiety D to the central phenyl ring of the structure according to formula Ia;
Z is a direct bond or selected from the group consisting of $CR^3R^4$, $C=CR^3R^4$, $C=NR^3$, $NR^3$, O, $SiR^3R^4$, S, S(O) and $S(O)_2$;
$R^1$ and $R^2$ are at each occurrence the same or different and are selected from the group consisting of

- H, deuterium;
- a linear alkyl group having 1 to 5 C atoms, wherein one or more H atoms may be substituted by deuterium, CN, $CF_3$ or $NO_2$;
- a linear alkenyl or alkynyl group having 2 to 8 C atoms, wherein one or more H atoms may be substituted by deuterium, CN, $CF_3$ or $NO_2$;
- a branched or cyclic alkyl, alkenyl or alkynyl group having 3 to 10 C atoms, wherein one or more H atoms

may be substituted by deuterium CN, $CF_3$ or $NO_2$;
- an aromatic or heteroaromatic ring system having 5 to 15 aromatic ring atoms, each of which may be substituted by one or more moieties $R^6$;
- $CF_3$ and
- CN;

$R^a$, $R^3$ and $R^4$ are at each occurrence the same or different and are selected from the group consisting of

- H, deuterium, $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group having 1 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- a linear alkenyl or alkynyl group having 2 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- a branched or cyclic alkyl, alkenyl, alkynyl, alkoxy or thioalkoxy group having 3 to 40 C atoms, each of which may be substituted by one or more moieties $R^5$, wherein one or more non-adjacent $CH_2$ groups may be substituted by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms may be substituted by deuterium, CN, $CF_3$ or $NO_2$;
- an aromatic or heteroaromatic ring system having 5 to 60 aromatic ring atoms, each of which may be substituted by one or more moieties $R^5$;
- an aryloxy or heteroaryloxy group having 5 to 60 aromatic ring atoms, which may be substituted by one or more moieties $R^5$; and
- a diarylamino group, diheteroarylamino group or arylheteroarylamino group having 10 to 40 aromatic ring atoms, which may be substituted by one or more moieties $R^5$;

$R^5$ is at each occurrence the same or different and is selected from the group consisting of

- H, deuterium, $N(R^6)_2$, OH, $Si(R^6)_3$, $B(OR^6)_2$, $OSO_2R^6$, $CF_3$, CN, F, Br, I;
- a linear alkyl, alkoxy or thioalkoxy group having 1 to 40 C atoms, each of which may be substituted by one or more moieties $R^6$, wherein one or more non-adjacent $CH_2$ groups may be substituted by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms may be substituted by deuterium, CN, $CF_3$ or $NO_2$;
- a linear alkenyl or alkynyl group having 2 to 40 C atoms, each of which may be substituted by one or more moieties $R^6$, wherein one or more non-adjacent $CH_2$ groups may be substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms may be substituted by deuterium, CN, $CF_3$ or $NO_2$;
- a branched or cyclic alkyl, alkenyl, alkynyl, alkoxy or thioalkoxy group having 3 to 40 C atoms, each of which may be substituted by one or more moieties $R^6$, wherein one or more non-adjacent $CH_2$ groups may be substituted by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms may be substituted by deuterium, CN, $CF_3$ or $NO_2$;
- an aromatic or heteroaromatic ring system having 5 to 60 aromatic ring atoms, each of which may be substituted by one or more moieties $R^6$;
- an aryloxy or heteroaryloxy group having 5 to 60 aromatic ring atoms, which may be substituted by one or more moieties $R^6$; and
- a diarylamino group, diheteroarylamino group or arylheteroarylamino group having 10 to 40 aromatic ring atoms, which may be substituted by one or more moieties $R^6$;

$R^6$ is at each occurrence the same or different and is selected from the group consisting of

- H, deuterium, OH, $CF_3$, CN, F, Br, I;
- a linear alkyl, alkoxy or thioalkoxy group having 1 to 5 C atoms, wherein one or more H atoms may be substituted by deuterium, CN, $CF_3$ or $NO_2$;
- a linear alkenyl or alkynyl group having 2 to 5 C atoms, wherein one or more H atoms may be substituted

by deuterium, CN, $CF_3$ or $NO_2$;
- a branched or cyclic alkyl, alkenyl, alkynyl, alkoxy or thioalkoxy group having 3 to 5 C atoms, wherein one or more H atoms may be substituted by deuterium, CN, $CF_3$ or $NO_2$;
- an aromatic or heteroaromatic ring system having 5 to 60 aromatic ring atoms;
- an aryloxy or heteroaryloxy group having 5 to 60 aromatic ring atoms; and
- a diarylamino group, diheteroarylamino group or arylheteroarylamino group having 10 to 40 aromatic ring atoms;

wherein each of the moieties $R^a$, $R^3$, $R^4$ or $R^5$ may also form a mono- or polycyclic, aliphatic, aromatic and/or benzo-fused ring system with one or more further moieties $R^a$, $R^3$, $R^4$ or $R^5$; and
wherein independently of each other exactly one $R^1$ and exactly one $R^2$ is $CF_3$ or CN.

2. Organic molecule according to claim 1, wherein exactly one $R^1$ and exactly one $R^2$ are CN and, optionally, the remaining moieties $R^1$ and $R^2$ are at each occurrence the same or different and are H or methyl.

3. Organic molecule according to claim 1 or 2, wherein exactly two $R^1$ or two $R^2$ are methyl and the remaining $R^1$ and one $R^2$ are H.

4. Organic molecule according to claim 1 or 2, wherein exactly one further moiety $R^1$ and exactly one further moiety $R^2$ are methyl and the further moieties $R^1$ and $R^2$ are H.

5. Organic molecule according to claims 1 to 4, wherein D comprises a structure of formula II:

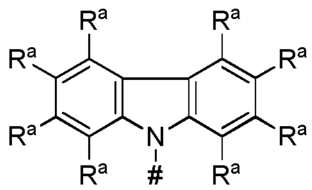

Formula II

wherein

each # is the attachment point of the moiety D to the central phenyl ring in the structure of formula Ia;
and wherein $R^a$ is as defined in claim 1.

6. Method for preparing an organic molecule according to claims 1 to 5, wherein a 1,2-dibromo-4,5-difluorobenzene is used as a reactant.

7. Use of an organic molecule according to claims 1 to 5 as a luminescent emitter and/or as a host material and/or as an electron transport material and/or as a hole injection material and/or as a hole blocking material in an organic optoelectronic device.

8. Use according to claim 7, wherein the organic optoelectronic device is selected from the group consisting of:

• organic light-emitting diodes (OLEDs),
• light-emitting electrochemical cells,
• OLED-sensors, in particular in gas and vapor sensors that are not hermetically shielded from the outside
• organic diodes,
• organic solar cells,
• organic transistors,
• organic field-effect transistors,
• organic lasers, and
• down-conversion elements.

9. Composition, comprising or consisting of:

(a) at least one organic molecule according to any one of claims 1 to 5, in particular as an emitter and/or a host, and
(b) one or more emitter and/or host materials, which differ from the molecule according to any one of claims 1 to 5
(c) optionally, one or more dyes and/or one or more solvents.

**10.** Organic optoelectronic device, comprising an organic molecule according to claims 1 to 5 or a composition according to claim 9, in particular in form of a device selected from the group consisting of organic light-emitting diode (OLED), light-emitting electrochemical cell, OLED-sensor, in particular gas and vapor sensors that are not hermetically shielded from the outside, organic diode, organic solar cell, organic transistor, organic field-effect transistor, organic laser, and down-conversion element.

**11.** Organic optoelectronic device according to claim 10, comprising

- a substrate,
- an anode, and
- a cathode, wherein the anode or the cathode are disposed on the substrate, and
- at least one light-emitting layer, which is arranged between anode and cathode and which comprises the organic molecule according to claims 1 to 5.

**12.** Method for producing an optoelectronic component, wherein an organic molecule according to claims 1 to 5 is used.

**13.** Method according to claim 12, comprising the processing of the organic molecule by a vacuum evaporation method or from a solution.

## Revendications

**1.** Molécule organique, comprenant une structure de formule I

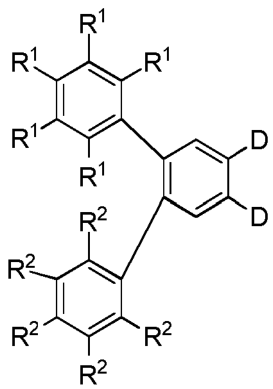

Formule Ia

dans laquelle
D =

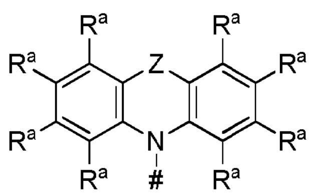

dans laquelle

# représente le point de liaison du motif D au cycle phényle central de la structure selon la formule la ;
Z représente une liaison directe ou est choisi dans le groupe constitué par $CR^3R^4$, $C=CR^3R^4$, $C=NR^3$, $NR^3$, O,

$SiR^3R^4$, S, S(O) et $S(O)_2$ ;
$R^1$ et $R^2$ sont choisis, en chaque occurrence, de manière identique ou différente, dans le groupe constitué par :

- H, deutérium ;
- un groupe alkyle linéaire, comprenant 1 à 5 atomes de carbone, un ou plusieurs atomes d'H pouvant être remplacés par deutérium, CN, $CF_3$ ou $NO_2$;
- un groupe alcényle ou alcynyle, linéaire, comprenant 2 à 8 atomes de carbone, un ou plusieurs atomes d'H pouvant être remplacés par deutérium, CN, $CF_3$ ou $NO_2$;
- un groupe alkyle, alcényle ou alcynyle, ramifié ou cyclique, comprenant 3 à 10 atomes de carbone, un ou plusieurs atomes d'H pouvant être remplacés par deutérium, CN, $CF_3$ ou $NO_2$;
- un système cyclique aromatique ou hétéroaromatique comprenant 5 à 15 atomes de cycle aromatique, qui peut à chaque fois être substitué par un ou plusieurs radicaux $R^6$ ;
- $CF_3$ et
- CN ;

$R^a$, $R^3$ et $R^4$ sont choisis, en chaque occurrence, de manière identique ou différente, dans le groupe constitué par :

- H, deutérium, $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy, linéaire, comprenant 1 à 40 atomes de carbone, qui peut à chaque fois être substitué par un ou plusieurs radicaux $R^5$, un ou plusieurs groupes $CH_2$ non adjacents pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes d'H pouvant être remplacés par deutérium, CN, $CF_3$ ou $NO_2$;
- un groupe alcényle ou alcynyle, linéaire, comprenant 2 à 40 atomes de carbone, qui peut à chaque fois être substitué par un ou plusieurs radicaux $R^5$, un ou plusieurs groupes $CH_2$ non adjacents pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes d'H pouvant être remplacés par deutérium, CN, $CF_3$ ou $NO_2$;
- un groupe alkyle, alcényle, alcynyle, alcoxy ou thioalcoxy, ramifié ou cyclique, comprenant 3 à 40 atomes de carbone, qui peut à chaque fois être substitué par un ou plusieurs radicaux $R^5$, un ou plusieurs groupes $CH_2$ non adjacents pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et un ou plusieurs atomes d'H pouvant être remplacés par deutérium, CN, $CF_3$ ou $NO_2$;
- un système cyclique aromatique ou hétéroaromatique comprenant 5 à 60 atomes de cycle aromatique, qui peut à chaque fois être substitué par un ou plusieurs radicaux $R^5$ ;
- un groupe aryloxy ou hétéroaryloxy comprenant 5 à 60 atomes de cycle aromatique, qui peut être substitué par un ou plusieurs radicaux $R^5$ ; et
- un groupe diarylamino, dihétéroarylamino ou arylhétéroarylamino comprenant 10 à 40 atomes de cycle aromatique, qui peut être substitué par un ou plusieurs radicaux $R^5$ ;

$R^5$ est choisi, en chaque occurrence, de manière identique ou différente, dans le groupe constitué par :

- H, deutérium, $N(R^6)_2$, OH, $Si(R^6)_3$, $B(OR^6)_2$, $OSO_2R^6$, $CF_3$, CN, F, Br, I ;
- un groupe alkyle, alcoxy ou thioalcoxy, linéaire, comprenant 1 à 40 atomes de carbone, qui peut à chaque fois être substitué par un ou plusieurs radicaux $R^6$, un ou plusieurs groupes $CH_2$ non adjacents pouvant être remplacés par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ et un ou plusieurs atomes d'H pouvant être remplacés par deutérium, CN, $CF_3$ ou $NO_2$;
- un groupe alcényle ou alcynyle, linéaire, comprenant 2 à 40 atomes de carbone, qui peut à chaque fois être substitué par un ou plusieurs radicaux $R^6$, un ou plusieurs groupes $CH_2$ non adjacents pouvant être remplacés par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ et un ou plusieurs atomes d'H pouvant être remplacés par deutérium, CN, $CF_3$ ou $NO_2$;
- un groupe alkyle, alcényle, alcynyle, alcoxy ou thioalcoxy, ramifié ou cyclique, comprenant 3 à 40 atomes de carbone, qui peut à chaque fois être substitué par un ou plusieurs radicaux $R^6$, un ou plusieurs groupes $CH_2$ non adjacents pouvant être remplacés par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ et un ou plusieurs atomes d'H pouvant être remplacés par deutérium, CN, $CF_3$ ou $NO_2$;
- un système cyclique aromatique ou hétéroaromatique comprenant 5 à 60 atomes de cycle aromatique,

qui peut à chaque fois être substitué par un ou plusieurs radicaux $R^6$ ;
- un groupe aryloxy ou hétéroaryloxy comprenant 5 à 60 atomes de cycle aromatique, qui peut être substitué par un ou plusieurs radicaux $R^6$ ; et
- un groupe diarylamino, dihétéroarylamino ou arylhétéroarylamino comprenant 10 à 40 atomes de cycle aromatique, qui peut être substitué par un ou plusieurs radicaux $R^6$ ;

$R^6$ est choisi, en chaque occurrence, de manière identique ou différente, dans le groupe constitué par :

- H, deutérium, OH, $CF_3$, CN, F, Br, I ;
- un groupe alkyle, alcoxy ou thioalcoxy, linéaire, comprenant 1 à 5 atomes de carbone, un ou plusieurs atomes d'H pouvant être remplacés par deutérium, CN, $CF_3$ ou $NO_2$;
- un groupe alcényle ou alcynyle, linéaire, comprenant 2 à 5 atomes de carbone, un ou plusieurs atomes d'H pouvant être remplacés par deutérium, CN, $CF_3$ ou $NO_2$ ;
- un groupe alkyle, alcényle, alcynyle, alcoxy ou thioalcoxy, ramifié ou cyclique, comprenant 3 à 5 atomes de carbone, un ou plusieurs atomes d'H pouvant être remplacés par deutérium, CN, $CF_3$ ou $NO_2$;
- un système cyclique aromatique ou hétéroaromatique comprenant 5 à 60 atomes de cycle aromatique ;
- un groupe aryloxy ou hétéroaryloxy comprenant 5 à 60 atomes de cycle aromatique ; et
- un groupe diarylamino, dihétéroarylamino ou arylhétéroarylamino comprenant 10 à 40 atomes de cycle aromatique ;

chacun des radicaux $R^a$, $R^3$, $R^4$ ou $R^5$ pouvant également former, avec un ou plusieurs autres radicaux $R^a$, $R^3$, $R^4$ ou $R^5$, un système cyclique monocyclique ou polycyclique, aliphatique, aromatique et/ou benzocondensé ; et exactement un radical $R^1$ et exactement un radical $R^2$ représentant, indépendamment l'un de l'autre, $CF_3$ ou CN.

**2.** Molécule organique selon la revendication 1, exactement un radical $R^1$ et exactement un radical $R^2$ représentant CN et éventuellement, les autres radicaux $R^1$ et $R^2$ représentant, en chaque occurrence, de manière identique ou différente, H ou méthyle.

**3.** Molécule organique selon la revendication 1 ou 2, exactement deux radicaux $R^1$ ou deux radicaux $R^2$ représentant méthyle et les autres radicaux $R^1$ et un $R^2$ représentant H.

**4.** Molécule organique selon la revendication 1 ou 2, exactement un autre radical $R^1$ et exactement un autre radical $R^2$ représentant méthyle et les autres radicaux $R^1$ et $R^2$ représentant H.

**5.** Molécule organique selon les revendications 1 à 4, D comprenant une structure de formule II :

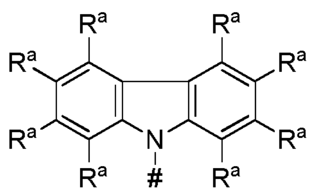

Formule II

dans laquelle

# représente à chaque fois le point de liaison du motif D au cycle phényle central de la structure selon la formule Ia ;
$R^a$ étant défini comme dans la revendication 1.

**6.** Procédé pour la préparation d'une molécule organique selon la revendication 1 à 5, un 1,2-dibromo-4,5-difluorobenzène étant utilisé comme produit de départ.

**7.** Utilisation d'une molécule organique selon les revendications 1 à 5 comme émetteur électroluminescent et/ou comme matériau hôte et/ou comme matériau de transport d'électrons et/ou comme matériau d'injection de trous et/ou comme matériau de blocage de trous dans un dispositif optoélectronique organique.

**8.** Utilisation selon la revendication 7, le dispositif optoélectronique organique étant choisi dans le groupe constitué par :

- les diodes électroluminescentes organiques (DELO),
- les piles électrochimiques électroluminescentes,
- les capteurs à DELO, en particulier dans les capteurs de gaz et de vapeur, non hermétiquement fermés par rapport à l'extérieur,
- les diodes organiques,
- les piles solaires organiques,
- les transistors organiques,
- les transistors organiques à effet de champ,
- les lasers organiques et
- les éléments de conversion vers le bas.

**9.** Composition comprenant ou constituée par :

(a) au moins une molécule organique selon l'une quelconque des revendications 1 à 5, en particulier en tant qu'un émetteur et/ou hôte, et
(b) un ou plusieurs matériaux émetteurs et/ou hôtes différents de la molécule selon l'une quelconque des revendications 1 à 5,
(c) éventuellement un ou plusieurs colorants et/ou un ou plusieurs solvants.

**10.** Dispositif optoélectronique organique, comprenant une molécule organique selon les revendications 1 à 5 ou une composition selon la revendication 9, en particulier formé sous forme d'un dispositif choisi dans le groupe constitué par les diodes électroluminescentes organiques (DELO), une pile électrochimique électroluminescente, un capteur à DELO, en particulier les capteurs de gaz et de vapeur, non hermétiquement fermés par rapport à l'extérieur, les diodes organiques, les piles solaires organiques, un transistor organique, un transistor organique à effet de champ, un laser organique et un élément de conversion vers le bas.

**11.** Dispositif optoélectronique organique selon la revendication 10, comprenant

- un substrat,
- une anode et
- une cathode, l'anode ou la cathode étant appliquées sur le substrat et
- au moins une couche électroluminescente, qui est disposée entre l'anode et la cathode et qui présente la molécule organique selon les revendications 1 à 5.

**12.** Procédé pour la fabrication d'un composant optoélectronique, une molécule organique selon la revendication 1 à 5 étant utilisée.

**13.** Procédé selon la revendication 12, comprenant la mise en œuvre de la molécule organique au moyen d'un procédé d'évaporation sous vide ou à partir d'une solution.

**Figur 1**

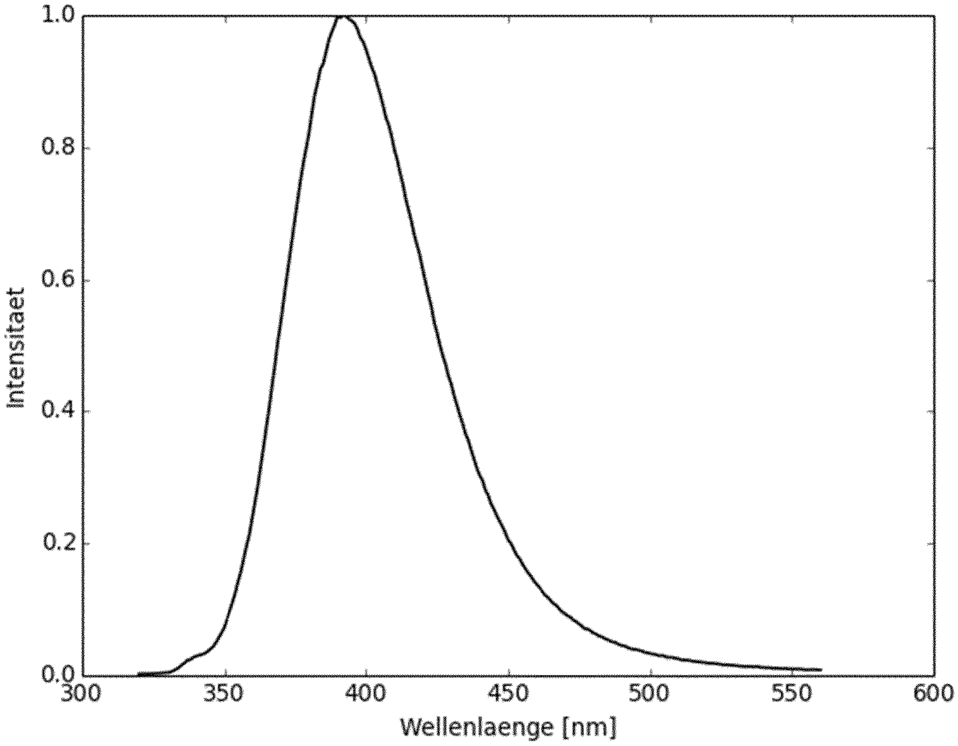


**Figur 2**

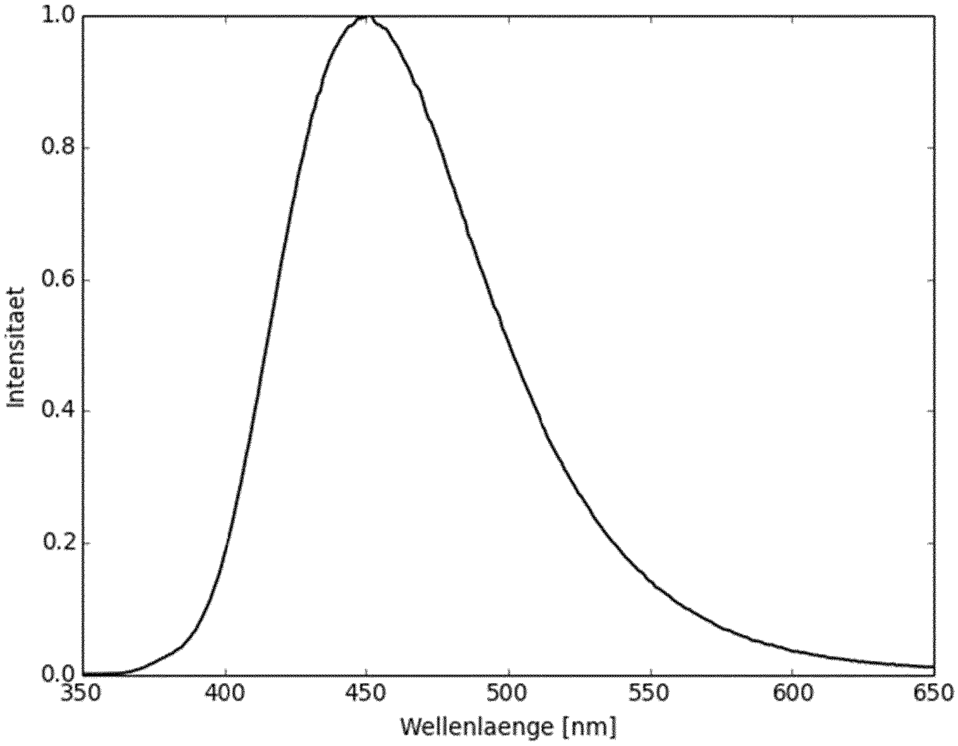

Figur 3

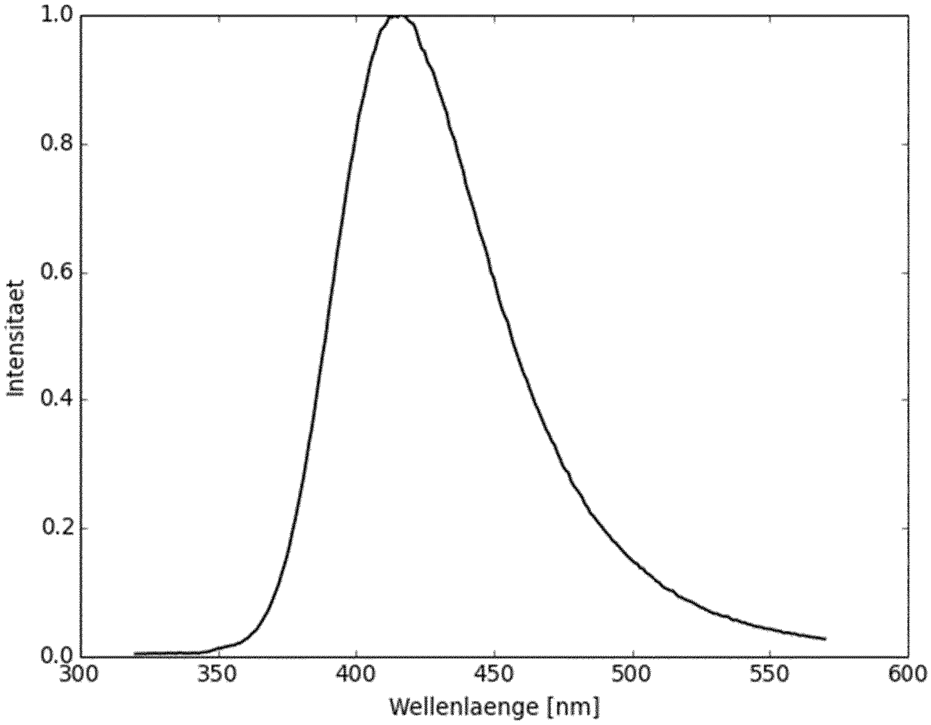


Figur 4

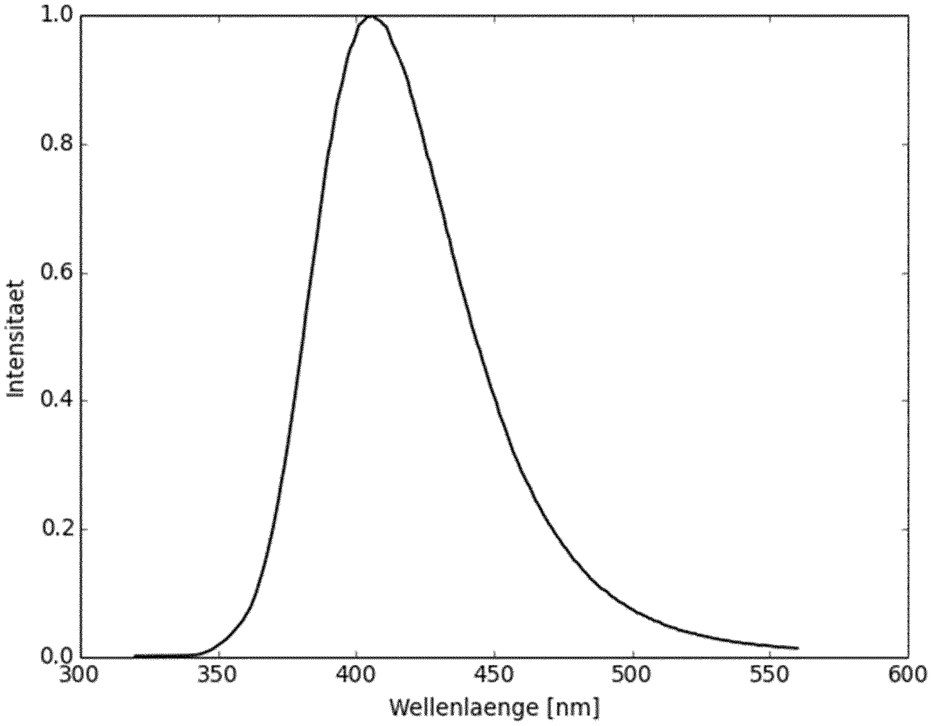

Figur 5

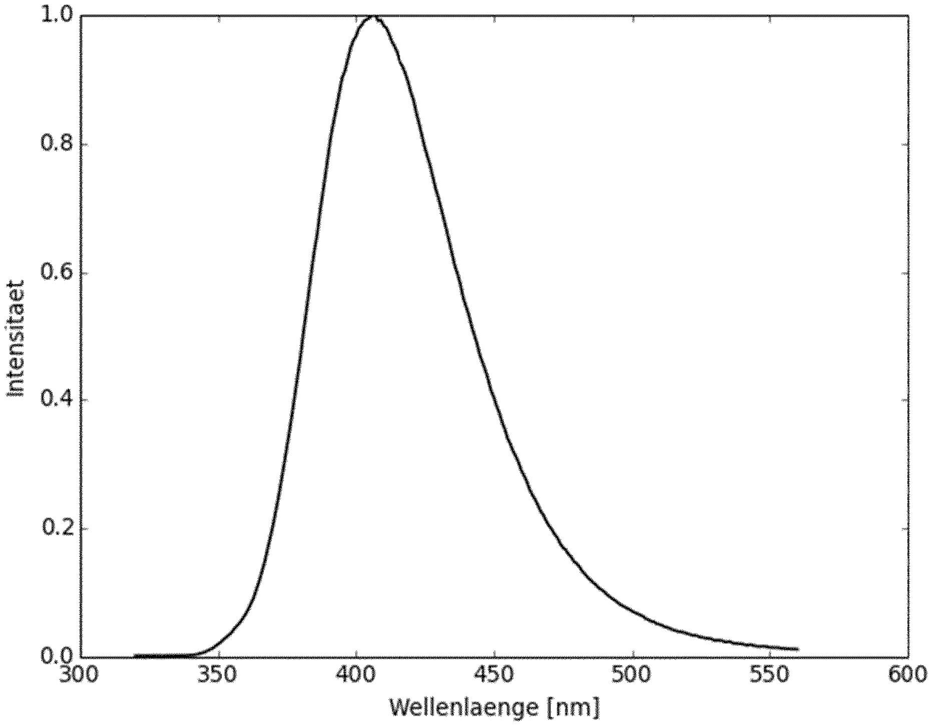


Figur 6

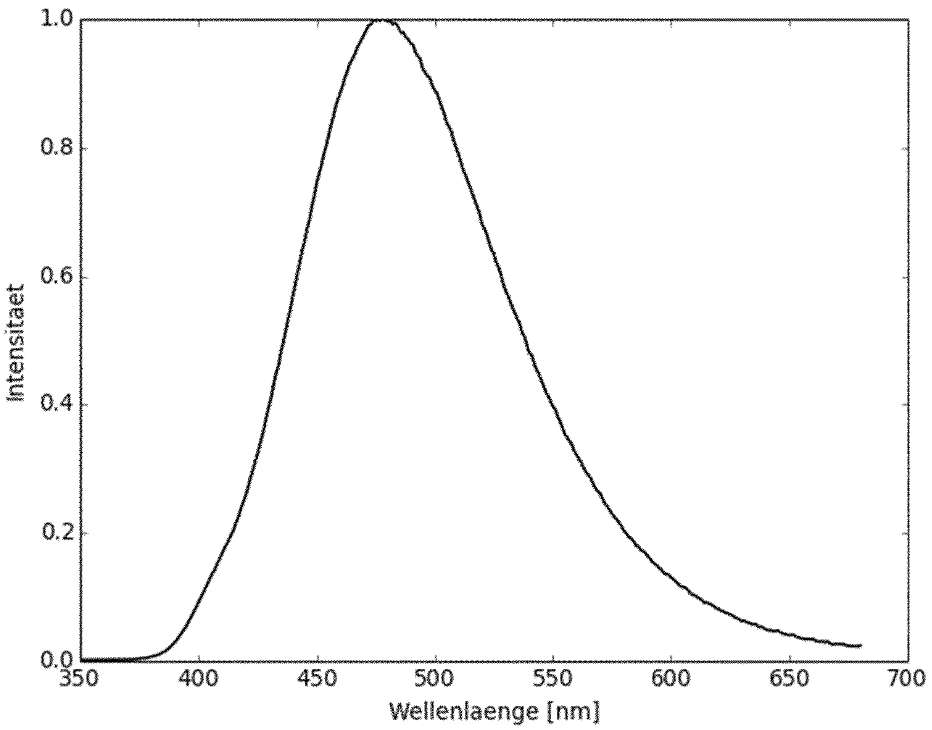

Figur 7

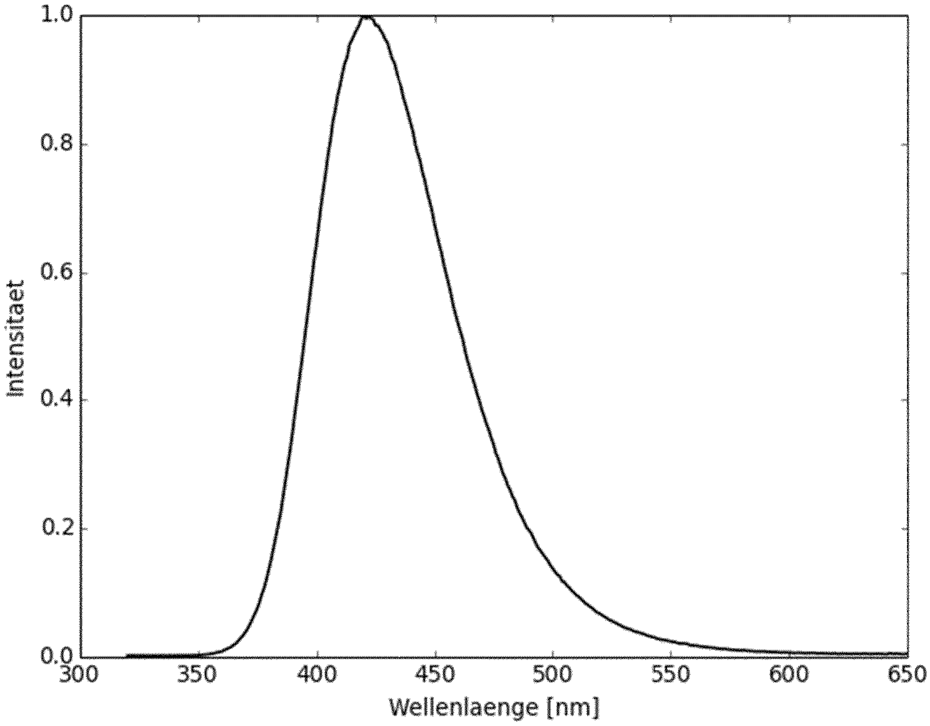


Figur 8

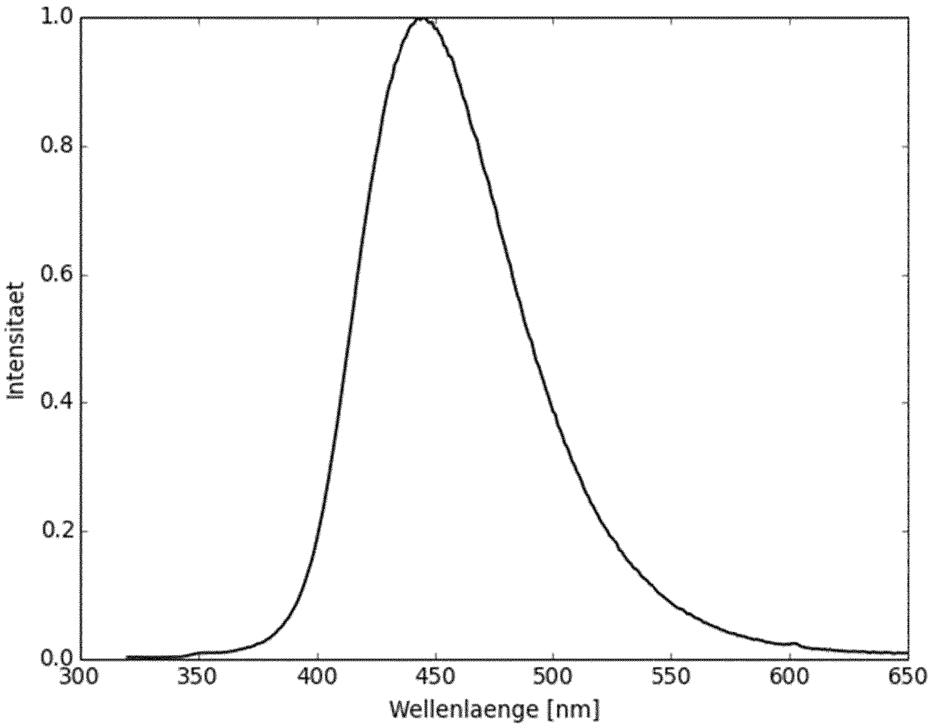

Figur 9

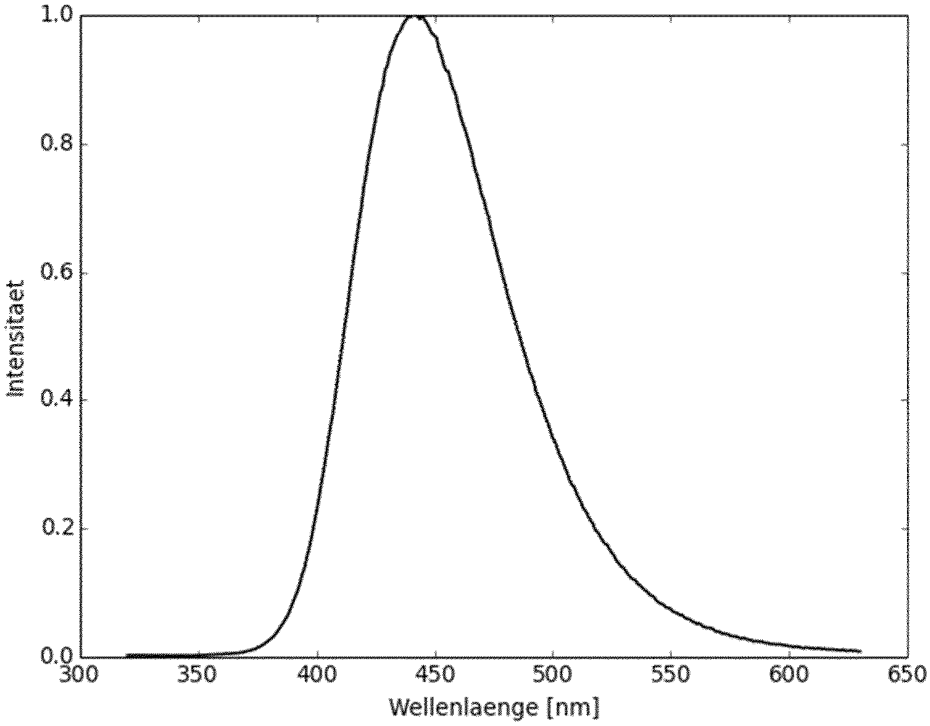


Figur 10

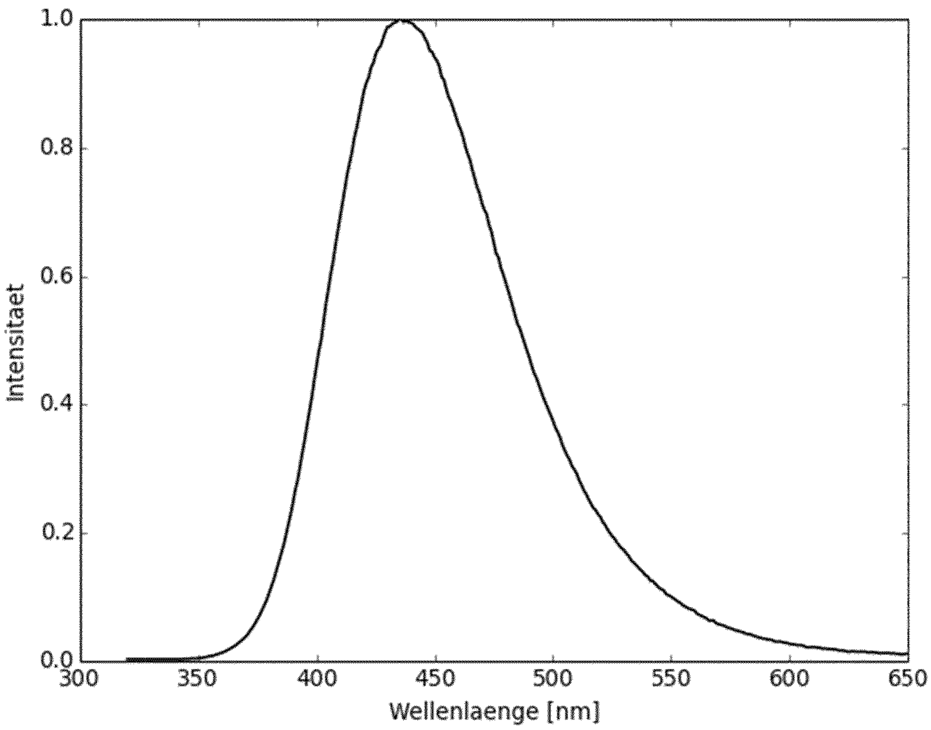

Figur 11

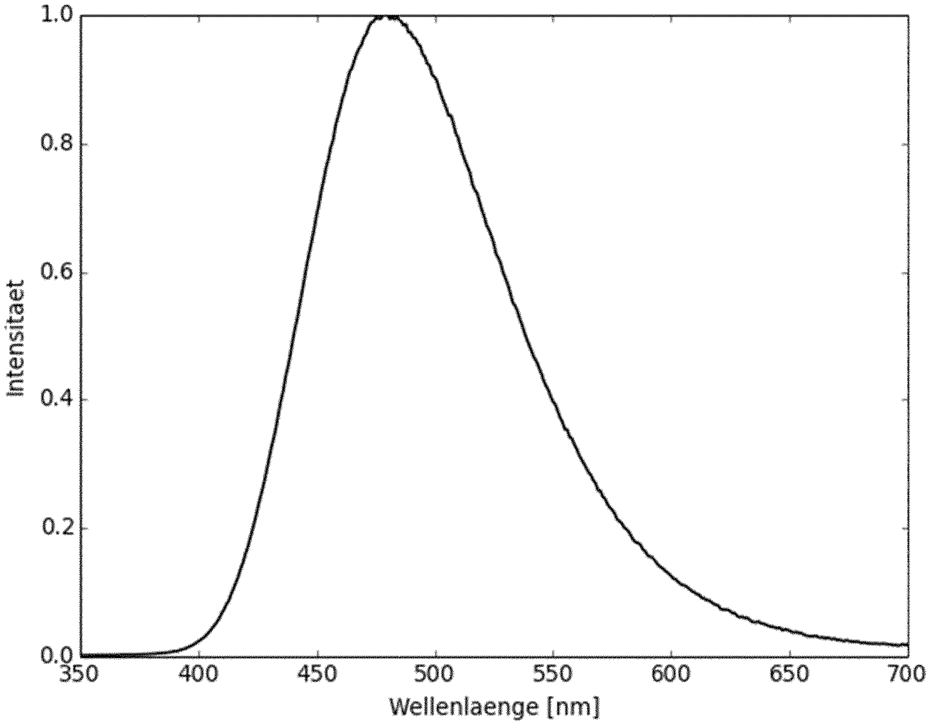


Figur 12

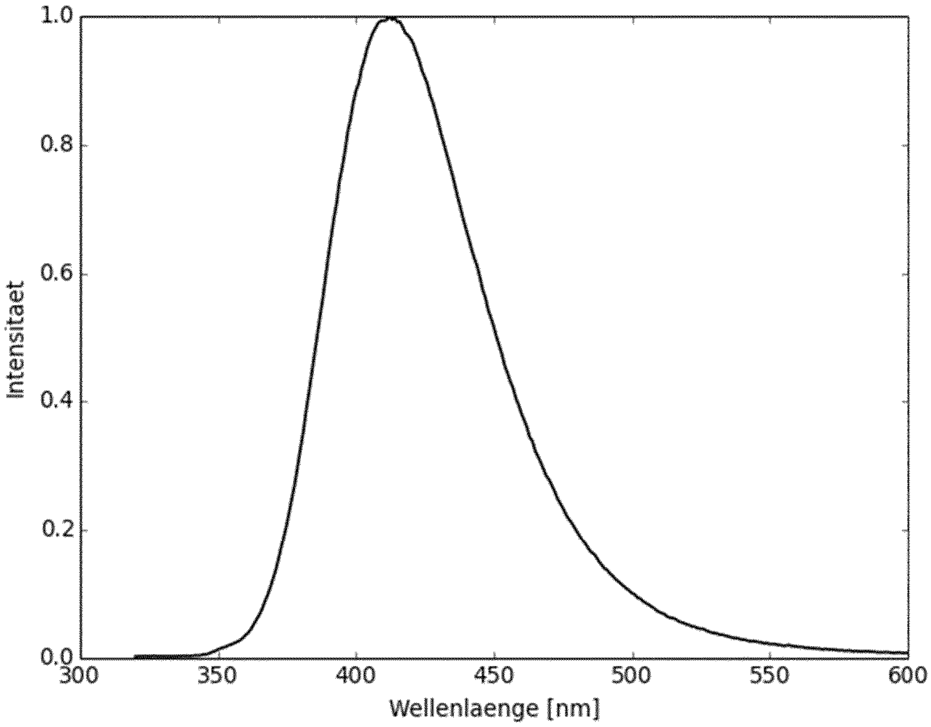

Figur 13

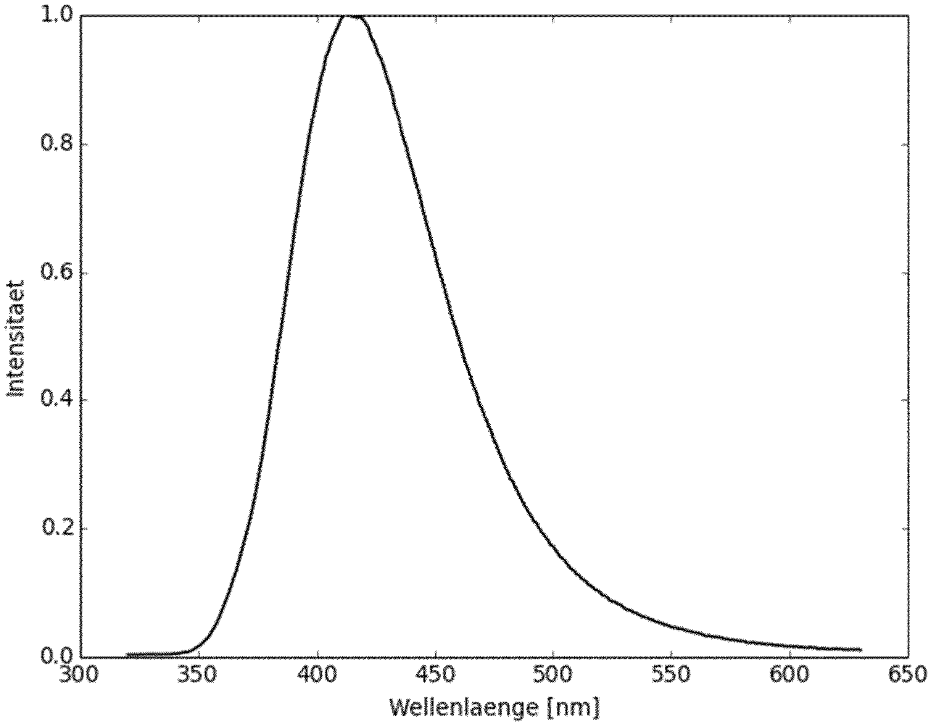


Figur 14

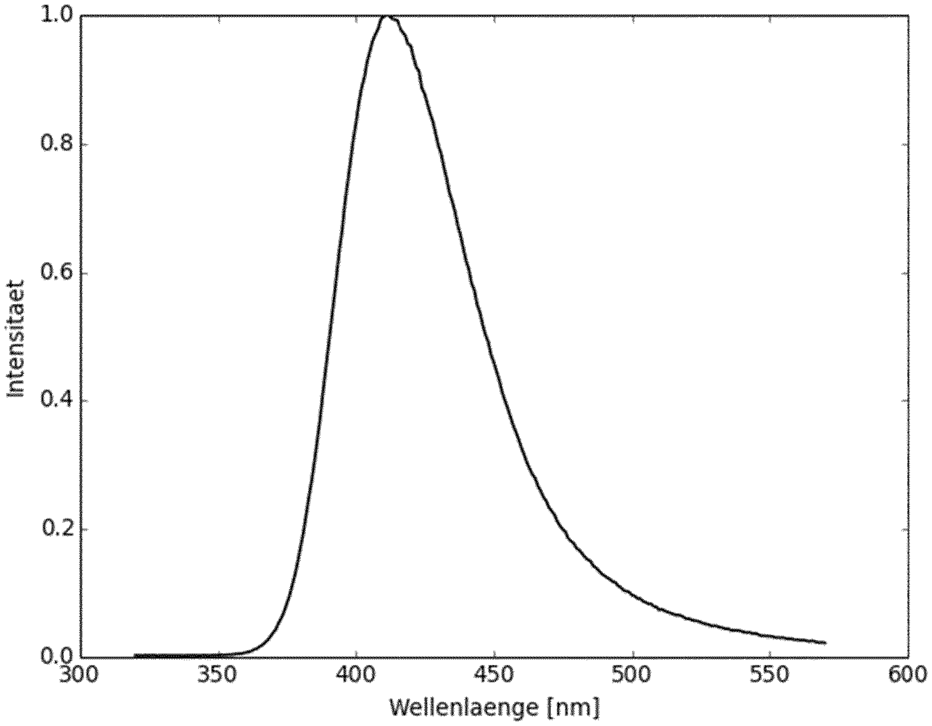

Figur 15

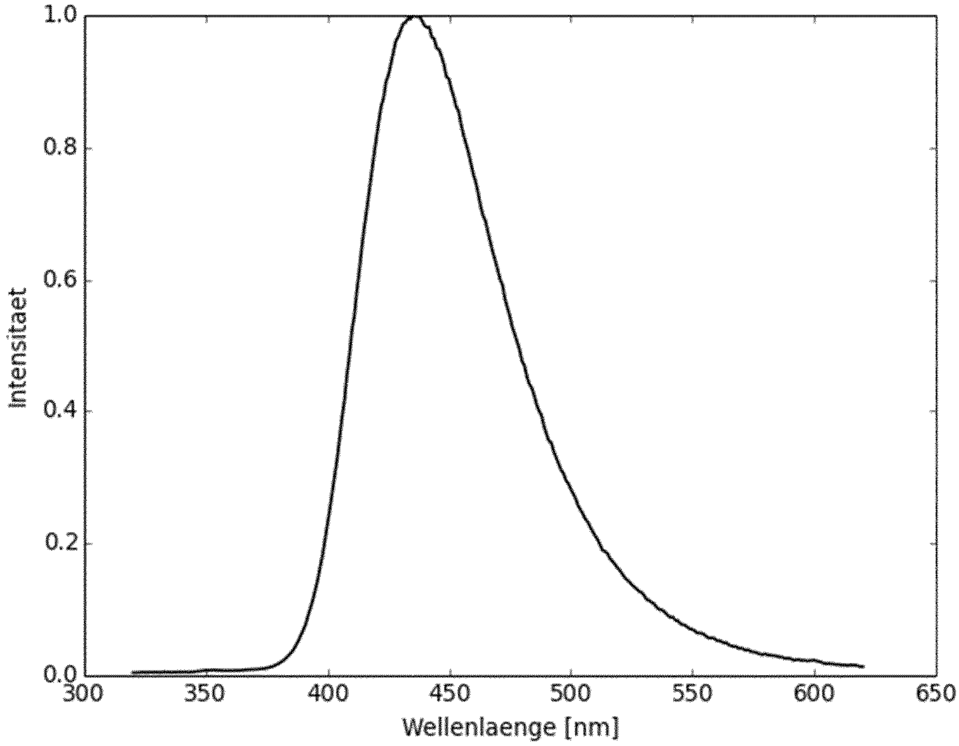


Figur 16

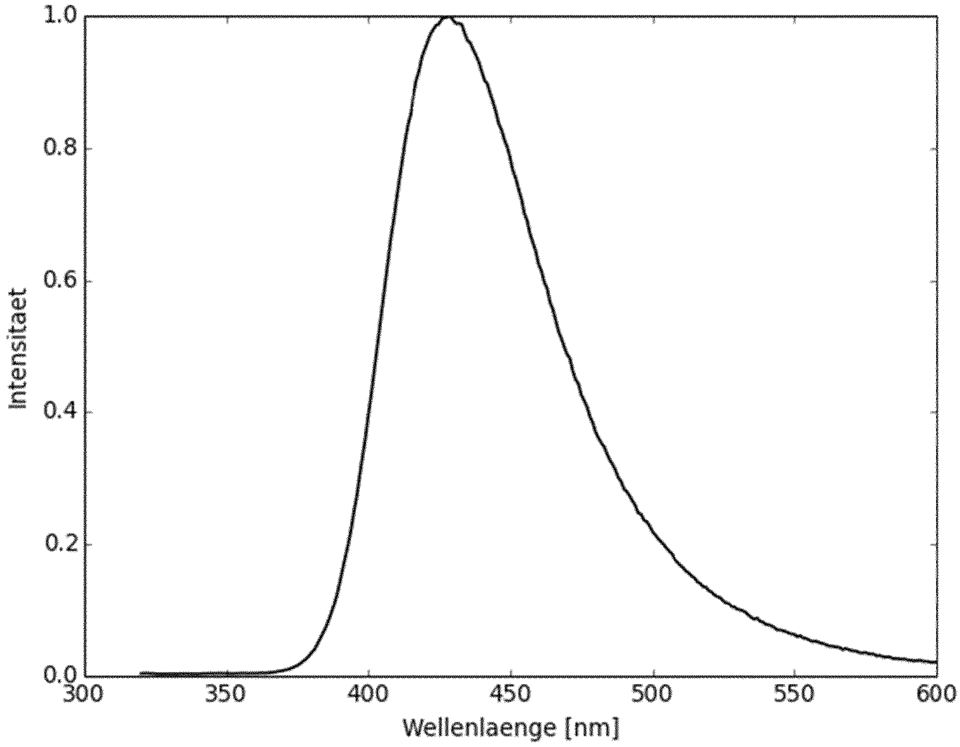

Figur 17

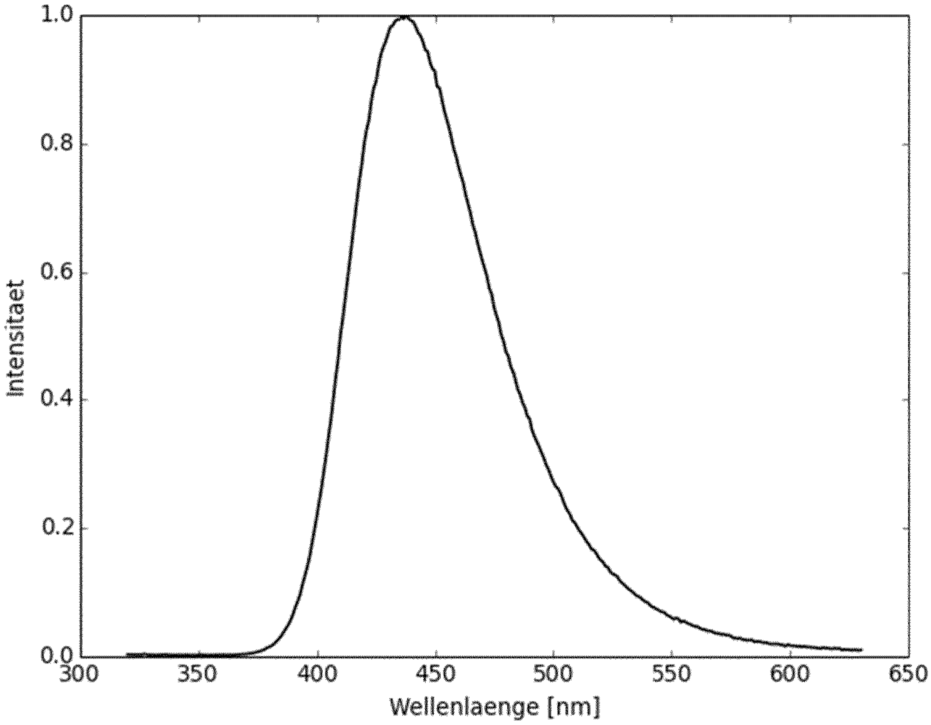


Figur 18

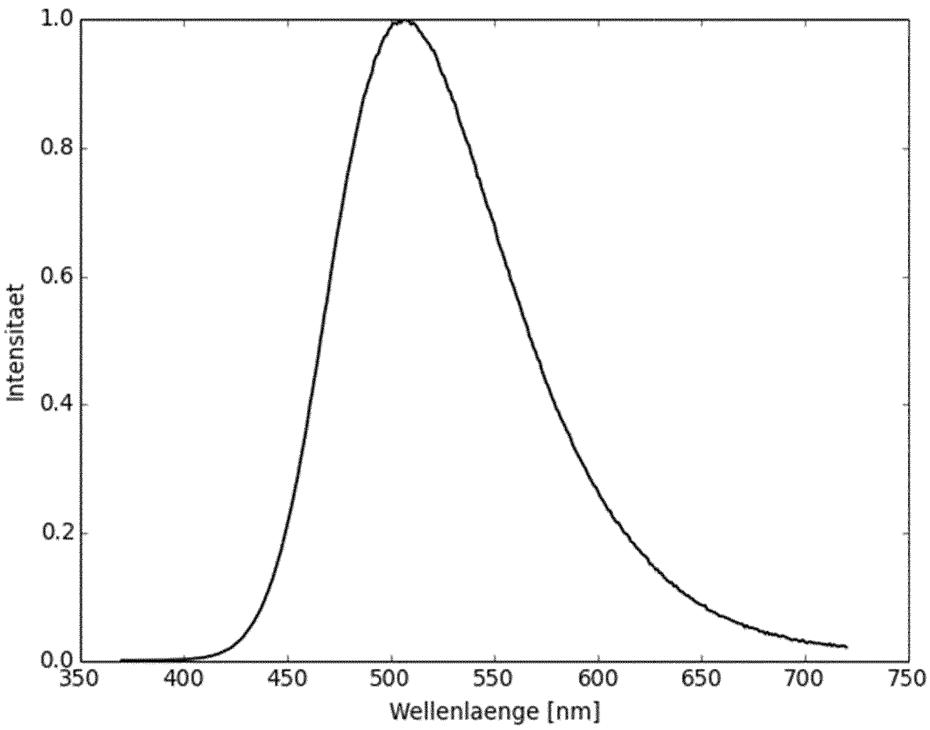

Figur 19

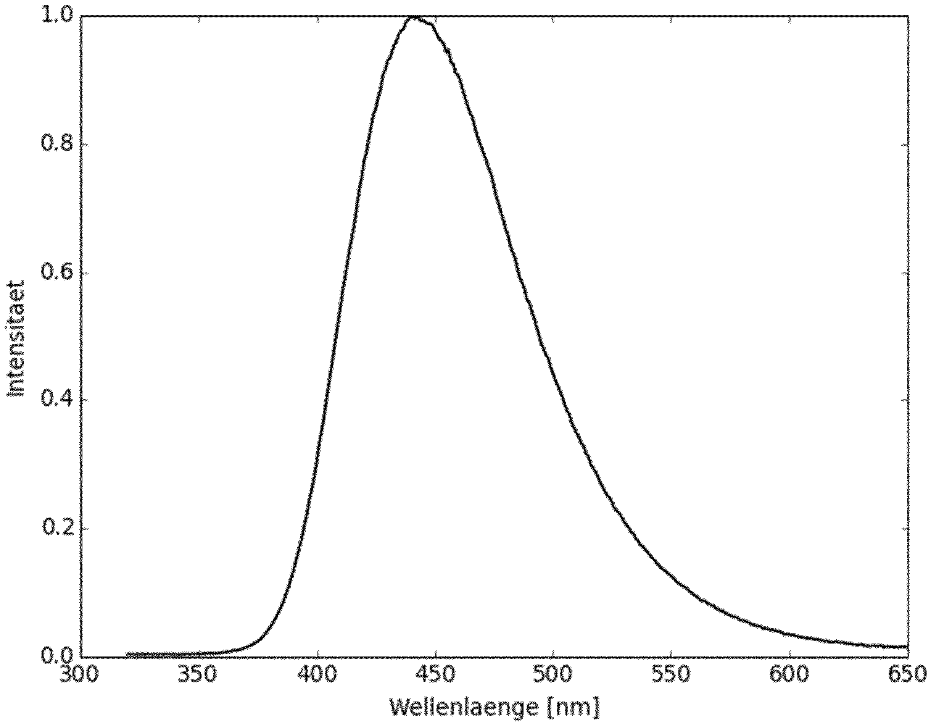


Figur 20

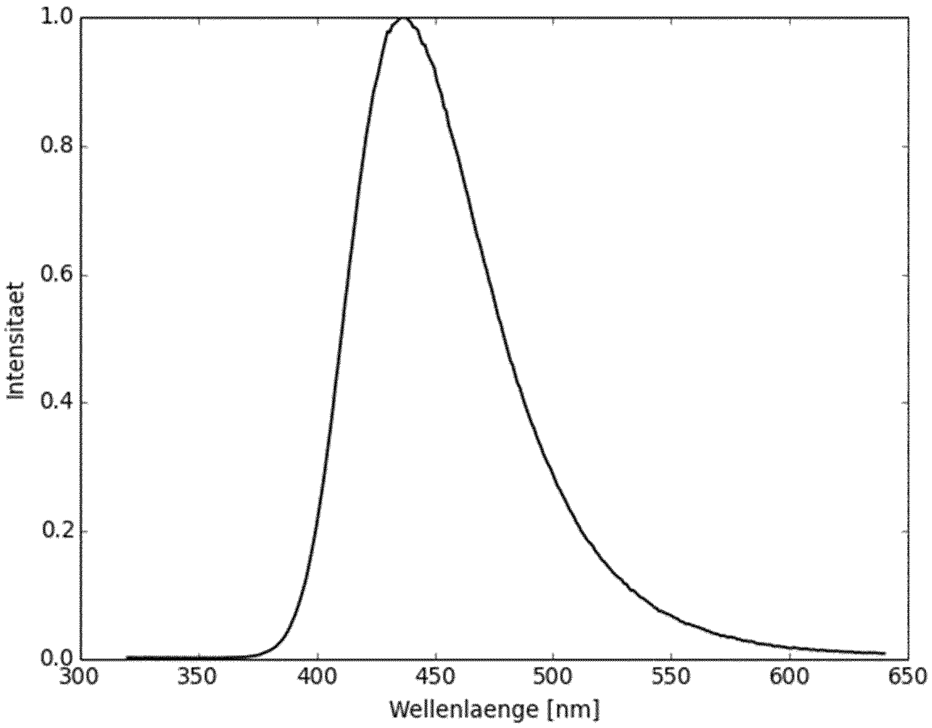

Figur 21

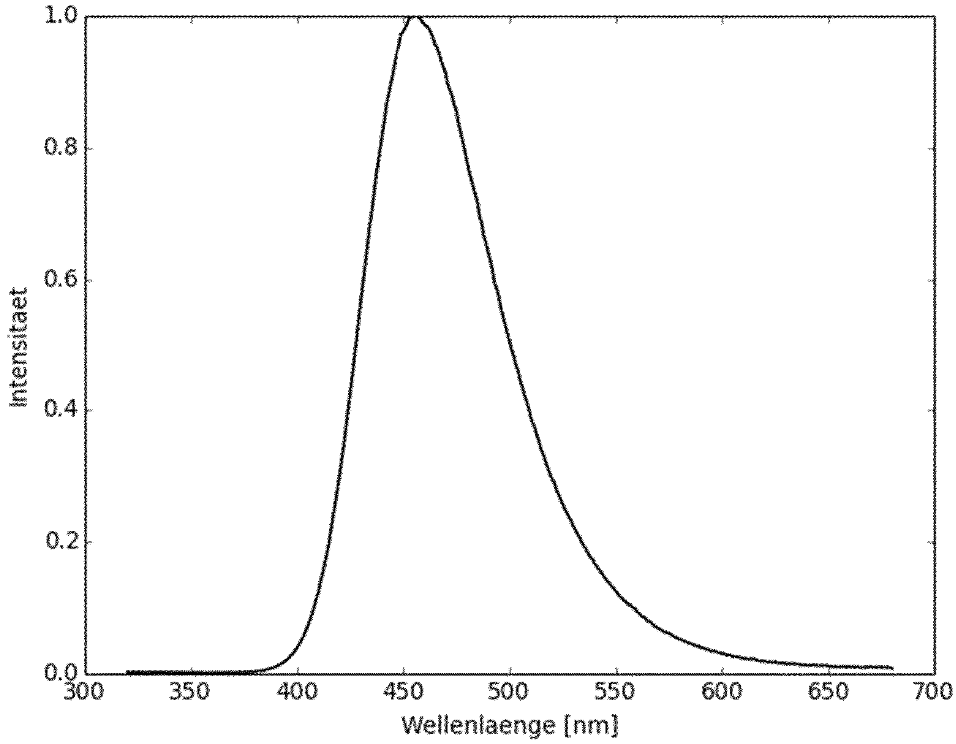


Figur 22

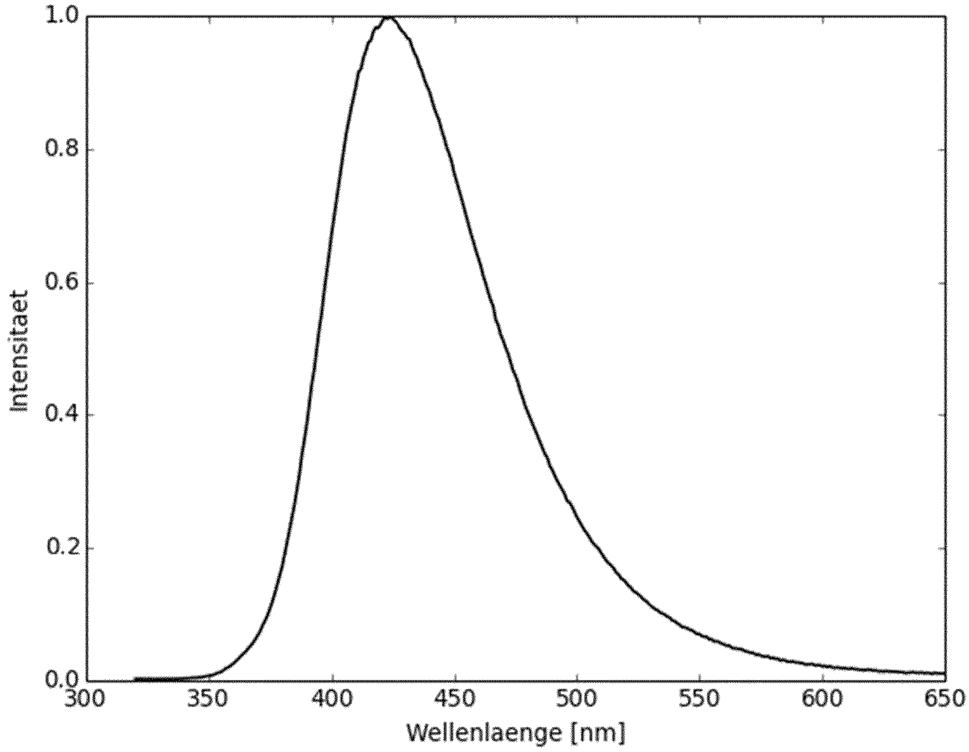

Figur 23

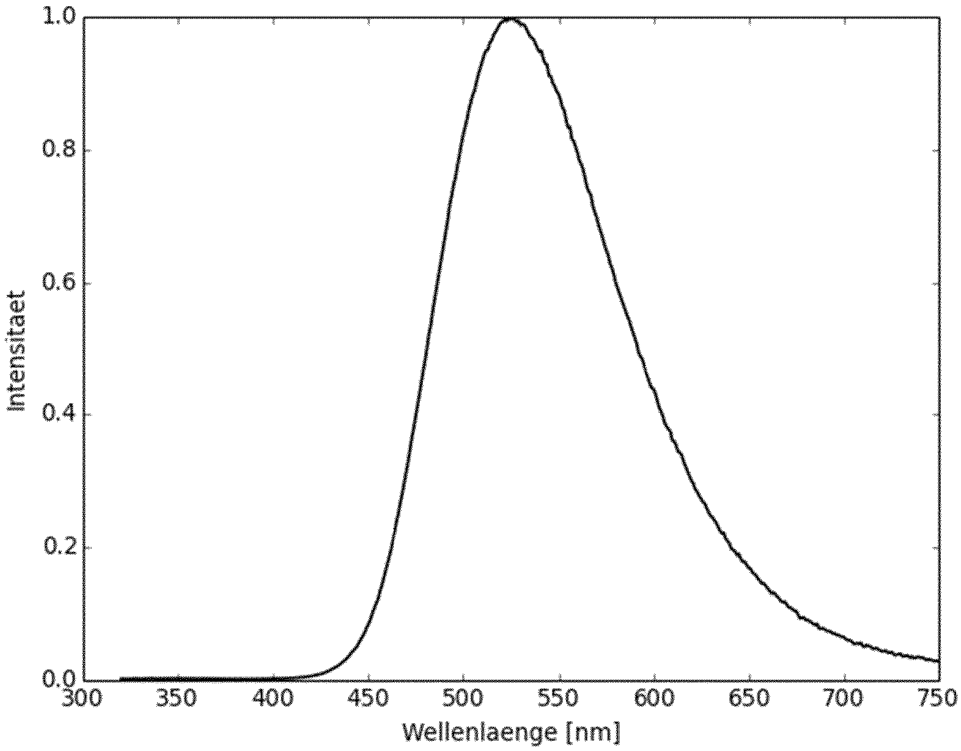


Figur 24

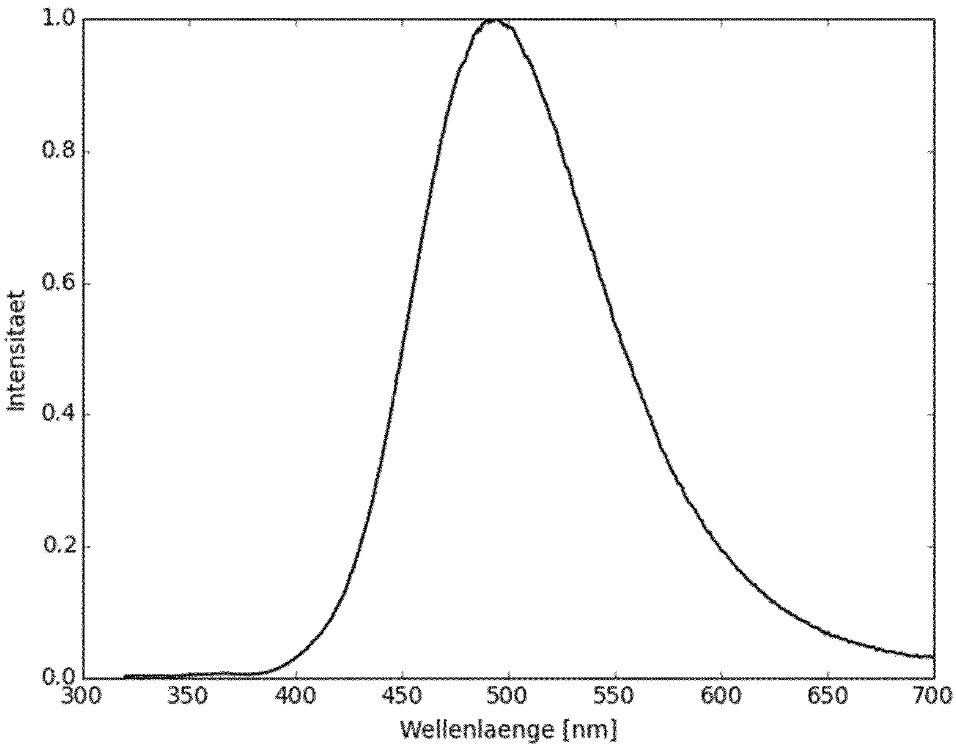

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2015111864 A1